# EUROPEAN PATENT SPECIFICATION

(11) **EP 3 962 524 B1**
(45) Date of publication and mention of the grant of the patent: **07.10.2026**
(21) Application number: 20798248.9
(22) Date of filing: 01.05.2020
(51) Int. Cl.: A61K 31/722, A61K 39/39, A61K 47/36, A61P 37/02, A61P 37/04, A61K 31/728, A61K 31/734, A61K 31/78, A61K 31/785, A61K 31/80, A61K 33/00, A61P 35/00, A61P 35/02

(54) **COMPOSITION COMPRISING CARBOXYMETHYL CHITOSAN FOR USE IN THE TREATMENT OF CANCER**
ZUSAMMENSETZUNG, DIE CARBOXYMETHYLCHITOSAN ZUR VERWENDUNG BEI DER BEHANDLUNG VON KREBS UMFASST
COMPOSITION COMPRENANT DU CARBOXYMÉTHYL CHITOSANE POUR UNE UTILISATION DANS LE TRAITEMENT DU CANCER

(30) Priority: 02.05.2019 US 201962842068 P
(43) Date of publication of application: 09.03.2022
(73) Proprietor: Surge Therapeutics, Inc., Cambridge MA 02142 (US)
(72) Inventor: GOLDBERG, Michael Solomon, Brookline, Massachusetts 02446 (US)
(74) Representative: EIP
(86) International application number: PCT/US2020/031169
(87) International publication number: WO 2020/223698

(56) References cited:
- WO-A1-2017/186088
- WO-A1-2018/045058
- WO-A1-2018/088933
- WO-A2-2007/052267
- RU-C2- 2 288 730
- US-A- 5 965 379
- US-A1- 2005 266 076
- LAUW F N ET AL: "Proinflammatory effects of IL-10 during human endotoxemia", THE JOURNAL OF IMMUNOLOGY, WILLIAMS & WILKINS CO, US, vol. 165, 1 January 2000 (2000-01-01), pages 2783 - 2789, XP002987136, ISSN: 0022-1767
- LAETITIA PICAUD ET AL: "Evaluation of the effects of hyaluronic acid-carboxymethyl cellulose barrier on ovarian tumor progression", JOURNAL OF OVARIAN RESEARCH, BIOMED CENTRAL LTD, LONDON, UK, vol. 7, no. 1, 16 April 2014 (2014-04-16), pages 40, XP021182417, ISSN: 1757-2215, DOI: 10.1186/1757-2215-7-40
- ALANIZ L ET AL: "Low molecular weight hyaluronan inhibits colorectal carcinoma growth by decreasing tumor cell proliferation and stimulating immune response", CANCER LETTERS, NEW YORK, NY, US, vol. 278, no. 1, 8 June 2009 (2009-06-08), pages 9 - 16, XP026065468, ISSN: 0304-3835, [retrieved on 20090129], DOI: 10.1016/J.CANLET.2008.12.029
- VO JIMMY LN ET AL: "Neoadjuvant immunotherapy with chitosan and interleukin-12 to control breast cancer metastasis", ONCOIMMUNOLOGY, vol. 3, no. 12, 26 November 2014 (2014-11-26), pages e968001, XP93010433, DOI: 10.4161/21624011.2014.968001
- PARK, CG ET AL.: "Extended release of perioperative immunotherapy prevents tumor recurrence and eliminates metastases", SCIENCE TRANSLATIONAL MEDICINE, vol. 10, 21 March 2018 (2018-03-21), XP055579366, DOI: 10.1126/scitranslmed.aar1916

## Description

### Background

Systemic administration of medication, nutrition, or other substances into the circulatory system affects the entire body. Systemic routes of administration include enteral *(e.g.,* oral dosage resulting in absorption of the drug through the gastrointestinal tract) and parenteral (e.g., intravenous, intramuscular, and subcutaneous injections) administration. Administration of immunotherapeutics typically relies on these systemic administration routes, which can lead to unwanted side effects. In some instances, certain promising therapeutics are extremely difficult to develop due to associated toxicities and the limitations of current administration methods and systems.

Surgery is often the first-line of treatment for solid tumor cancers and is generally used in combination with systemic administration of anti-cancer therapy. However, surgery-induced immunosuppression has been implicated in the development of post-operative septic complications and tumor metastasis due to changes in a variety of metabolic and endocrine responses, ultimately resulting in the death of many patients (Hiller, J.G. et al. Nature Reviews Clinical Oncology, 2018, 15, 205-218). Vo, Jimmy LN, et al. "Neoadjuvant immunotherapy with chitosan and interleukin-12 to control breast cancer metastasis" Oncoimmunology 3(12) (2014): e968001 disclose the use of neoadjuvant immunotherapy with chitosan and interleukin-12 to control breast cancer metastasis.

### Summary

The invention is defined in the appended claims.

### Reference Technical Disclosure

The reference technical disclosure set out below may in some respects go beyond the disclosure of the invention *per se,* and may also provide technical background for related technical developments, as illustrated *inter alia* in the examples. It will therefore be appreciated that this reference technical disclosure is not intended to define the invention as such, but rather to place it in a broader technical context.

Any references to methods of treatment by therapy or surgery or *in vivo* diagnosis methods in this description are to be interpreted as references to the materials of the present invention for use in those methods.

Systemic administration of immunotherapies can result in adverse side effects, e.g., inducing toxicities that are undesirable for non-cancerous cells and/or tissues such as non-tumor-specific immune cells, and/or requiring high doses in order to achieve sufficient concentration at a target site to induce a therapeutic response. Surgical resection of tumors can result in immunosuppression. Such changes in immune responses that may occur at a surgical site following tumor resection might promote or facilitate activation of dormant micrometastases and/or propagation of residual cancer cells, thus increasing the risk of cancer recurrence.

The present inventor has previously described a system comprising a biomaterial and a payload that may be or comprise an innate immunity modulatory agent (see, for example, WO 2018/045058) can be remarkably useful, among other things, when administered to subjects who have undergone or are undergoing tumor resection. Attributes of this system addressed the source of one or more problems associated with certain prior technologies including, for example, certain conventional approaches to cancer treatment. For example, this system could reduce and/or avoid certain adverse events (*e.g.,* skin rashes, hepatitis, diarrhea, colitis, hypophysitis, thyroiditis, and adrenal insufficiency) that can be associated with systemic administration of immunotherapeutic agents. Among other things, this system could reduce or eliminate exposure of non-tumor-specific immune cells to systemically-administered immunotherapeutic drug(s) and/or to high doses of such drug(s) that are often required in order for systemic administration to achieve sufficient concentration in the tumor to induce a desired response; among other things, the system could provide local agonism of innate immunity following tumor resection, which, among other things, can improve efficacy by concentrating the action of the drug where it is needed. Additionally or alternatively, systems that provide local agonism of innate immunity following resection can, among other things, break local immune tolerance toward cancer and allow for development of systemic antitumor immunity, which can, for example, in some cases, lead to eradiation of disseminated disease.

The present disclosure provides a further surprising insight that certain biomaterials, including polymeric biomaterials, may be able to provide sufficient immunomodulatory activity, e.g., innate immunity modulatory activity, to achieve beneficial effects, *e.g.,* as described herein, even absent a separate immunomodulatory payload, such as an innate immunity modulatory payload.

Biomaterials have been typically used as auxiliary carriers and/or adjuvants to deliver a therapeutic agent and are not previously considered as driving efficacy of a therapeutic treatment. For example, rather than acting as an active ingredient in a therapeutic, chitosan is commonly known as a vehicle to deliver gene therapy and/or chemotherapy, or as an adjuvant to enhance the immunostimulatory activity of a delivered antigen. The present disclosure provides insights that certain types of biomaterials, even when administered in the absence of a known therapeutic agent, can be useful for cancer treatment. Thus, the present disclosure teaches usefulness for cancer therapy of biomaterials previously not considered as a main driver for treatment and furthermore teaches treatment strategies that are particularly effective and/or desirable for these and other biomaterials, including polymeric biomaterials.

The present disclosure recognizes, among other things, that certain biomaterials (*e.g*., polymeric biomaterials) can themselves modulate immune response(s) to a degree and/or or a nature sufficient that, when placed at a target site following tumor resection, can facilitate and/or promote antitumor immunity. For example, the present disclosure recognizes, among other things, that certain biomaterials (*e.g.*, polymeric biomaterials) can themselves stimulate innate immunity to a degree and/or of a nature sufficient that, when placed at a target site following tumor resection, can induce antitumor immunity. In some cases, such antitumor immunity can lead to a reduction in the risk and/or incidence of cancer recurrence and/or metastasis and/or mortality related thereto. Thus, the present disclosure provides systems and/or compositions that can concentrate the immunostimulatory action to a target site in need thereof (*e.g.,* a site at which a tumor has been surgically removed). Such systems and/or compositions can be particularly useful for treating cancer, for example, by reducing or inhibiting (*e.g*., delaying onset of, reducing extent of) tumor recurrence and/or metastasis, in some cases while minimizing adverse side effects and/or systemic exposure to agonists of innate immunity.

In some aspects, disclosed are methods comprising administering to a target site in a subject following tumor removal, a composition comprising an innate immunity modulatory component, wherein the innate immunity modulatory component consists essentially of or consists of a biomaterial (*e.g*., a polymeric biomaterial) agonist of innate immunity.

In some cases, a biomaterial (*e.g*., a polymeric biomaterial) agonist of innate immunity disclosed and/or utilized herein can, indirectly or directly, activate a pattern recognition receptor such that innate immunity is induced. Examples of such a pattern recognition receptor is or comprises a C-type Lectin Receptor (CLR), a NOD-Like Receptor (NLR), a RIG-I-Like Receptor (RLR), and/or a Toll-Like Receptor (TLR). In some cases, a biomaterial (*e.g*., a polymeric biomaterial) agonist of innate immunity disclosed and/or utilized herein can, indirectly or directly, activate an inflammasome (*e.g.*, but not limited to an AIM2 inflammasome, an NLRP1 (NALP1b) inflammasome, an NLRP3 (NALP3) inflammasome, and/or an NLRC4 (IPAF) inflammasome) such that innate immunity is induced. In some cases, a biomaterial (*e.g.,* a polymeric biomaterial) agonist of innate immunity disclosed and/or utilized herein can, indirectly or directly, activate a cGAS-STING pathway, such that innate immunity is induced. In some cases, a biomaterial (*e.g.,* a polymeric biomaterial) agonist of innate immunity disclosed and/or utilized herein can, indirectly or directly, induce activity and/or level of NFₖB. In some cases, a biomaterial *(e.g.,* a polymeric biomaterial) agonist of innate immunity disclosed and/or utilized herein can, indirectly or directly, lead to production of reactive oxygen species.

For example, in some cases, a biomaterial (*e.g.,* a polymeric biomaterial) agonist of innate immunity disclosed and/or utilized herein is characterized in that, when assessed at 24 hours after administration to a target site in a subject in need thereof, more proinflammatory cytokine(s) is present at a target site and/or in body circulation of the subject than is observed when the biomaterial (*e.g.,* polymeric biomaterial) agonist of innate immunity is not administered to the target site. Non-limiting examples of such proinflammatory cytokine(s) include CXCL10, IFN-α, IFN-β, IL-1β, IL-6, IL-18, TNF-α, and combinations thereof.

In some cases, a biomaterial (*e.g.,* a polymeric biomaterial) agonist of innate immunity disclosed and/or utilized herein is or comprises a carbohydrate polymer. An exemplary carbohydrate polymer that is useful in accordance with the present disclosure is or comprises chitosan or variants thereof, or combinations thereof. In some cases, additional examples of polymers for use in a biomaterial agonist of innate immunity disclosed and/or utilized herein include, but are not limited to hyaluronic acid, alginate, polyacrylic acid, polyphosphazene, silica gel, and variants thereof, and combinations thereof.

In some cases, a biomaterial (*e.g.,* a polymeric biomaterial) agonist of innate immunity disclosed and/or utilized herein is characterized in that, when assessed *in vivo* by administering to a target site in a test subject, at least 10% of such a biomaterial (*e.g.,* a polymeric biomaterial) agonist of innate immunity remains at the target site *in vivo* 2 days after the administration.

In some cases, a biomaterial (*e.g.,* a polymeric biomaterial) agonist of innate immunity disclosed and/or utilized herein is characterized by a storage modulus of 10 Pa to 50,000 Pa. For example, in some cases, such a biomaterial (*e.g.,* a polymeric biomaterial) agonist of innate immunity is a viscous polymer solution. In some cases, such a biomaterial (*e.g.,* a polymeric biomaterial) agonist of innate immunity is or comprises a polymer network biomaterial (*e.g*., a hydrogel), which in some cases can be or comprise a crosslinked and/or non-crosslinked polymer network biomaterial.

In some cases, a biomaterial (*e.g*., a polymeric biomaterial) agonist of innate immunity disclosed and/or utilized herein is biodegradable *in vivo.*

Technologies disclosed herein are useful for treating subjects after their tumor has been removed (*e.g*., by surgical resection). Accordingly, in some cases, compositions described and/or utilized herein are administered to subjects who have undergone tumor removal (*e.g.,* by surgical resection). In some cases, a composition described and/or utilized herein is administered to a subject who has undergone (*e.g*., who has recently undergone) a tumor resection.

In some cases, a composition described and/or utilized herein is administered to a target site in a subject after the subject's tumor is removed (*e.g*., as described herein). For example, in some cases, such a target site for administration is or comprises a tumor resection site. In some cases, such a tumor resection site may be characterized by absence of gross residual tumor antigen. In some cases, such a target site for administration is or comprises a site in close proximity (*e.g.,* within 4 inches) to a tumor resection site. In some cases, such a target site for administration is or comprises a sentinel lymph node.

As will be understood by one of ordinary skill in the art, compositions that are useful in accordance with the present disclosure can be administered to a target site in subjects in need thereof using appropriate delivery approaches known in the art. For example, in some cases, disclosed technologies can be amenable for administration by injection. For example, in some cases, a composition for such injection is liquid, and a biomaterial (e.g., a polymeric biomaterial) agonist of innate immunity in the composition is or comprises *a* viscous solution (*e.g.,* a viscous polymer solution). In other cases, a composition for such injection is liquid, and a biomaterial (*e.g*., a polymeric biomaterial) agonist of innate immunity in the composition forms a polymer network biomaterial (*e.g.,* as described and/or utilized herein) *in situ* at a target site to which it is injected.

In some cases, technologies disclosed herein can be amenable for administration by implantation. For example, in some cases, a biomaterial agonist of innate immunity in a composition in accordance with the present disclosure is a pre-formed biomaterial (*e.g.,* a pre-formed polymer network biomaterial, which in some cases can be or comprise a crosslinked polymer network biomaterial and/or a non-crosslinked polymer network biomaterial). An exemplary polymer network biomaterial is or comprises a hydrogel.

In some cases, technologies disclosed herein may be useful for treating subjects who are suffering from metastatic cancer. For example, in some cases, a method disclosed herein may comprise administering to a target site (*e.g.,* as described herein) in a subject suffering from one or more metastases who has undergone a tumor resection (*e.g*., surgical resection of a primary tumor), and optionally monitoring at least one metastatic site in the subject after the administration.

In some cases, the present disclosure relates to technologies such that administration of a biomaterial (*e.g*., a polymeric biomaterial) agonist of innate immunity (*e.g.,* as described and/or utilized herein) by itself is sufficient to provide antitumor immunity and thus do not necessarily require administration of, *e.g.,* a tumor antigen, and/or a microparticle, and/or adoptive transfer of immune cells (*e.g.,* T cells) to a subject in need thereof (*e.g.,* as described herein). Accordingly, in some cases, technologies disclosed herein do not include administering a tumor antigen and/or a microparticle to a subject who has received a composition as described and/or utilized herein. In certain cases, technologies disclosed herein do not include adoptive transfer of immune cells (*e.g.,* T cells) to a subject who has received a composition as described and/or utilized herein.

These, and other aspects encompassed by the present disclosure, are described in more detail below and in the claims.

### Brief Description of the Drawing

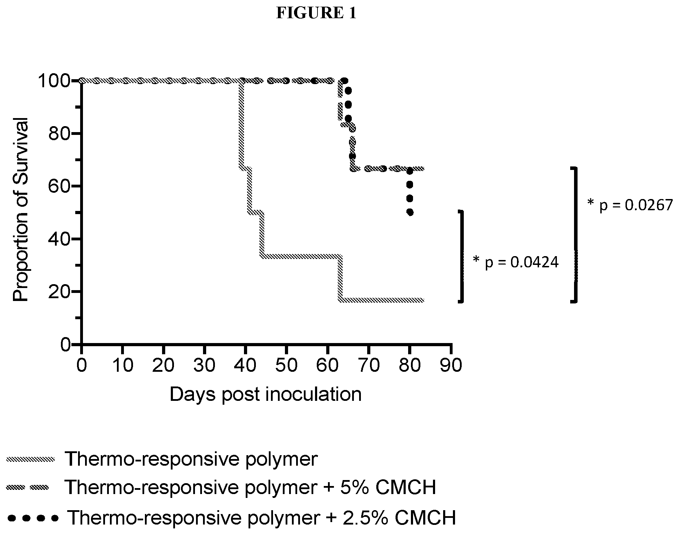

*Figure* 1 shows survival data of animals receiving a liquid preparation of biomaterial agonist of innate immunity (e.g., a liquid preparation of a combination of carboxymethyl chitosan (CMCH) at different concentrations and a thermo-responsive polymer), as compared to animals receiving a liquid preparation of a thermo-responsive polymer in the absence of a biomaterial agonist of innate immunity.

### Certain Definitions

***Activator of adaptive immune response:*** The term "activator of adaptive immune response" refers to an agent that activates an adaptive immune system (and/or one or more features of an adaptive immune system) in a subject (*e.g.,* in a subject to whom it is administered and/or who is otherwise in need thereof). Such activation can restore antitumor function, for example, by neutralizing inhibitory immune checkpoints or by triggering co-stimulatory receptors, ultimately generating helper and/or effector T cell responses against immunogenic antigens expressed by cancer cells and producing memory B cell, plasma cells, and/or T cell populations. In certain cases, the activator of adaptive immune response involves modulation of adaptive immune response and/or leukocyte trafficking. Examples of activators of adaptive immune response include, *e.g.,* ones described in WO 2018/045058. In some cases, those of skill in the art, reading the present disclosure, will appreciate that activators of adaptive immune response that are excluded from compositions or pharmaceutical compositions described herein, in some cases, are or comprise small molecules, polypeptides (e.g., cytokines), and/or nucleic acids.

***Activator of innate immune response:*** The term "activator of innate immune response" refers to an agent that activates an innate immune system (and/or one or more features of an innate immune system) in a subject (*e.g.,* in a subject to whom it is administered and/or who is otherwise in need thereof). Such activation can stimulate (e.g., can increase level and/or activity of) one or more agents that initiate an inflammatory response and/or that help to induce adaptive immune responses, leading to the development of antigen-specific acquired immunity. In some cases, activation of the innate immune system can lead to recruitment of relevant immune cells including, e.g., but not limited to neutrophils, basophils, eosinophils, natural killer cells, dendritic cells, monocytes, and macrophages, cytokine production, enhanced leukocyte proliferation and/or survival, as well as improved T cell priming by augmenting presentation of antigens and/or level and/or activity of co-stimulatory molecules by antigen-presenting cells. Examples of activators of innate immune response include, *e.g.,* ones described in WO 2018/045058. In some cases, those of skill in the art, reading the present disclosure, will appreciate that activators of innate immune response that are excluded from compositions or pharmaceutical compositions described herein, in some cases, are or comprise small molecules, polypeptides (e.g., cytokines), and/or nucleic acids.

***Administering:*** As used herein, the term "administering" or "administration" typically refers to the administration of a composition to a subject to achieve delivery of an agent that is, or is included in, a composition to a target site or a site to be treated. Those of ordinary skill in the art will be aware of a variety of routes that may, in appropriate circumstances, be utilized for administration of different agents to a subject, for example a human. For example, while the terms "administer," "administering," or "administration" generally refer to implanting, absorbing, ingesting, injecting, inhaling, parenteral administration, or otherwise introducing a composition as described herein, in the context of administering a composition comprising a biomaterial (*e.g*., a polymeric biomaterial) agonist of innate immunity disclosed herein, administering may refer to, in some cases, implanting, or in some cases, injecting.

***Agonist:*** Those skilled in the art will appreciate that the term "agonist" may be used to refer to an agent, condition, or event whose presence, level, degree, type, or form correlates with increased level and/or activity of another agent (*i.e.,* the agonized agent). In general, an agonist may be or include an agent of any chemical class including, for example, small molecules, polypeptides, nucleic acids, carbohydrates, lipids, metals, inorganic crystals, and/or any other entity that shows the relevant activating activity. In some cases, an agonist may be direct (in which case it exerts its influence directly upon its target); in some cases, an agonist may be indirect (in which case it exerts its influence by other than binding to its target; *e.g.,* by interacting with a regulator of the target, so that level or activity of the target is altered).

***Antagonist:*** Those skilled in the art will appreciate that the term "antagonist" may refer to an agent, condition, or event whose presence, level, degree, type, or form is associated with a decreased level and/or activity of another agent *(i.e.,* the antagonized agent). In general, an antagonist may include an agent of any chemical class including, for example, small molecules, polypeptides, nucleic acids, carbohydrates, lipids, metals, and/or any other entity that shows the relevant inhibitory activity. In some cases, an antagonist may be a "direct antagonist" in that it binds directly to its target; in some cases, an antagonist may be an "indirect antagonist" in that it exerts its influence by means other than binding directly to its target; *e.g.,* by interacting with a regulator of the target, so that the level or activity of the target is altered).

***Antibody:*** The term "antibody" refers to a functional component of serum and is often referred to either as a collection of molecules (antibodies or immunoglobulins) or as one molecule (the antibody molecule or immunoglobulin molecule). An antibody is a polypeptide capable of binding to or reacting with a specific antigenic determinant (the antigen or the antigenic epitope), which in turn may lead to induction of immunological effector mechanisms. An individual antibody is usually regarded as monospecific, and a composition of antibodies may be monoclonal (*i.e.,* consisting of identical antibody molecules) or polyclonal (*i.e.,* consisting of two or more different antibodies reacting with the same or different epitopes on the same antigen or even on distinct, different antigens). Each antibody has a unique structure that enables it to bind specifically to its corresponding antigen, and all natural antibodies have the same overall basic structure of two identical light chains and two identical heavy chains. Antibodies are also known collectively as immunoglobulins. An antibody may be of human or non-human (for example, rodent such as murine, dog, camel, *etc*.) origin (*e.g.,* may have a sequence originally developed in a human or non-human cell or organism), or may be or comprise a chimeric, humanized, reshaped, or reformatted antibody based, *e.g.,* on a such a human or non-human antibody (or, in some cases, on an antigen-binding portion thereof). In some cases, as will be clear from context, the term "antibody" as used herein encompasses formats that include epitope-binding sequences of an antibody, which such formats include, for example chimeric and/or single chain antibodies (*e.g*., a nanobody or Fcab), as well as binding fragments of antibodies, such as Fab, Fv fragments or single chain Fv (scFv) fragments, as well as multimeric forms such as dimeric IgA molecules or pentavalent IgM molecules. Also included are bispecific antibodies, bispecific T cell engagers (BiTEs), immune mobilizing monoclonal T cell receptors against cancer (ImmTACs), dual-affinity re-targeting (DART); alternative scaffolds or antibody mimetics (*e.g*., anticalins, FN3 monobodies, DARPins, Affibodies, Affilins, Affimers, Affitins, Alphabodies, Avimers, Fynomers, Im7, VLR, VNAR, Trimab, CrossMab, Trident); nanobodies, binanobodies, F(ab')2, Fab', di-sdFv, single domain antibodies, trifunctional antibodies, diabodies, and minibodies.

***Bioadhesive:*** The term "bioadhesive" refers to a biocompatible agent that can adhere to a target surface, *e.g.,* a tissue surface. In some cases, a bioadhesive can adhere to a target surface, *e.g.,* a tissue surface, and retain on the target surface, *e.g.,* for a period of time. In some cases, a bioadhesive may be biodegradable. In some cases, a bioadhesive may be a natural agent, which may have been prepared or obtained, for example, by isolation or by synthesis; in some cases, a bioadhesive may be a non-natural agent, *e.g.,* as may have been designed and/or manufactured by the hand of man (*e.g.,* by processing, ₛynthetic, and/or recombinant production, depending on the agent, as will be understood by those skilled in the art. In some particular cases, a bioadhesive may be or comprise a polymeric material, e.g., as may be comprised of or contain a plurality of monomers such as sugars. Certain exemplary bioadhesives include a variety of FDA-approved agents such as, for example, cyanoacrylates (Dermabond, 2-Octyl cyanoacrylate; Indermil, n-Butyl-2-cyanoacrylate; Histoacryl and Histoacryl Blue, n-Butyl-2-cyanoacrylate), albumin and glutaraldehyde (BioGlue^{™}, bovine serum albumin and 10% glutaraldehyde), fibrin glue (Tisseel^{™}, human pooled plasma fibrinogen and thrombin; Evicel^{™}, human pooled plasma fibrinogen and thrombin; Vitagel^{™}, autologous plasma fibrinogen and thrombin; Cryoseal^{™} system, autologous plasma fibrinogen and thrombin), gelatin and/or resorcinol crosslinked by formaldehyde and/or glutaraldehyde, polysaccharide-based adhesives (*e.g.,* alginate, chitosan, collagen, dextran, and/or gelatin), PEG, acrylates, polyamines, or urethane variants (isocyanate-terminated prepolymer, and/or combinations thereof. Other examples of bioadhesives that are known in the art, *e.g.,* as described in Mehdizadeh and Yang "Design Strategies and Applications of Tissue Bioadhesives" Macromol Biosci 13:271-288 (2013), can be used for the purposes of the methods described herein. In some cases, a bioadhesive can be a degradable bioadhesive. Examples of such a degradable bioadhesive include, but are not limited to fibrin glues, gelatin-resorcinol-formaldehyde/glutaraldehyde glues, poly(ethylene glycol) (PEG)-based hydrogel adhesives, polysaccharide adhesives, polypeptide adhesives, polymeric adhesives, biomimetic bioadhesives, and ones described in Bhagat and Becker "Degradable Adhesives for Surgery and Tissue Engineering" Biomacromolecules 18: 3009-3039 (2017).

***Biocompatible:*** The term "biocompatible", as used herein, refers to materials that do not cause significant harm to living tissue when placed in contact with such tissue, *e.g., in vivo.* In certain cases, materials are "biocompatible" if they themselves are not toxic to cells. In certain cases, materials are "biocompatible" if their addition to cells *in vitro* results in less than or equal to 20% cell death and/or their administration *in vivo* does not induce significantly severe inflammation that is clinically undesirable for purposes described herein or other such adverse effects. As will be understood by those skilled in the art that such significantly severe inflammation is distinguishable from mild, transient inflammation, which typically accompanies surgery or introduction of foreign objects into a living organism. Furthermore, one of skill in the art will appreciate, reading the present disclosure, that polymers and/or biomaterial (*e.g*., polymeric biomaterial) agonists of innate immunity are biocompatible if extent of innate immunity agonism over a defined period of time is clinically beneficial and/or desirable, *e.g*., to provide antitumor immunity.

***Biodegradable:*** As used herein, the term "biodegradable" refers to materials that, when introduced into cells, are broken down *(e.g.,* by cellular machinery, such as by enzymatic degradation, by hydrolysis, and/or by combinations thereof) into components that cells can either reuse or dispose of without significant toxic effects on the cells. In certain cases, components generated by breakdown of a biodegradable material are biocompatible and therefore do not induce significantly severe inflammation that is clinically undesirable for purposes described herein and/or other adverse effects *in vivo.* In some cases, biodegradable polymer materials break down into their component monomers. In some cases, biodegradable polymer materials may be biologically degraded, *e.g.,* by enzymatic activity or cellular machinery, in some cases, for example, through exposure to a lysozyme (e.g., having relatively low pH), or by simple hydrolysis. In some cases, breakdown of biodegradable materials (including, for example, biodegradable polymer materials) involves hydrolysis of ester bonds. Alternatively or additionally, in some cases, breakdown of biodegradable materials (including, for example, biodegradable polymer materials) involves cleavage of urethane linkages. Exemplary biodegradable polymers include, for example, polymers of hydroxy acids such as lactic acid and glycolic acid, including but not limited to poly(hydroxyl acids), poly(lactic acid)(PLA), poly(glycolic acid)(PGA), poly(lactic-co-glycolic acid)(PLGA), and copolymers with PEG, polyanhydrides, poly(ortho)esters, polyesters, polyurethanes, poly(butyric acid), poly(valeric acid), poly(caprolactone), poly(hydroxyalkanoates), poly(lactide-co-caprolactone), blends and copolymers thereof. Many naturally occurring polymers are also biodegradable, including, for example, proteins such as albumin, collagen, gelatin and prolamines, for example, zein, and polysaccharides such as alginate, cellulose variants and polyhydroxyalkanoates, for example, polyhydroxybutyrate blends and copolymers thereof. Those of ordinary skill in the art will appreciate or be able to determine when such polymers are biocompatible and/or biodegradable variants thereof (*e.g.,* related to a parent polymer by substantially identical structure that differs only in substitution or addition of particular chemical groups as is known in the art).

***Biologic:*** The terms "biologic," "biologic drug," and "biological product" refer to a wide range of products such as vaccines, blood and blood components, allergenics, somatic cells, gene therapy, tissues, nucleic acids, and proteins. Biologics may include proteins, or nucleic acids, or complex combinations of these substances, or may be living entities such as cells and tissues. Biologics may be isolated from a variety of natural sources (*e.g*., human, animal, microorganism) and/or may be produced by biotechnological methods and/or other technologies.

***Biomaterial:*** The term "biomaterial" refers to a biocompatible substance characterized in that it can be administered to a subject for a medical purpose (*e.g.*, therapeutic, diagnostic) without eliciting an unacceptable (according to sound medical judgement) reaction. Biomaterials can be obtained or derived from nature or synthesized. In some cases, a biomaterial may be or comprise a polymeric biomaterial. In some cases, a biomaterial can be in a form of a polymer network. In some cases, a biomaterial can be in an injectable format, *e.g.,* a viscous solution. For example, a biomaterial can comprise its precursor components to be formed *in situ (e.g.,* upon administration to a subject). In some cases, a biomaterial can be a liquid. In some cases, a biomaterial is a viscous solution. In some cases, a biomaterial can be a solid. In some cases, a biomaterial can be a crystal (e.g., an inorganic crystal). In some cases, a biomaterial is not a nucleic acid. In some cases, a biomaterial is not a polypeptide.

***Cancer:*** The term "cancer" refers to a malignant neoplasm *(*Stedman's Medical Dictionary, 25th ed.; Hensyl ed.; Williams & Wilkins: Philadelphia, 1990). Of particular interest in the context of some cases of the present disclosure are cancers treated by cell killing and/or removal therapies (*e.g*., surgical resection and/or certain chemotherapeutic therapies such as cytotoxic therapies, *etc*.). In some cases, a cancer that is treated in accordance with the present disclosure is one that has been surgically resected (*i.e.,* for which at least one tumor has been surgically resected). In some cases, a cancer that is treated in accordance with the present disclosure is one for which resection is standard of care. In some cases, a cancer that is treated in accordance with the present disclosure is one that has metastasized. In certain cases, exemplary cancers may include one or more of acoustic neuroma; adenocarcinoma; adrenal gland cancer; anal cancer; angiosarcoma (*e.g*., lymphangiosarcoma, lymphangioendotheliosarcoma, hemangiosarcoma); appendix cancer; benign monoclonal gammopathy; biliary cancer (*e.g*., cholangiocarcinoma); bile duct cancer; bladder cancer; bone cancer; breast cancer (*e.g*., adenocarcinoma of the breast, papillary carcinoma of the breast, mammary cancer, medullary carcinoma of the breast); brain cancer (*e.g.,* meningioma, glioblastomas, glioma (*e.g.,* astrocytoma, oligodendroglioma), medulloblastoma); bronchus cancer; carcinoid tumor; cardiac tumor; cervical cancer (*e.g.,* cervical adenocarcinoma); choriocarcinoma; chordoma; craniopharyngioma; colorectal cancer *(e.g.,* colon cancer, rectal cancer, colorectal adenocarcinoma); connective tissue cancer; epithelial carcinoma; ductal carcinoma *in situ;* ependymoma; endotheliosarcoma *(e.g.,* Kaposi's sarcoma, multiple idiopathic hemorrhagic sarcoma); endometrial cancer *(e.g.,* uterine cancer, uterine sarcoma); esophageal cancer (*e.g.*, adenocarcinoma of the esophagus, Barrett's adenocarcinoma); Ewing's sarcoma; eye cancer (*e.g*., intraocular melanoma, retinoblastoma); familiar hypereosinophilia; gall bladder cancer; gastric cancer (*e.g*., stomach adenocarcinoma); gastrointestinal stromal tumor (GIST); germ cell cancer; head and neck cancer *(e.g.,* head and neck squamous cell carcinoma, oral cancer *(e.g.,* oral squamous cell carcinoma), throat cancer (e.g., laryngeal cancer, pharyngeal cancer, nasopharyngeal cancer, oropharyngeal cancer)); hematopoietic cancers (e.g., leukemia such as acute lymphocytic leukemia (ALL) *(e.g.,* B-cell ALL, T-cell ALL), acute myelocytic leukemia (AML) *(e.g.,* B-cell AML, T-cell AML), chronic myelocytic leukemia (CML) *(e.g.,* B-cell CML, T-cell CML), and chronic lymphocytic leukemia (CLL) *(e.g.,* B-cell CLL, T-cell CLL)); lymphoma such as Hodgkin lymphoma (HL) *(e.g.,* B-cell HL, T-cell HL) and non-Hodgkin lymphoma (NHL) *(e.g.,* B-cell NHL such as diffuse large cell lymphoma (DLCL) *(e.g.,* diffuse large B-cell lymphoma), follicular lymphoma, chronic lymphocytic leukemia/small lymphocytic lymphoma (CLL/SLL), mantle cell lymphoma (MCL), marginal zone B-cell lymphomas (e.g., mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma *(i.e.,* Waldenström's macroglobulinemia), hairy cell leukemia (HCL), immunoblastic large cell lymphoma, precursor B-lymphoblastic lymphoma and primary central nervous system (CNS) lymphoma; and T-cell NHL such as precursor T-lymphoblastic lymphoma/leukemia, peripheral T-cell lymphoma (PTCL) *(e.g.,* cutaneous T-cell lymphoma (CTCL) (*e.g.,* mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, and anaplastic large cell lymphoma); a mixture of one or more leukemia/lymphoma as described above; multiple myeloma; heavy chain disease (*e.g.,* alpha chain disease, gamma chain disease, mu chain disease); hemangioblastoma; histiocytosis; hypopharynx cancer; inflammatory myofibroblastic tumors; immunocytic amyloidosis; kidney cancer *(e.g.,* nephroblastoma a.k.a. Wilms' tumor, renal cell carcinoma); liver cancer (e.g., hepatocellular cancer (HCC), malignant hepatoma); lung cancer (e.g., bronchogenic carcinoma, small cell lung cancer (SCLC), non-small cell lung cancer (NSCLC), adenocarcinoma of the lung); leiomyosarcoma (LMS); mastocytosis (*e.g*., systemic mastocytosis); melanoma; midline tract carcinoma; multiple endocrine neoplasia syndrome; muscle cancer; myelodysplastic syndrome (MDS); mesothelioma; myeloproliferative disorder (MPD) (*e.g.*, polycythemia vera (PV), essential thrombocytosis (ET), agnogenic myeloid metaplasia (AMM) *a.k.a.* myelofibrosis (MF), chronic idiopathic myelofibrosis, chronic myelocytic leukemia (CML), chronic neutrophilic leukemia (CNL), hypereosinophilic syndrome (HES)); nasopharynx cancer; neuroblastoma; neurofibroma (*e.g.,* neurofibromatosis (NF) type 1 or type 2, schwannomatosis); neuroendocrine cancer (e.g., gastroenteropancreatic neuroendocrine tumor (GEP-NET), carcinoid tumor); osteosarcoma (*e.g.,* bone cancer); ovarian cancer *(e.g.,* cystadenocarcinoma, ovarian embryonal carcinoma, ovarian adenocarcinoma); papillary adenocarcinoma; pancreatic cancer (*e.g.,* pancreatic andenocarcinoma, intraductal papillary mucinous neoplasm (IPMN), Islet cell tumors); parathryroid cancer; papillary adenocarcinoma; penile cancer (*e.g*., Paget's disease of the penis and scrotum); pharyngeal cancer; pinealoma; pituitary cancer; pleuropulmonary blastoma; primitive neuroectodermal tumor (PNT); plasma cell neoplasia; paraneoplastic syndromes; intraepithelial neoplasms; prostate cancer (e.g., prostate adenocarcinoma); rectal cancer; rhabdomyosarcoma; retinoblastoma; salivary gland cancer; skin cancer (*e.g.,* squamous cell carcinoma (SCC), keratoacanthoma (KA), melanoma, basal cell carcinoma (BCC)); small bowel cancer *(e.g.,* appendix cancer); soft tissue sarcoma (*e.g.,* malignant fibrous histiocytoma (MFH), liposarcoma, malignant peripheral nerve sheath tumor (MPNST), chondrosarcoma, fibrosarcoma, myxosarcoma); sebaceous gland carcinoma; stomach cancer; small intestine cancer; sweat gland carcinoma; synovioma; testicular cancer (e.g., seminoma, testicular embryonal carcinoma); thymic cancer; thyroid cancer (*e.g*., papillary carcinoma of the thyroid, papillary thyroid carcinoma (PTC), medullary thyroid cancer); urethral cancer; uterine cancer; vaginal cancer; and vulvar cancer (e.g., Paget's disease of the vulva).

***Carbohydrate polymer:*** The term "carbohydrate polymer" refers to a polymer that is or comprises one or more carbohydrates, e.g., having a carbohydrate backbone. For example, in some cases, a carbohydrate polymer refers to a polysaccharide or an oligosaccharide, or a polymer containing a plurality of monosaccharide units connected by covalent bonds. The monosaccharide units may all be identical, or, in some cases, there may be more than one type of monosaccharide unit present within the carbohydrate polymer. In certain cases, a polymer is naturally occurring. In certain cases, a polymer is synthetic (*i.e.,* not naturally occurring). In some cases, a carbohydrate polymer is a linear polymer. In some cases, a carbohydrate polymer is a branched polymer.

***Chemotherapeutic agent:*** The term "chemotherapeutic agent" refers to a therapeutic agent directed to cancer cells for inhibiting the proliferation of rapidly growing cancer cells and/or killing cancer cells. Examples of such chemotherapeutic agents include, but are not limited to alkylating agents, antimetabolites, topoisomerase inhibitors, and/or mitotic inhibitors.

***Combination therapy:*** As used herein, the term "combination therapy" refers to those situations in which a subject is simultaneously exposed to two or more therapeutic regimens (*e.g.,* two or more therapeutic agents). In some cases, the two or more regimens may be administered simultaneously; in some cases, such regimens may be administered sequentially (*e.g.,* all "doses" of a first regimen are administered prior to administration of any doses of a second regimen); in some cases, such agents are administered in overlapping dosing regimens. In some cases, "administration" of combination therapy may involve administration of one or more agent(s) or modality(ies) to a subject receiving the other agent(s) or modality(ies) in the combination. For clarity, combination therapy does not require that individual agents be administered together in a single composition (or even necessarily at the same time), although in some cases, two or more agents, or active moieties thereof, may be administered together in a combination composition, or even in a combination compound (e.g., as part of a single chemical complex or covalent entity).

***Comparable:*** As used herein, the term "comparable" refers to two or more agents, entities, situations, sets of conditions, *etc.,* that may not be identical to one another but that are sufficiently similar to permit comparison therebetween so that one skilled in the art will appreciate that conclusions may reasonably be drawn based on differences or similarities observed. In some cases, comparable sets of conditions, circumstances, individuals, or populations are characterized by a plurality of substantially identical features and one or a small number of varied features. Those of ordinary skill in the art will understand, in context, what degree of identity is required in any given circumstance for two or more such agents, entities, situations, sets of conditions, *etc.* to be considered comparable. For example, those of ordinary skill in the art will appreciate that sets of circumstances, individuals, or populations are comparable to one another when characterized by a sufficient number and type of substantially identical features to warrant a reasonable conclusion that differences in results obtained or phenomena observed under or with different sets of circumstances, individuals, or populations are caused by or indicative of the variation in those features that are varied.

***Crosslinker:*** As used interchangeably herein, the term "crosslinker" or "crosslinking agent" refers to an agent that links one entity *(e.g.,* one polymer chain) to another entity *(e.g.,* another polymer chain). In some cases, linkage *(i.e.,* the "crosslink") between two entities is or comprises a covalent bond. In some cases, linkage between two entities is or comprises an ionic bond or interaction. In some cases, a crosslinker is a small molecule (*e.g*., dialdehydes or genipin) for inducing formation of a covalent bond between an aldehyde and an amino group. In some cases, a crosslinker comprises a photo-sensitive functional group. In some cases, a crosslinker comprises a pH-sensitive functional group. In some cases, a crosslinker comprises a thermal-sensitive functional group.

***Effective amount:*** An "effective amount" is an amount sufficient to elicit a desired biological response, *e.g*., treating a condition from which a subject may be suffering. As will be appreciated by those of ordinary skill in this art, the effective amount of a composition or an agent included in the composition may vary depending on such factors as the desired biological endpoint, the physical, chemical, and/or biological characteristics (e.g., pharmacokinetics and/or degradation) of agents in the composition, the condition being treated, and the age and health of the subject. An effective amount encompasses therapeutic and prophylactic treatment. For example, in treating cancer, an effective amount may prevent tumor regrowth, reduce the tumor burden, or stop the growth or spread of a tumor. Those skilled in the art will appreciate that an effective amount need not be contained in a single dosage form. Rather, administration of an effective amount may involve administration of a plurality of doses, potentially over time (e.g., according to a dosing regimen).

*Hydrogel:* The term "hydrogel" refers to a material formed from a network of polymer chains that are hydrophilic, sometimes found as a colloidal gel in which an aqueous phase is the dispersion medium. In some cases, hydrogels are highly absorbent (e.g., they can absorb and/or retain over 90% water) natural or synthetic polymeric networks. In some cases, hydrogels possess a degree of flexibility similar to natural tissue, for example due to their significant water content.

***Immunotherapy:*** The term "immunotherapy" refers to a therapeutic agent that promotes the treatment of a disease by inducing, enhancing, or suppressing an immune response. Immunotherapies designed to elicit or amplify an immune response are classified as activation immunotherapies, while immunotherapies that reduce or suppress an immune response are classified as suppression immunotherapies. Immunotherapies are typically, but not always, biotherapeutic agents. Numerous immunotherapies are used to treat cancer. These include, but are not limited to, monoclonal antibodies, adoptive cell transfer, cytokines, chemokines, vaccines, small molecule inhibitors, and small molecule agonists. For example, useful immunotherapies may include, but are not limited to, inducers of type I interferon, interferons, stimulator of interferon genes (STING) agonists, TLR7/8 agonists, IL-15 superagonists, anti-PD-1 antibodies, anti-CD137 antibodies, and anti-CTLA-4 antibodies. In some cases, technologies disclosed herein do not include administration of such immunotherapy as described herein.

***Immunomodulatory payload:*** As used herein, the term "immunomodulatory payload" refers to a separate immunomodulatory agent (*e.g.,* small molecules, polypeptides (including, *e.g.,* cytokines), nucleic acids, *etc*.) that can be carried by or distributed in a biomaterial (*e.g*., a polymeric biomaterial such as ones as disclosed and/or utilized herein), wherein the immunomodulatory agent provides a therapeutic effect of modulating or altering *(e.g.,* inducing, enhancing, or suppressing, *etc*.) one or more aspects of an immune response in a subject. Examples of an immunomodulatory payload include, but are not limited to activators of adaptive immunity, activators of innate immunity, inhibitors of a proinflammatory pathway, immunomodulatory cytokines, or immunomodulatory therapeutic agents as well as ones as described in WO 2018/045058 and PCT/US19/23157, and any combinations thereof. In some cases, an immunomodulatory payload is or comprises an innate immunity modulatory payload *(e.g.,* an immunomodulatory payload that induces or stimulates innate immunity and/or one or more features of innate immunity). In some cases, an innate immunity modulatory payload is or comprises an activator of innate immune response. In some cases, an immunomodulatory payload is or comprises an adaptive immunity modulatory payload, *e.g.,* an activator of adaptive immune response. In some cases, an immunomodulatory payload is or comprises an inhibitor of a proinflammatory pathway, *e.g.,* an inhibitor of proinflammatory immune response mediated by a p38 mitogen-activated protein kinase (MAPK) pathway. In some cases, an immunomodulatory payload is or comprises an immunomodulatory cytokine. In some cases, an immunomodulatory payload is or comprises an immunomodulatory therapeutic agent. In some cases, those of skill in the art, reading the present disclosure, will appreciate that immunomodulatory payloads that are excluded from compositions or pharmaceutical compositions described herein, in some cases, are or comprise small molecules, polypeptides (*e.g.,* cytokines), and/or nucleic acids. As will be understood by those skilled in the art, an immunomodulatory payload does not include components *(e.g.,* precursor components) and/or by-products of a biomaterial (*e.g., a* polymeric biomaterial) agonist of innate immunity (*e.g*., as described and/or utilized herein) generated, *e.g.,* by chemical, enzymatic, and/or biological reactions such as, *e.g.,* degradation.

*Implanting:* The terms "implantable," "implantation," "implanting," and "implant" refer to positioning a composition of interest at a specific location in a subject, such as within a tumor resection site or in a sentinel lymph node, and typically by general surgical methods.

***Increased, Induced, or Reduced:*** As used herein, these terms or grammatically comparable comparative terms, indicate values that are relative to a comparable reference measurement. For example, in some cases, an assessed value achieved with a biomaterial (*e.g.,* a polymeric biomaterial) agonist of innate immunity may be "increased" relative to that obtained with a comparable reference biomaterial (*e.g*., a biomaterial such as a polymeric biomaterial that is not known to induce innate immunity). Alternatively or additionally, in some cases, an assessed value achieved in a subject may be "increased" relative to that obtained in the same subject under different conditions (*e.g*., prior to or after an event; or presence or absence of an event such as administration of a biomaterial agonist of innate immunity, *e.g.*, a polymeric biomaterial agonist of innate immunity), or in a different, comparable subject (*e.g.*, in a comparable subject that differs from the subject of interest in prior exposure to a condition, *e.g*., absence of administration of a biomaterial agonist of innate immunity such as a polymeric biomaterial agonist of innate immunity, *etc.).* In some cases, comparative terms refer to statistically relevant differences (*e.g*., that are of a prevalence and/or magnitude sufficient to achieve statistical relevance). Those skilled in the art will be aware, or will readily be able to determine, in a given context, a degree and/or prevalence of difference that is required or sufficient to achieve such statistical significance.

**Inhibit:** The term "inhibit" or "inhibition" is not limited to only total inhibition. Thus, in some cases, partial inhibition or relative reduction is included within the scope of the term "inhibition." For example, in the context of risk and/or incidence of tumor recurrence and/or metastasis, the term, in some cases, refers to a reduction of the risk or incidence of tumor recurrence and/or metastasis to a level that is reproducibly and/or statistically significantly lower than an initial or other appropriate reference level, which may, for example, be a baseline level of risk or incidence of tumor recurrence and/or metastasis in the absence or prior to administration of a composition described herein. In some cases, the term refers to a reduction of the risk or incidence of tumor recurrence and/or metastasis to a level that is less than 75%, less than 50%, less than 40%, less than 30%, less than 25%, less than 20%, less than 10%, less than 9%, less than 8%, less than 7%, less than 6%, less than 5%, less than 4%, less than 3%, less than 2%, less than 1%, less than 0.5%, less than 0.1%, less than 0.01%, less than 0.001%, or less than 0.0001% of an initial level, which may, for example, be a baseline level of risk or incidence of tumor recurrence and/or metastasis in the absence or prior to administration of a composition described herein.

***Inhibitor:*** As used herein, the term "inhibitor" refers to an agent whose presence or level correlates with decreased level or activity of a target to be modulated. In some cases, an inhibitor may act directly (in which case it exerts its influence directly upon its target, for example by binding to the target); in some cases, an inhibitor may act indirectly (in which case it exerts its influence by interacting with and/or otherwise altering a regulator of a target, so that level and/or activity of the target is reduced). In some cases, an inhibitor is one whose presence or level correlates with a target level or activity that is reduced relative to a particular reference level or activity (e.g., that observed under appropriate reference conditions, such as presence of a known inhibitor, or absence of the inhibitor as disclosed herein, *etc.).*

***Inhibitor of a proinflammatory pathway:*** The term "inhibitor of a proinflammatory pathway" as used herein, in some cases, refers to an agent that inhibits or reduces inflammation that is associated with immunosuppression. In some cases, such an inhibitor of a proinflammatory pathway refers to an agent that prevents recruitment of immunosuppressive cells or prevents acute inflammation. Such acute inflammation and/or recruitment of immunosuppressive cells can occur after local trauma, including that which is caused by surgery. In some cases, an inhibitor of a proinflammatory pathway may inhibit, for example, an immune response that induces inflammation, including, *e.g*., production of inflammatory cytokines (including, *e.g.*, but not limited to TGF-β and IL-10), increased activity and/or proliferation of M2-like macrophages, recruitment of relevant immune cells including, *e.g.,* but not limited to myeloid cells, neutrophils, and mast cells, *etc.* Examples of inhibitors of a proinflammatory pathway include, *e.g.,* ones described in International Application Number PCT/US19/23157. In some cases, those of skill in the art, reading the present disclosure, will appreciate that inhibitors of a proinflammatory pathway that are excluded from compositions or pharmaceutical compositions described herein, in some cases, are or comprise small molecules, polypeptides (*e.g.*, cytokines), and/or nucleic acids.

***Innate immunity modulatory component:*** As used herein, the term "innate immunity modulatory component" refers to a constituent or part of a composition (which, in some cases, may be the entire composition) that modulates or alters (*e.g*., agonizes, antagonizes, activates, reduces, *etc.)* innate immunity (and/or one or more features of an innate immune response) in a subject (*e.g*., in a subject to whom it is administered and/or who is otherwise in need thereof). In some cases, an innate immunity modulatory component is a constituent or part of a composition (which, in some cases, may be the entire composition) that can, indirectly or directly, agonize or activate the innate immune system (and/or one or more features of the innate immune system) of a subject (*e.g*., in a subject to whom it is administered and/or who is otherwise in need thereof). In some cases, an innate immunity modulatory component as described herein can stimulate (*e.g*., can increase level and/or activity) one or more agents that initiate an inflammatory response at a target site, that recruit immune cells to a target site, and/or that help to induce one or more adaptive immune responses (and/or one or more features of an adaptive immune response), *e.g*., leading to the development of antigen-specific acquired immunity. In some cases, an innate immunity modulatory component as described herein can lead to recruitment of relevant immune cells including, *e.g*., but not limited to neutrophils, basophils, eosinophils, natural killer cells, dendritic cells, monocytes, and macrophages, *etc.,* cytokine production, enhanced leukocyte proliferation and/or survival, as well as improved T cell priming, e.g., by augmenting presentation of antigens and/or level and/or activity of co-stimulatory molecules by antigen-presenting cells.

***Metastasis:*** The term "metastasis," "metastatic," or "metastasize" refers to the spread or migration of cancerous cells from a primary or original tumor to another organ or tissue and is typically identifiable by the presence of a "secondary tumor" or "secondary cell mass" of the tissue type of the primary or original tumor and not of that of the organ or tissue in which the secondary (metastatic) tumor is located. For example, a prostate cancer that has migrated to bone is said to be metastasized prostate cancer and includes cancerous prostate cancer cells growing in bone tissue.

***Microparticle:*** As used herein, the term "microparticle" refers to a particle having a diameter between 1 micrometer and 1000 micrometers (µm). In some cases, a microparticle has a diameter of between 1 µm and 500 µm. In some cases, a microparticle has a diameter of between 1 µm and 100 µm.

***Monosaccharide:*** As used herein, the term "monosaccharide" is given its ordinary meaning as used in the art and refers to a simple form of a sugar that consists of a single saccharide unit which cannot be further decomposed to smaller saccharide building blocks or moieties. Common examples of monosaccharides include, e.g., glucose (dextrose), fructose, galactose, mannose, ribose, *etc.* Monosaccharides can be classified according to the number of carbon atoms of the carbohydrate, for example, triose, having 3 carbon atoms such as glyceraldehyde and/or dihydroxyacetone; tetrose, having 4 carbon atoms such as erythrose, threose and/or erythrulose; pentose, having 5 carbon atoms such as arabinose, lyxose, ribose, xylose, ribulose and/or xylulose; hexose, having 6 carbon atoms such as allose, altrose, galactose, glucose, gulose, idose, mannose, talose, fructose, psicose, sorbose and/or tagatose; heptose, having 7 carbon atoms such as mannoheptulose, and/or sedoheptulose; octose, having 8 carbon atoms such as 2-keto-3-deoxy-manno-octonate; nonose, having 9 carbon atoms such as sialose; and decose, having 10 carbon atoms. The above monosaccharides encompass both D- and L-monosaccharides. Alternatively, a monosaccharide can be a monosaccharide variant, in which the saccharide unit comprises one or more substituents other than a hydroxyl. Such variants can be, but are not limited to, ethers, esters, amides, acids, phosphates and amines. Amine variants (*i.e.,* amino sugars) include, for example, glucosamine, galactosamine, fructosamine and/or mannosamine. Amide variants include, for example, N-acetylated amine variants of saccharides (*e.g*., N-acetylglucosamine, and/or N-acetylgalactosamine).

***Nanoparticle:*** As used herein, the term "nanoparticle" refers to a particle having a diameter of less than 1000 nanometers (nm). In some cases, a nanoparticle has a diameter of less than 300 nm. In some cases, a nanoparticle has a diameter of less than 100 nm.

***Pharmaceutically acceptable salt:*** The term "pharmaceutically acceptable salt" refers to those salts which are, within the scope of sound medical judgment, suitable for use in contact with the tissues of, for example, humans and/or animals without undue toxicity, irritation, allergic response, and the like and are commensurate with a reasonable benefit/risk ratio. Pharmaceutically acceptable salts are well known in the art. For example, Berge et al. describe pharmaceutically acceptable salts in detail in J. Pharmaceutical Sciences, 1977, 66, 1-19. Pharmaceutically acceptable salts that may be utilized in accordance with certain cases of the present disclosure may include, for example, those derived from suitable inorganic and organic acids and bases. Examples of pharmaceutically acceptable, non-toxic acid addition salts are salts of an amino group formed with inorganic acids, such as hydrochloric acid, hydrobromic acid, phosphoric acid, sulfuric acid, and perchloric acid or with organic acids, such as acetic acid, oxalic acid, maleic acid, tartaric acid, citric acid, succinic acid, or malonic acid or by using other methods known in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium, and N⁺(C₁₋C₄ alkyl)₄⁻ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

***Polymer:*** The term "polymer" is given its ordinary meaning as used in the art, *i.e.,* a molecular structure comprising one or more repeat units (monomers), connected by covalent bonds. The repeat units may all be identical, or, in some cases, there may be more than one type of repeat unit present within the polymer (*e.g.,* in a copolymer). In certain cases, a polymer is naturally occurring. In certain cases, a polymer is synthetic (*i.e.,* not naturally occurring). In some cases, a polymer is a linear polymer. In some cases, a polymer is a branched polymer. In some cases, a polymer for use in accordance with the present disclosure is not a polypeptide. In some cases, a polymer for use in accordance with the present disclosure is not a nucleic acid.

***Polymeric biomaterial:*** A "polymeric biomaterial", as described herein, is a material that is or comprises a polymer or a polymeric moiety and is biocompatible. In many cases, a polymeric biomaterial is or includes at least one polymer; in some cases, a polymer may be or comprise a copolymer. In some cases, a polymeric biomaterial is or comprises a preparation of a particular polymer (*e.g.,* of chitosan). Those skilled in the art will be aware that certain polymers may exist and/or be available in a variety of forms (*e.g.,* length, molecular weight, charge, topography, surface chemistry, degree and/or type of modification such as alkylation, acylation, quaternization, hydroxyalkylation, carboxyalkylation, thiolation, phosphorylation, glycosylation, *etc*.); in some cases, a preparation of such a polymer may include a specified level and/or distribution of such form or forms. Additionally or alternatively, those skilled in the art will appreciate that, in some cases, one or more immunomodulatory properties of a polymeric biomaterial may be tuned by its biomaterial property(ies), including, *e.g.,* surface chemistry of a polymeric biomaterial (*e.g.,* modulated by hydrophobicity and/or hydrophilicity portions of a polymeric biomaterial, chemical moieties, and/or charge characteristics) and/or topography of a polymeric biomaterial (*e.g*., modulated by size, shape, and/or surface texture), for example as described in Mariani et al. "Biomaterials: Foreign Bodies or Tuners for the Immune Response?" International Journal of Molecular Sciences, 2019, 20, 636.

***Polymer network:*** The term "polymer network" is used herein to describe an assembly of polymer chains interacting with each other. In some cases, a polymer network forms a three-dimensional structure material. In some cases, a polymer network may be formed by linking polymer chains ("crosslinked polymer network") using a crosslinker (e.g., as described herein), *e.g.,* to produce a covalent polymer network. In some cases, a polymer network may be formed simply by intermingling or blending polymer chains in a mixture ("non-crosslinked polymer network"). In some cases, such a non-crosslinked polymer network may be formed by non-covalent or non-ionic intermolecular association of polymer chains, e.g., through hydrogen bonding. In some cases, a polymer network may be formed by a combination of crosslinking polymer chains and non-covalent or non-ionic intermolecular association of polymer chains.

***Proinflammatory cytokine:*** As used herein, the term "proinflammatory cytokine" refers to a protein or glycoprotein molecule secreted by a cell (*e.g.,* a cell of an immune system) that induces an inflammatory response. As will be appreciated by one of skilled in the art, inflammation may be immunostimulatory or immunosuppressive depending on the biological context.

***Proinflammatory immune response:*** The term "proinflammatory immune response" as used herein refers to an immune response that induces inflammation, including, *e.g.,* production of proinflammatory cytokines (including, *e.g.,* but not limited to CXCL10, IFN-α, IFN-β, IL-1β, IL-6, IL-18, and/or TNF-alpha), increased activity and/or proliferation of Th1 cells, recruitment of myeloid cells, *etc.* In some cases, a proinflammatory immune response may be or comprise one or both of acute inflammation and chronic inflammation.

***Prophylactically effective amount:*** A "prophylactically effective amount" is an amount sufficient to prevent (*e.g*., significantly delay onset or recurrence of one or more symptoms or characteristics of, for example so that it/they is/are not detected at a time point at which they would be expected absent administration of the amount) a condition. A prophylactically effective amount of a composition means an amount of therapeutic agent(s), alone or in combination with other agents, that provides a prophylactic benefit in the prevention of the condition. The term "prophylactically effective amount" can encompass an amount that improves overall prophylaxis or enhances the prophylactic efficacy of another prophylactic agent. Those skilled in the art will appreciate that a prophylactically effective amount need not be contained in a single dosage form. Rather, administration of an effective amount may involve administration of a plurality of doses, potentially over time *(e.g.,* according to a dosing regimen).

***Small molecule:*** The term "small molecule" or "small molecule therapeutic" refers to a molecule, whether naturally occurring or artificially created (*e_{.}g.,* via chemical synthesis) that has a relatively low molecular weight. Typically, a small molecule is an organic compound (i*.e.,* it contains carbon). The small molecule may contain multiple carbon-carbon bonds, stereocenters, and other functional groups (*e.g*., amines, hydroxyl, carbonyls, and heterocyclic rings, *etc.).* In certain cases, the molecular weight of a small molecule is not more than about 1,000 g/mol, not more than about 900 g/mol, not more than about 800 g/mol, not more than about 700 g/mol, not more than about 600 g/mol, not more than about 500 g/mol, not more than about 400 g/mol, not more than about 300 g/mol, not more than about 200 g/mol, or not more than about 100 g/mol. In certain cases, the molecular weight of a small molecule is at least about 100 g/mol, at least about 200 g/mol, at least about 300 g/mol, at least about 400 g/mol, at least about 500 g/mol, at least about 600 g/mol, at least about 700 g/mol, at least about 800 g/mol, or at least about 900 g/mol, or at least about 1,000 g/mol. Combinations of the above ranges (*e.g.,* at least about 200 g/mol and not more than about 500 g/mol) are also possible. In certain cases, a small molecule is a therapeutically active agent such as a drug (*e.g.,* a molecule approved by the U.S. Food and Drug Administration as provided in the Code of Federal Regulations (C.F.R.)). A small molecule may also be complexed with one or more metal atoms and/or metal ions. In this instance, the small molecule is also referred to as a "small organometallic molecule." Preferred small molecules are biologically active in that they produce a biological effect in animals, preferably mammals, more preferably humans. Small molecules include, but are not limited to, radionuclides and imaging agents. In certain cases, a small molecule is a drug. Preferably, though not necessarily, the drug is one that has already been deemed safe and effective for use in humans or animals by the appropriate governmental agency or regulatory body. For example, drugs approved for human use are listed by the FDA under 21 C.F.R. §§ 330.5, 331 through 361, and 440 through 460; drugs for veterinary use are listed by the FDA under 21 C.F.R. §§ 500 through 589. All listed drugs are considered acceptable for use in accordance with the present invention.

***Subject:*** A "subject" to which administration is contemplated includes, but is not limited to, a human (*i.e.,* a male or female of any age group, *e.g.,* a pediatric subject (*e.g.,* infant, child, adolescent) or adult subject (*e.g*., young adult, middle-aged adult, or senior adult)) and/or a non-human animal, for example, a mammal (*e.g.,* a primate (*e.g.,* cynomolgus monkey, rhesus monkey); a domestic animal such as a cow, pig, horse, sheep, goat, cat, and/or dog; and/or a bird *(e.g.,* a chicken, duck, goose, and/or turkey). In certain cases, the animal is a mammal (*e.g*., at any stage of development). In some cases, an animal (*e.g.,* a non-human animal) may be a transgenic or genetically engineered animal. In some cases, a subject is a tumor resection subject, *e.g.,* a subject who has recently undergone tumor resection. In some cases, a tumor resection subject is a subject who has undergone tumor resection in less than 72 hours (including, *e.g*., less than 48 hours, less than 24 hours, less than 12 hours, less than 6 hours, or lower) prior to receiving a composition described herein. In some cases, a tumor resection subject is a subject who has undergone tumor resection in less than 48 hours prior to receiving a composition described herein. In some cases, a tumor resection subject is a subject who has undergone tumor resection in less than 24 hours prior to receiving a composition described herein. In some cases, a tumor resection subject is a subject who has undergone tumor resection in less than 12 hours prior to receiving a composition described herein.

***Substantially:*** As used herein, the term "substantially" refers to the qualitative condition of exhibiting total or near-total extent or degree of a characteristic or property of interest. Those skilled in the art will understand that an agent of interest, if ever, achieves or avoids an absolute result, *e.g.,* an agent of interest that indeed has zero effect on an immune response, e.g., inflammation. The term "substantially" is therefore used herein to capture the potential lack of absoluteness inherent in many biological and chemical effects.

***Sustained:*** As used interchangeably herein, the term "sustained" or "extended" typically refers to prolonging an effect and/or a process over a desirable period of time. For example, in the context of sustained stimulation of innate immunity (*e.g.*, in the presence of a biomaterial agonist of innate immunity), such an effect induced by a biomaterial of interest may be observed for a longer period of time, as compared to that which is observed when such a biomaterial of interest is not utilized. In the context of sustained release of one or more agents of interest (*e.g.*, therapeutic agents as described herein and/or degradation or dissolution products and/or soluble components of a biomaterial agonist of innate immunity) from a biomaterial agonist of innate immunity over a period of time, such release may occur on a timescale ranging from 30 minutes to several weeks. In some cases, the extent of sustained release or extended release can be characterized *in vitro* or *in vivo.* For example, in some cases, the release kinetics can be tested *in vitro* by placing a composition described herein in an aqueous buffered solution (*e.g*., PBS at pH 7.4). In some cases, when a composition described herein is placed in an aqueous buffered solution (*e.g*., PBS at pH 7.4), less than 100% or lower (including, *e.g*., less than or equal to 90%, less than or equal to 80%, less than or equal to 70%, less than or equal to 50% or lower) of one or more agents of interest (*e.g.,* therapeutic agents such as ones disclosed herein and/or degradation or dissolution products and/or soluble components of a biomaterial that mediate or activate innate immunity) is released within 3 hours from a biomaterial. In some cases, the release kinetics can be tested *in vivo* by implanting a composition at a target site (*e.g.,* mammary fat pad) of an animal subject (*e.g.,* a mouse subject). In some cases, when a composition is implanted at a target site (*e.g.,* mammary fat pad) of an animal subject (*e.g.,* a mouse subject), less than or equal to 70% or lower (including, *e.g.*, less than or equal to 60%, less than or equal to 50%, less than 40%, less than 30% or lower) of one or more agents of interest (*e.g.*, therapeutic agents such as ones disclosed herein and/or degradation or dissolution products and/or soluble components of a biomaterial that mediate or activate innate immunity) is released *in vivo* 8 hours after the implantation.

***Test subject:* A**s used herein, the term "test subject" refers to a subject to which technologies disclosed herein are applied for experimental investigation, e.g., to assess biomaterial degradation, and/or efficacy of biomaterials in innate immunity agonism and/or antitumor immunity. In some cases, a test subject may be a human subject or across a population of human subjects. For example, in some cases, such a human test subject may be a normal healthy subject. In some cases, such a human test subject may be a tumor resection subject. In some cases, a test subject may be a mammalian non-human animal or across a population of mammalian non-human animals. Non-limiting examples of such mammalian non-human animals include mice, rats, dogs, pigs, rabbits, *etc.,* which in some cases may be normal healthy subjects, while in some cases may be tumor resection subjects. In some cases, such mammalian non-human animals may be transgenic or genetically engineered animals.

***Therapeutic agent:*** The term "therapeutic agent" refers to an agent having one or more properties that produce a desired, usually beneficial, physiological effect. For example, a therapeutic agent may treat, ameliorate, and/or prevent disease. Those skilled in the art, reading the present disclosure, will appreciate that the term "therapeutic agent", as used herein, does not require a particular level or type of therapeutic activity, such as might be required for a regulatory agency to consider an agent to be "therapeutically active" for regulatory purposes. As will be understood by those skill in the art, reading the present disclosure, in some cases, a biomaterial (*e.g.,* a polymeric biomaterial) agonist of innate immunity may have one or more properties that contribute to and/or achieve a desired physiological effect, and therefore may be considered to be a "therapeutic agent" as that term is used here (whether or not such biomaterial would or would not be considered to be pharmaceutically active by any particular regulatory agency). In some cases, a therapeutic agent that may be utilized in compositions and/or methods described herein (*e.g*., involving a biomaterial (*e.g*., a polymeric biomaterial) agonist of innate immunity) may be a non-immunomodulatory therapeutic agent, *e.g*., comprising a biologic, a small molecule, or a combination thereof. In some cases, a therapeutic agent that may be utilized in compositions and/or methods described herein *(e.g.,* involving a biomaterial (*e.g.,* a polymeric biomaterial) agonist of innate immunity) may be or comprise a chemotherapeutic agent, which in some cases may be or comprise a cytotoxic agent. In some cases, a therapeutic agent that may be utilized in compositions and/or methods described herein *(e.g.,* involving a biomaterial *(e.g.,* a polymeric biomaterial) agonist of innate immunity) does not comprise an immunomodulatory payload *(e.g.,* as described herein).

***Therapeutically effective amount:*** A "therapeutically effective amount" is an amount sufficient to provide a therapeutic benefit in the treatment of a condition, which therapeutic benefit may be or comprise, for example, reduction in frequency and/or severity, and/or delay of onset of one or more features or symptoms associated with the condition. A therapeutically effective amount means an amount of therapeutic agent(s), alone or in combination with other therapies, that provides a therapeutic benefit in the treatment of the condition. The term "therapeutically effective amount" can encompass an amount that improves overall therapy, reduces or avoids symptoms or causes of the condition, or enhances the therapeutic efficacy of another therapeutic agent. Those skilled in the art will appreciate that a therapeutically effective amount need not be contained in a single dosage form. Rather, administration of an effective amount may involve administration of a plurality of doses, potentially over time (*e.g*., according to a dosing regimen).

*Treat:* The terms "treatment," "treat," and "treating" refer to reversing, alleviating, delaying the onset of, or inhibiting the progress of a "pathological condition" *(e.g.,* a disease, disorder, or condition, including one or more signs or symptoms thereof) described herein, *e.g*., cancer or tumor. In some cases, treatment may be administered after one or more signs or symptoms have developed or have been observed. Treatment may also be continued after symptoms have resolved, for example, to delay or prevent recurrence and/or spread.

***Tumor:*** The terms "tumor" and "neoplasm" are used herein interchangeably and refer to an abnormal mass of tissue wherein the growth of the mass surpasses and is not coordinated with the growth of a normal tissue. A neoplasm or tumor may be "benign" or "malignant," depending on the following characteristics: degree of cellular differentiation (including morphology and functionality), rate of growth, local invasion, and metastasis. A "benign neoplasm" is generally well differentiated, has characteristically slower growth than a malignant neoplasm, and remains localized to the site of origin. In addition, a benign neoplasm does not have the capacity to infiltrate, invade, or metastasize to distant sites. Exemplary benign neoplasms include, but are not limited to, lipoma, chondroma, adenomas, acrochordon, senile angiomas, seborrheic keratoses, lentigos, and sebaceous hyperplasias. In some cases, certain "benign" tumors may later give rise to malignant neoplasms, which may result from additional genetic changes in a subpopulation of the tumor's neoplastic cells, and these tumors are referred to as "pre-malignant neoplasms." An example of a pre-malignant neoplasm is a teratoma. In contrast, a "malignant neoplasm" is generally poorly differentiated (anaplasia) and has characteristically rapid growth accompanied by progressive infiltration, invasion, and destruction of the surrounding tissue. Furthermore, a malignant neoplasm generally has the capacity to metastasize to distant sites.

***Tumor removal:*** As used herein, the term "tumor removal" encompasses partial or complete removal of a tumor, which may be resulted from a cancer therapy, e.g., surgical resection. **In** some cases, tumor removal refers to physical removal of part or all of a tumor by surgery (*i.e.,* "tumor resection"). **In** some cases, tumor removal may be resulted from a surgical tumor resection and an adjuvant therapy (*e.g*., chemotherapy, immunotherapy, and/or radiation therapy). **In** some cases, an adjuvant therapy may be administered after a surgical tumor resection, e.g., at least 24 hours or more after a surgical tumor resection.

***Tumor resection subject:*** As used herein, the term "tumor resection subject" refers to a subject who is undergoing or has recently undergone a tumor resection procedure. **In** some cases, a tumor resection subject is a subject who has at least 70% or more (including, at least 80%, at least 90%, at least 95%, at least 98%, at least 99%, or higher (including 100%) of gross tumor mass removed by surgical resection. Those of skill in the art will appreciate that, in some cases, there may be some residual cancer cells microscopically present at a visible resection margin even though gross examination by the naked eye shows that all of the gross tumor mass has been apparently removed. **In** some cases, a tumor resection subject may be determined to have a negative resection margin *(i.e.,* no cancer cells seen microscopically at the resection margin, e.g., based on histological assessment of tissues surrounding the tumor resection site). **In** some cases, a tumor resection subject may be determined to have a positive resection margin (*i.e.,* cancer cells are seen microscopically at the resection margin, e.g., based on histological assessment of tissues surrounding the tumor resection site). **In** some cases, a tumor resection subject may have micrometastases and/or dormant disseminated cancer cells that can be driven to progress/proliferate by the physiologic response to surgery. **In** some cases, a tumor resection subject receives a composition (*e.g.*, as described and/or utilized herein) immediately after the tumor resection procedure is performed (*e.g.*, intraoperative administration). **In** some cases, a tumor resection subject receives a composition (*e.g*., as described and/or utilized herein) postoperatively within 24 hours or less, including, *e.g*., within 18 hours, within 12 hours, within 6 hours, within 3 hours, within 2 hours, within 1 hour, within 30 mins, or less.

***Variant:*** As used herein, the term "variant" refers to an entity that shows significant structural identity with a reference entity but differs structurally from the reference entity in the presence or level of one or more chemical moieties as compared with the reference entity. **In** many cases, a variant also differs functionally from its reference entity. In general, whether a particular entity is properly considered to be a "variant" of a reference entity is based on its degree of structural identity with the reference entity. As will be appreciated by those skilled in the art, any biological or chemical reference entity has certain characteristic structural elements. A variant, by definition, is a distinct chemical entity that shares one or more such characteristic structural elements. To give but a few examples, a small molecule may have a characteristic core structural element (*e.g.,* a macrocycle core) and/or one or more characteristic pendent moieties so that a variant of the small molecule is one that shares the core structural element and the characteristic pendent moieties but differs in other pendent moieties and/or in types of bonds present (single vs double, E vs Z, *etc*.) within the core, a polypeptide may have a characteristic sequence element comprised of a plurality of amino acids having designated positions relative to one another in linear or three-dimensional space and/or contributing to a particular biological function, a nucleic acid may have a characteristic sequence element comprised of a plurality of nucleotide residues having designated positions relative to on another in linear or three-dimensional space. For example, a variant biomaterial (*e.g*., a variant polymeric biomaterial) may differ from a reference biomaterial (*e.g*., a reference polymeric biomaterial) as a result of one or more structural modifications (*e.g*., but not limited to, additions, deletions, and/or modifications of chemical moieties, and/or grafting) provided that the variant biomaterial (*e.g*., variant polymeric biomaterial) can act on an immune system (*e.g.,* by stimulating innate immunity), *e.g.,* when used in a method described herein. In some cases, a variant biomaterial (*e.g*., a variant polymeric biomaterial) is characterized in that, when assessed at 24 hours after administration of such a variant biomaterial (*e.g.*, a variant polymeric biomaterial) to a target site in a subject, an amount of one or more proinflammatory cytokines (e.g., but not limited to CXCL10, IFN-α, IFN-β, IL-1β, IL-6, IL-18, and/or TNF-α) observed at the target site and/or body circulation of the subject is at least 60% or more (*e.g.,* including, *e.g.,* at least 70%, at least 80%, at least 90%, at least 95%, at least 98%, or up to 100%) of that observed when a reference biomaterial (*e.g.,* a reference polymeric biomaterial) is administered at the target site. In some cases, a variant biomaterial (*e.g*., a variant polymeric biomaterial) is characterized in that, when assessed at 24 hours after administration of such a variant biomaterial (*e.g*., a variant polymeric biomaterial) to a target site in a subject, an amount of one or more proinflammatory cytokines (*e.g*., but not limited to CXCL10, IFN-α, IFN-β, IL-1β, IL-6, IL-18, and/or TNF-α) observed at the target site and/or body circulation of the subject is at least 1.1-fold or more *(e.g.,* including, *e.g.,* at least 1.5-fold, at least 2-fold, at least 3-fold, at least 4-fold, at least 5-fold, or more) of that observed when a reference biomaterial (*e.g.,* a reference polymeric biomaterial) is administered at the target site. In some cases, a variant biomaterial (*e.g.,* a variant polymeric biomaterial) exhibits at least one physical characteristic that is different from that of a reference biomaterial (*e.g*., a reference polymeric biomaterial). For example, in some cases, a variant biomaterial (*e.g.*, a variant polymeric biomaterial) can exhibit increased water solubility (*e.g.,* at a physiological pH) as compared to that of a reference biomaterial (*e.g*., a reference polymeric biomaterial). In some cases, a variant has 10, 9, 8, 7, 6, 5, 4, 3, 2, or 1 structural modifications as compared with a reference. In some cases, a variant has a small number (*e.g*., fewer than 5, 4, 3, 2, or 1) number of structural modifications (*e.g.*, alkylation, acylation, quaternization, hydroxyalkylation, carboxyalkylation, thiolation, phosphorylation, glycosylation, *etc*.). In some cases, a variant has not more than 5, 4, 3, 2, or 1 additions or deletions of chemical moieties, and in some cases has no additions or deletions, as compared with a reference. In some cases, a variant is an entity that can be generated from a reference by chemical manipulation. In some cases, a variant is an entity that can be generated through performance of a synthetic process substantially similar to (*e.g.,* sharing a plurality of steps with) one that generates a reference.

### Detailed Description

The detailed reference technical disclosure set out below may in some respects go beyond the disclosure of the invention *per se,* and may also provide technical background for related technical developments, as illustrated *inter alia* in the reference examples. It will therefore be appreciated that this reference technical disclosure is not intended to define the invention as such, but rather to place it in a broader technical context. The present disclosure, among other things, concerns technologies relating to cancer treatment and/or to achieving local modulation, *in situ,* of an immunological event or process in a body.

In some cases, disclosed technologies are particularly useful for treating cancer patients who have recently undergone and/or are undergoing tumor removal (partial or complete), *e.g.,* by surgical resection. In particular cases, disclosed technologies can be useful for treating tumor resection subjects, *e.g.,* treating tumors such as, *e.g.,* solid tumors, in an intraoperative setting. Surgical resection of tumors can result in immunosuppression, e.g., immunosuppression locally at or near the resection site (*e.g.,* in response to wound healing) and/or immunosuppression at one or more remote sites such as, for example, one or more remote tumor sites, *e.g.,* sites of metastasis (*e.g.,* resulted from systemic diffusion of immunosuppressive factors). See, *e.g.,* Krall et al. "The systemic response to surgery triggers the outgrowth of distant immune-controlled tumors in mouse models of dormancy" Sci Transl Med., 10:eaan3464 (2018). Such changes in immune responses that may occur at a surgical site following tumor resection can have local and/or systemic consequences and might promote or facilitate activation of dormant micrometastases and/or propagation of residual cancer cells, thus increasing the risk of cancer recurrence. See, *e.g.,* Tohme et al. "Surgery for cancer: a trigger for metastases" Cancer Res., 77:1548-1552 (2017).

In some cases, the present disclosure provides insights that certain types of biomaterials (e.g., polymeric biomaterials) that are not previously considered as driving efficacy of a cancer treatment, but rather as an auxiliary material (e.g., a drug carrier or a vaccine adjuvant), can be useful for inducing antitumor immunity by themselves and thus do not necessarily require a separate immunomodulatory payload, especially in the post-tumor resection context, e.g., where there are very few or no detectable remaining cancer cells. Accordingly, the present disclosure teaches treatment strategies that are particularly effective and/or desirable for such and other biomaterials including polymeric biomaterials.

In particular cases, the present disclosure provides insights that certain biomaterials (*e.g.,* polymeric biomaterials) by themselves, when placed at a target site (*e.g., a* site at which a tumor has been removed, *e.g.,* by surgical resection), can be sufficient to stimulate innate antitumor immunity. In some cases, such innate antitumor immunity can lead to a reduction in the risk and/or incidence of cancer recurrence and/or metastasis and/or mortality related thereto. Thus, the present disclosure concerns technologies that can concentrate the immunostimulatory action to a target site in need thereof. Such technologies disclosed herein can be particularly useful for treating cancer, for example, by reducing or inhibiting (e.g., delaying onset of, reducing extent of) tumor recurrence and/or metastasis, in some cases while minimizing adverse side effects that may be associated with systemic administration of immunotherapies.

### I. Compositions

The present disclosure concerns immunomodulatory compositions as described herein (*e.g.,* that comprise an immunomodulatory agent consisting essentially of (or consisting of) a biomaterial, including, *e.g.,* a polymeric biomaterial, characterized in that it is an agonist of innate immunity as described herein (*e.g*., when tested in one or more assays as described herein)).

In some cases, the present disclosure concerns compositions (e.g., immunomodulatory compositions as described herein) that are useful for cancer treatment. In particular cases, disclosed compositions are useful for administration to subjects who are undergoing or have undergone tumor removal. For example, in some cases, disclosed compositions are useful for administration to tumor resection subjects.

In some cases, compositions in accordance with the present disclosure include an innate immunity modulatory component. For example, in some cases, such an innate immunity modulatory component activates or stimulates one or more innate immune responses (and/or one or more features of an innate immune response) in a subject (e.g., in a subject to whom it is administered and/or who is otherwise in need thereof). In some cases, an innate immunity modulatory component provided in a composition, directly or indirectly, induces an inflammatory response at a target site (*e.g.,* a tumor resection site) and/or recruits immune cells to a target site (*e.g.,* a tumor resection site). Those skilled in the art will appreciate that, in some cases, an innate immunity modulatory component provided in a composition as described herein can stimulate (*e.g.*, can increase level and/or activity of) one or more agents that initiate an inflammatory response at a target site and/or that recruit immune cells to a target site.

In some cases, a disclosed composition comprises an innate immunity modulatory component and, optionally, one or more other components *(i.e.,* one or more materials/agents that is not an innate immunity modulatory agent).

In some cases, an innate immunity modulatory component consists essentially of or consists of a biomaterial. In some cases, an innate immunity modulatory component consists essentially of or consists of a polymeric biomaterial. Thus, in some cases, the innate immunity modulatory component of a composition as disclosed herein consists essentially of or consists of such a biomaterial (*e.g*., a polymeric biomaterial); to the extent that such a composition may include one or more material(s)/agent(s) other than the biomaterial (*e.g*., polymeric biomaterial), such other material(s)/agent(s) do not, individually or together, materially alter relevant innate immunity modulatory characteristic(s) of the biomaterial (*e.g.*, polymeric biomaterial).

The present inventor has previously described a system comprising a biomaterial and a payload that may be or comprise an innate immunity modulatory agent (see, for example, an activator of innate immune response as described in WO 2018/045058) can be remarkably useful, among other things, when administered to subjects who have undergone or are undergoing a tumor resection. Among other things, the present disclosure provides the surprising insight that certain biomaterials (*e.g*., polymeric biomaterials) may be able to provide sufficient innate immunity modulatory activity to achieve beneficial effects even absent a separate innate immunity modulatory payload.

In some cases, not only is an innate immunity modulatory component of a composition described and/or utilized herein substantially free of an innate immunity modulatory payload, but also such a composition of the present disclosure may not necessarily require inclusion of at least one or more (*e.g.,* at least two or more, at least three or more) other types of immunomodulatory payloads, including, *e.g*., adaptive immunity modulatory payloads, immunomodulatory cytokines, immunomodulatory chemotherapeutics, immunomodulatory therapeutic agents, and/or combinations thereof. By way of example only, in some cases, a composition of the present disclosure is substantially free of an innate immunity modulatory payload and an adaptive immunity modulatory payload. In some cases, a composition of the present disclosure is substantially free of an innate immunity modulatory payload, an adaptive immunity modulatory payload, and an immunomodulatory cytokine. In some cases, a composition of the present disclosure comprises a biomaterial (*e.g.,* a polymeric biomaterial) agonist of immunity in the absence of an immunomodulatory payload.

### Biomaterial (e.g., polymeric biomaterial) agonist of innate immunity

A biomaterial agonist of innate immunity for use in accordance with the present disclosure is a biomaterial that, upon administration, functions as an agonist or activator of innate immunity. In some cases, such a biomaterial agonist of innate immunity is or comprises a polymeric biomaterial agonist of innate immunity. As will be appreciated by those skilled in the art, reading the present disclosure, a polymeric biomaterial utilized in accordance with the present disclosure is a matrix biomaterial (*e.g.,* in some cases with one or more biomaterial properties as described herein, including, *e.g.,* storage modulus, viscosity, and/or phase angle), rather than individual nucleic acid or polypeptide molecules. In some cases, a polymer for use in the context of such a polymeric biomaterial is not a polypeptide. In some cases, a polymer for use in the context of a polymeric biomaterial is not a nucleic acid. In some cases, a polymeric biomaterial may be or comprise a nucleic acid and/or a polypeptide.

In some cases, a biomaterial (*e.g.*, a polymeric biomaterial) agonist of innate immunity disclosed and/or utilized herein can, indirectly or directly, activate one or more pattern recognition receptors of one or more types of cells of an innate immune system, such as, e.g., dendritic cells, macrophages, monocytes, neutrophils, and/or natural killer (NK) cells, such that at least one or more innate immune responses are induced (*e.g.,* as described herein). Examples of such a pattern recognition receptor is or comprises a C-type Lectin Receptor (CLR), a Nucleotide-binding Oligomerization Domain-Like Receptor (NOD-Like receptor or NLR), a Retinoic acid-inducible gene-I-Like Receptor (RLR), and/or a Toll-Like Receptor (TLR). In some cases, a biomaterial (e.g., a polymeric biomaterial) agonist of innate immunity utilized herein can, directly or indirectly, activate at least one or more C-type Lectin Receptors (CLRs) of many different cells of an innate immune system (*e.g.,* dendritic cells, macrophages, *etc*.), which include, *e.g.,* mannose receptors, and/or asialoglycoprotein receptor family (*e.g.*, Dectin-1, Dectin-2, macrophage-inducible C-type lectin (Mincle), dendritic cell-specific ICAM3-grabbing nonintegrin (DC-SIGN), and DC NK lectin group receptor-1 (DNGR-1)). In some cases, a biomaterial (*e.g*., a polymeric biomaterial) agonist of innate immunity utilized herein can, directly or indirectly, activate at least one or more NOD-Like Receptors (NLRs) of different types of leukocytes (*e.g*., lymphocytes, macrophages, dendritic cells), which include, *e.g.,* NLRA (*e.g.,* CIITA), NLRB (*e.g.,* NAIP), NLRC *(e.g.,* NOD1, NOD2, NLRC3, NLRC4, NLRC5, NLRX1) and/or NLRP (*e.g.,* NLRP1, NLRP2, NLRP3, NLRP4, NLRP5, NLRP6, NLRP7, NLRP8, NLRP9, NLRP10, NLRP11, NLRP12, NLRP13, NLRP14). In some cases, a biomaterial (*e.g.*, a polymeric biomaterial) agonist of innate immunity utilized herein can, directly or indirectly, activate at least one or more RIG-I-Like Receptors (RLRs) of, *e.g.,* myeloid cells, which include, *e.g.,* RIG-I, MDA5, and/or LGP2. In some cases, a biomaterial (*e.g*., a polymeric biomaterial) agonist of innate immunity for use in accordance with the present disclosure can, directly or indirectly, activate at least one or more Toll-Like Receptors (TLRs) of different types of leukocytes (*e.g.,* dendritic cells, myeloid dendritic cells, monocytes, macrophages, and/or neutrophils), which include, *e.g.,* TLR1, TLR2, TLR3, TLR4, TLR5, TLR6, TLR7, TLR8, TLR9, and/or TLR10.

In some cases, a biomaterial (*e.g.*, polymeric biomaterial) agonist of innate immunity disclosed and/or utilized herein can, indirectly or directly, activate or induce (*e.g.,* increase level and/or activity of) an inflammasome, *e.g.*, in myeloid cells, such that at least one or more innate immune responses (and/or one or more features of an innate immune response) are induced (*e.g.,* as described herein). In some cases, an inflammasome is typically a multi-protein complex that activates one or more inflammatory responses, such as, e.g., promoting maturation and/or secretion of one or more proinflammatory cytokines such as, *e.g.,* interleukin 1β and/or interleukin 18. In some cases, a biomaterial (*e.g.,* polymeric biomaterial) agonist of innate immunity disclosed and/or utilized herein can, indirectly or directly, activate or induce (*e.g*., increase level and/or activity of) an inflammasome comprising an Absent in Melanoma 2 (AIM2)-Like Receptor ("AIM2 inflammasome"). In some cases, a biomaterial (*e.g*., polymeric biomaterial) agonist of innate immunity disclosed and/or utilized herein can, indirectly or directly, activate or induce (*e.g*., increase level and/or activity of) an inflammasome comprising one or more NLRs, including, *e.g.,* NLRP1 (*e.g.,* NALP1b), NLRP3 (*e.g.,* NALP3), and/or NLRC4 (*e.g.,* IPAF).

In some cases, a biomaterial (e.g., a polymeric biomaterial) agonist of innate immunity for use in accordance with the present disclosure can, directly or indirectly, activate one or more components involved in a cGAS-STING pathway (*e.g.*, a cGAS-STING pathway and/or components thereof as described in Chen et al., "Regulation and function of the cGAS-STING pathway of cytosolic DNA sensing" Nature Immunology (2016) 17: 1142-1149), such that innate immunity is induced. In some cases, a biomaterial (e.g., a polymeric biomaterial) agonist of innate immunity for use in accordance with the present disclosure can, directly or indirectly, induce activity and/or level of NFκB and/or other components associated with an NFκB pathway (*e.g*., NFκB activation during innate immune response, *e.g.,* as described in Dev et al., "NF-κB and innate immunity" Curr Top Microbiol Immunol. (2011) 349: 115-43). In some cases, a biomaterial (*e.g.*, a polymeric biomaterial) agonist of innate immunity for use in accordance with the present disclosure can, directly or indirectly, lead to production of reactive oxygen species, *e.g*., during innate immune response.

As will be clear to one of those skilled in the art reading the present disclosure, in some cases, a biomaterial (*e.g*., a polymeric biomaterial) agonist of innate immunity disclosed and/or utilized herein can, directly or indirectly, activate one or more of components and/or pathways (*e.g*., ones as described herein) associated with activation of innate immunity. For example, in some cases, a biomaterial (*e.g*., a polymeric biomaterial) agonist of innate immunity disclosed and/or utilized herein can, directly or indirectly, activate one or more pattern recognition receptors of one or more types of cells of an innate immune system *(e.g.,* ones as described herein) and also activate or induce (*e.g.,* increase level and/or activity of) an inflammasome, *e.g*., in myeloid cells.

For example, in some cases, a biomaterial (*e.g.*, polymeric biomaterial) agonist of innate immunity disclosed and/or utilized herein is characterized in that, when assessed at an specified time (*e.g.,* 24 hours, 48 hours, or 72 hours) after administration to a target site in a subject in need thereof (*e.g.,* as described herein), more proinflammatory cytokine(s) is present at a target site and/or in body circulation of the subject than that which is observed when the biomaterial (*e.g*., polymeric biomaterial) agonist of innate immunity is not administered to the target site. Non-limiting examples of such proinflammatory cytokine(s) include CXCL10, IFN-α, IFN-β, IL-1β, IL-6, IL-18, TNF-α, and combinations thereof. In some cases, such an increase in one or more (*e.g.,* 1, 2, 3, 4, 5, 6, or more) proinflammatory cytokine(s) at a target site (*e.g.,* a tumor resection site) and/or in body circulation (*e.g.,* peripheral blood) is at least 10% or higher (including, *e.g.,* at least 20%, at least 30%, at least 40%, at least 50%, at least 60%, at least 70%, at least 80%, at least 90%, at least 95% or more), as compared to that which is observed when a biomaterial (*e.g*., polymeric biomaterial) agonist of innate immunity or a composition comprising the same is not administered.

In some cases, a biomaterial (*e.g*., polymeric biomaterial) agonist of innate immunity disclosed and/or utilized herein is characterized in that, when assessed at an specified time (*e.g.,* 24 hours, 48 hours, or 72 hours) after administration to a target site in a subject in need thereof (*e.g.,* as described herein), more activated dendritic cells are present at a target site (*e.g.,* a tumor resection site) and/or in a secondary lymphoid organ *(e.g.,* a lymph node or spleen) and/or in body circulation (*e.g*., peripheral blood) of the subject than that which is observed when the biomaterial (*e.g.*, polymeric biomaterial) agonist of innate immunity is not administered to the target site. Those of skill in the art will appreciate that various markers for activation of dendritic cells are known and can be used to assess activation status of dendritic cells. For example, in some cases, activation of dendritic cells can be assessed by detecting level of antigen presentation machinery (*e.g.*, MHC I and/or MHC II) and/or of co-stimulatory molecules (*e.g.,* CD40, CD80, and/or CD86) on the surface of dendritic cells. In some cases, such an increase in activated dendritic cells (*e.g.,* in terms of the number of activated dendritic cells and/or level of one or more such activation markers on dendritic cells) at a target site (*e.g.,* a tumor resection site) and/or in a secondary lymphoid organ *(e.g.,* a lymph node or spleen) and/or in body circulation *(e.g.,* peripheral blood) is at least 10% or higher (including, *e.g.,* at least 20%, at least 30%, at least 40%, at least 50%, at least 60%, at least 70%, at least 80%, at least 90%, at least 95% or more) as compared to that which is observed when a biomaterial (*e.g.*, polymeric biomaterial) agonist of innate immunity or a composition comprising the same is not administered.

Various methods for assessing activation of antigen presentation machinery, co-stimulatory molecules, and/or proinflammatory cytokines are known in the art. Those skilled in the art will appreciate that, in some cases, level of antigen presentation machinery, co-stimulatory molecules, and/or proinflammatory cytokines can be assessed based on gene expression (*e.g.,* mRNA levels) in target immune cells, *e.g.,* using quantitative polymerase chain reaction. Those skilled in the art will also appreciate that immunoassays (e.g., ELISA, immunostaining, and/or flow cytometry) can be used to assess such activation as well. For example, protein expression of antigen presentation machinery and/or co-stimulatory molecules on the surface of dendritic cells can be assessed, e.g., by immunostaining and/or flow cytometry. Concentrations of proinflammatory soluble factors such as proinflammatory cytokines in conditioned culture media or peripheral blood can be measured, for example, using ELISA and/or multiplexing laser bead technology.

In some cases, a biomaterial (*e.g*., polymeric biomaterial) agonist of innate immunity disclosed and/or utilized herein is characterized in that, when assessed at an specified time (*e.g.,* 24 hours, 48 hours, or 72 hours) after administration to a target site in a subject in need thereof (*e.g.,* as described herein), more plasmacytoid dendritic cells are present at a target site (*e.g.,* a tumor resection site) and/or in a secondary lymphoid organ (*e.g.,* a lymph node or spleen) and/or in body circulation of the subject than that which is observed when the biomaterial (*e.g.*, polymeric biomaterial agonist of innate immunity is not administered to the target site. As will be understood by those skill in the art, in some cases plasmacytoid dendritic cells can be assessed by detection of relevant activation markers (*e.g.*, B220 and/or PDCA1). In some cases, such an increase in plasmacytoid dendritic cells (*e.g.,* in terms of the number of plasmacytoid dendritic cells and/or level of one or more activation markers in plasmacytoid dendritic cells) at a target site *(e.g.,* a tumor resection site) and/or in a secondary lymphoid organ *(e.g.,* a lymph node or spleen) and/or in body circulation *(e.g.,* peripheral blood) is at least 10% or higher (including, *e.g.,* at least 20%, at least 30%, at least 40%, at least 50%, at least 60%, at least 70%, at least 80%, at least 90%, at least 95% or more) as compared to that which is observed when a biomaterial (*e.g*., polymeric biomaterial) agonist of innate immunity or a composition comprising the same is not administered.

In some cases, a biomaterial (*e.g*., polymeric biomaterial) agonist of innate immunity disclosed and/or utilized herein is characterized in that, when assessed at an specified time (*e.g.,* 24 hours, 48 hours, or 72 hours) after administration to a target site in a subject in need thereof *(e.g.,* as described herein), more NK cells are present at a target site *(e.g.,* a tumor resection site) and/or in a secondary lymphoid organ *(e.g.,* a lymph node or spleen) and/or in body circulation of the subject than that which is observed when the biomaterial (e.g., polymeric biomaterial) agonist of innate immunity is not administered to the target site. As will be understood by those of skill in the art, in some cases, NK cells can be detected by assessment of relevant activation markers (*e.g*., CD69 and/or KLRG1) and/or high effector markers (e.g., CD11b and/or CD27). In some cases, such an increase in NK cells *(e.g.,* in terms of the number of NK cells and/or level of one or more such activation and/or high effector markers in NK cells) at a target site (*e.g.,* a tumor resection site) and/or in a secondary lymphoid organ *(e.g.,* a lymph node or spleen) and/or in body circulation *(e.g.,* peripheral blood) is at least 10% or higher (including, *e.g.,* at least 20%, at least 30%, at least 40%, at least 50%, at least 60%, at least 70%, at least 80%, at least 90%, at least 95% or more) as compared to that which is observed when a biomaterial (*e.g.*, polymeric biomaterial) agonist of innate immunity or a composition comprising the same is not administered.

In some cases, a biomaterial (*e.g.*, polymeric biomaterial) agonist of innate immunity disclosed and/or utilized herein is characterized in that, when assessed at an specified time (*e.g.,* 2 weeks, 3 weeks, 1 month or longer) after administration to a target site in a subject in need thereof (*e.g.,* as described herein), the subject displays at least one or more adaptive antitumor responses, as compared to that which is observed when the biomaterial (*e.g*., polymeric biomaterial) agonist of innate immunity is not administered to the target site. For example, as will be understood by those of skill in the art, in some cases, an adaptive antitumor response can be assessed by evaluating the number of T cells and/or proportion of T cells that express one or more activation markers (*e.g.,* CD69 and/or GITR). For example, in some cases, such a biomaterial (*e.g*., polymeric biomaterial) agonist of innate immunity is characterized in that, when assessed at an specified time *(e.g.,* 2 weeks, 3 weeks, 1 month or longer) after administration to a target site in a subject in need thereof *(e.g.,* as described herein), the number of T cells (e.g., expressing CD4 but not FoxP3, or expressing CD8) at a target site *(e.g.,* a tumor resection site) and/or in a secondary lymphoid organ *(e.g.,* a lymph node or spleen) and/or in body circulation (e.g., peripheral blood) is at least 10% or higher (including, *e.g.,* at least 20%, at least 30%, at least 40%, at least 50%, at least 60%, at least 70%, at least 80%, at least 90%, at least 95% or more) as compared to that which is observed when a biomaterial (e.g., polymeric biomaterial) agonist of innate immunity or a composition comprising the same is not administered. Additionally or alternatively, such a biomaterial (e.g., polymeric biomaterial) agonist of innate immunity is characterized in that, when assessed at an specified time (*e.g.,* 2 weeks, 3 weeks, 1 month or longer) after administration to a target site in a subject in need thereof (*e.g.,* as described herein), the number of activated T cells (*e.g.,* expressing CD69 and/or GITR) at a target site (*e.g.,* a tumor resection site) and/or in a secondary lymphoid organ (*e.g.,* a lymph node or spleen) and/or in body circulation (*e.g.,* peripheral blood) is at least 10% or higher (including, *e.g.,* at least 20%, at least 30%, at least 40%, at least 50%, at least 60%, at least 70%, at least 80%, at least 90%, at least 95% or more) than that which is observed when a biomaterial (e.g., polymeric biomaterial) agonist of innate immunity or a composition comprising the same is not administered.

In some cases, a biomaterial agonist of innate immunity comprises one or more polymers. In some cases, a biomaterial agonist of innate immunity comprises at least two or more polymers, at least one of which functions as an agonist or activator of innate immunity. In some such cases, a biomaterial agonist of innate immunity may comprise at least one polymer that functions as an agonist or activator of innate imunity and at least one polymer that is non-immunomodulatory. In some cases where a biomaterial agonist of innate immunity comprises one or more polymers, polymer(s) for use in such a biomaterial may have an average molecular weight of at least 2 kDa, 2.5 kDa, at least 5 kDa, at least 10 kDa or higher, including, *e.g.,* at least 20 kDa, at least 30 kDa, at least 40 kDa, at least 50 kDa, at least 60 kDa, at least 70 kDa, at least 80 kDa, at least 90 kDa, at least 100 kDa, at least 110 kDa, at least 120 kDa, at least 130 kDa, at least 140 kDa, at least 150 kDa, at least 160 kDa, at least 170 kDa, at least 180 kDa, at least 190 kDa, at least 200 kDa, at least 210 kDa, at least 220 kDa, at least 230 kDa, at least 240 kDa, at least 250 kDa, at least 260 kDa, at least 270 kDa, at least 280 kDa, at least 290 kDa, at least 300 kDa, at least 350 kDa, at least 400 kDa, at least 500 kDa, at least 600 kDa, at least 700 kDa, or higher. In some cases, such polymer(s) may have an average molecular weight of no more than 1000 kDa or lower, including, *e.g.,* no more than 900 kDa, no more than 800 kDa, no more than 700 kDa, no more than 600 kDa, no more than 500 kDa, no more than 400 kDa, no more than 300 kDa, no more than 200 kDa, no more than 100 kDa, no more than 50 kDa, or lower. Combinations of the above-mentioned ranges are also possible. For example, in some cases, such polymer(s) is characterized by an average molecular weight of 2 kDa to 1000 kDa, or 5 kDa to 600 kDa, or 10 kDa to 400 kDa. Those skilled in the art will appreciate that a polymer is typically characterized by a molecular weight distribution, which, in some cases, may be used to determine an average molecular weight. Such skilled in the art will further appreciate that an average molecular weight can be represented by different ways, including, e.g., number average molecular weight, weight average molecular weight, and peak average molecular weight, and can be determined, for example, by gel electrophoresis (*e.g.,* sodium dodecyl sulfate polyacrylamide gel electrophoresis (SDS-PAGE), size exclusion gel chromatography, mass spectroscopy (e.g., MALDI or ESI), or high performance liquid chromatography (HPLC), refractive index detection (RID), light scattering, or any combination thereof *(e.g.,* HPLC-RID).

In some cases, a biomaterial (*e.g.,* polymeric biomaterial) agonist of innate immunity that is useful for a composition disclosed herein (*e.g.*, exhibiting one or more characteristics described herein, such as, *e.g.,* increased level of one or more proinflammatory cytokines and/or increased number and/or activation of dendritic cell and/or NK cells) can be or comprise a carbohydrate polymer. In some cases, such a carbohydrate polymer may comprise one or more amino sugars connected by covalent bonds. Exemplary amino sugars that may be useful in such a carbohydrate polymer are or comprise glucosamine, galactosamine, fructosamine and/or mannosamine. In some cases, such a carbohydrate polymer is characterized in that when administered to a subject, the carbohydrate polymer induces production of IFN *(e.g.,* Type I IFN), *e.g.,* through activation of a cGAS-STING pathway or one or more components thereof, and/or induces production of IL-1β, *e.g.,* through activation of an inflammasome (*e.g.,* NLRP3). In some cases, such a carbohydrate polymer is characterized in that when administered a subject, the carbohydrate polymer induces production of one or more proinflamamtory cytokines (*e.g.,* as described herein) and/or upregulation of antigen presentation machinery and/or costimulatory molecules (e.g., as described herein). In certain cases, a carbohydrate polymer that is useful in accordance with the present disclosure is or comprises a glucan (*e.g*., dextran such as alpha-glucan, zymosan such as beta-glucan), a fructan (*e.g*., inulin), a mannan, chitin and/or chitosan, a mycobacterial carbohydrate (*e.g*_{.}, lipoarabinomannan (LAM), muramyldipeptide (MDP), D-murapalmitine, trehalose-6-6-dimycolate), a lipopolysaccharide (LPS), a saponin compound (*e.g.,* QS-21), and/or a carbohydrate-containing compound as described in Petrovsky and Cooper, "Carbohydrate-based immune adjuvants" Expert Rev Vaccines, 10: 523-537 (2011).

Those skilled in the art will appreciate that chitosan and/or chitin can induce innate immunity. See, *e.g.,* Bueter et al., "Innate Sensing of Chitin and Chitosan" PLOS Pathogens, 9(1): e1003080 (2013); Carroll et al. "The Vaccine Adjuvant Chitosan Promotes Cellular Immunity via DNA Sensor cGAS-STING-Dependent Induction of Type I interferons" Immunity, 44(3): 597-608 (2016); and Riteau and Sher "Chitosan: An Adjuvant with an Unanticipated STING" Immunity, 44(5) 522-524. Accordingly, in particular cases, a carbohydrate polymer that is useful in accordance with the present disclosure is or comprises chitosan or variants thereof, or combinations thereof. In particular cases, a carbohydrate polymer that is useful in accordance with the present disclosure is or comprises chitin. One of those skilled in the art will appreciate that the size of chitin particles may determine the type of immune response, *e.g.,* as described in Bueter et al., "Innate Sensing of Chitin and Chitosan," PLOS Pathogens 9(1):e1003080. Thus, in some cases, chitin for use in compositions described herein may be particles of 40-70 µm, which were shown to induce a proinflammatory response.

Additional examples of polymers for use in a biomaterial (*e.g*., polymeric biomaterial) agonist of innate immunity utilized herein include, but are not limited to alginate (which can induce innate immune activation through a NF-κB pathway; see, *e.g.,* Yang and Jones "Effect of alginate on innate immune activation of macrophages" J Biomed Mater Res A, 90: 411-418(2009)), polyacrylic acid (which can induce innate immune activation through reactive oxygen species (ROS) production; see, *e.g.,* Gartlan et al., "Sterile inflammation induced by Carbopol elicits robust adaptive immune responses in the absence of pathogen-associated molecular patterns" Vaccine 34: 2188-2196 (2016)), polyphosphazene, silica gel (which can induce innate immune activation through NLRP3 inflammasome), and variants thereof, and combinations thereof. Exemplary polyphosphazene may be or comprise poly[(organo)phosphazenes], including, *e.g.,* poly[di(carboxylatoethylphenoxy)phosphazene] (PCEP), poly[di(carboxylatophenoxy)phosphazene] (PCPP), and salts thereof (*e.g*., sodium salts thereof). In some cases, a PCEP may be or comprise poly[di(sodium carboxylatoethylphenoxy)phosphazene]. Those skilled in the art will appreciate that polyphosphazenes can activate TLRs and NLRP3 inflammasome, e.g., as described in Magiri et al., "Recent advances in experimental polyphosphazene adjuvants and their mechanisms of action." Cell Tissue Res., 374: 465-471 (2018).

Polymers utilized in and/or biomaterial (*e.g.*, polymeric biomaterial) agonists of innate immunity themselves are typically biocompatible. In some cases, polymers utilized in and/or biomaterial (*e.g*., polymeric biomaterial) agonists of innate immunity themselves are biodegradable *in vivo.* One of those skilled in the art will appreciate, reading the present disclosure, that degradation rate of different polymers and/or polymeric biomaterial agonists of innate immunity may be selected, *e.g*., based on types of cancer, patients' physical condition and/or medical history, characteristics (*e.g.*, immunogenicity) of materials used, and/or desirable treatment duration for which a method is being practiced. As will be understood by one of those skilled in the art, a biomaterial (e.g., polymeric biomaterial) has a longer residence time at a target site upon administration if the biomaterial (e.g., polymeric biomaterial) has a slower *in vivo* degradation rate.

In some cases, a biomaterial (e.g., polymeric biomaterial) agonist of innate immunity disclosed and/or utilized herein is characterized in that, when assessed *in vivo* by administering to a target site (*e.g.,* a tumor resection site) in a test subject (*e.g.,* as described herein), at least 10% or more, including, *e.g.,* at least 20%, at least 30%, at least 40%, at least 50%, at least 60%, at least 70%, at least 80%, at least 90%, or more, of such a biomaterial *(e.g.,* polymeric biomaterial) agonist of innate immunity remains at the target site *in vivo 2* days or more after the administration. In some cases, less than or equal to 90%, less than or equal to 80%, less than or equal to 70%, less than or equal to 60%, less than or equal to 50%, less than or equal to 40%, less than or equal to 30%, less than or equal to 20%, or lower, of such a biomaterial (*e.g*., polymeric biomaterial) agonist of innate immunity remains at a target site *in vivo* 2 days or more after the administration. Combinations of the above-mentioned are also possible. For example, in some cases, a biomaterial (e.g., polymeric biomaterial) agonist of innate immunity disclosed and/or utilized herein is characterized in that, when assessed *in vivo* by administering to a target site (*e.g.,* a tumor resection site) in a test subject (*e.g.,* as described herein), 30%-80% or 40%-70% of such a biomaterial (*e.g.,* polymeric biomaterial) agonist of innate immunity remains at the target site *in vivo* 2 days or more after the administration.

In some cases, a biomaterial (e.g., polymeric biomaterial) agonist of innate immunity disclosed and/or utilized herein is characterized in that, when assessed *in vivo* by administering to a target site (*e.g.,* a tumor resection site) in a test subject (*e.g.,* as described herein), at least 10% or more, including, *e.g.,* at least 20%, at least 30%, at least 40%, at least 50%, at least 60%, at least 70%, at least 80%, at least 90%, or more, of such a biomaterial (*e.g.,* polymeric biomaterial) agonist of innate immunity remains at the target site *in vivo 3* days or more after the administration. In some cases, less than or equal to 90%, less than or equal to 80%, less than or equal to 70%, less than or equal to 60%, less than or equal to 50%, less than or equal to 40%, less than or equal to 30%, less than or equal to 20%, or lower, of such a biomaterial (*e.g.*, polymeric biomaterial) agonist of innate immunity remains at a target site *in vivo* 3 days or more after the administration. Combinations of the above-mentioned are also possible. For example, in some cases, a biomaterial (*e.g*., polymeric biomaterial) agonist of innate immunity disclosed and/or utilized herein is characterized in that, when assessed *in vivo* by administering to a target site *(e.g.,* a tumor resection site) in a test subject (*e.g.,* as described herein), 30%-80% or 40%-70% of such a biomaterial (*e.g.,* polymeric biomaterial) agonist of innate immunity remains at the target site *in vivo* 3 days or more after the administration.

In some cases, a biomaterial (*e.g*., polymeric biomaterial) agonist of innate immunity disclosed and/or utilized herein is characterized in that, when assessed *in vivo* by administering to a target site (*e.g.,* a tumor resection site) in a test subject (*e.g.,* as described herein), at least 10% or more, including, *e.g.,* at least 20%, at least 30%, at least 40%, at least 50%, at least 60%, at least 70%, at least 80%, at least 90%, or more, of such a biomaterial *(e.g.,* polymeric biomaterial) agonist of innate immunity remains at the target site *in vivo 5* days or more after the administration. In some cases, less than or equal to 90%, less than or equal to 80%, less than or equal to 70%, less than or equal to 60%, less than or equal to 50%, less than or equal to 40%, less than or equal to 30%, less than or equal to 20%, or lower, of such a biomaterial (*e.g.,* polymeric biomaterial) agonist of innate immunity remains at a target site *in vivo* 5 days or more after the administration. Combinations of the above-mentioned are also possible. For example, in some cases, a biomaterial (*e.g*., polymeric biomaterial) agonist of innate immunity disclosed and/or utilized herein is characterized in that, when assessed *in vivo* by administering to a target site (*e.g.,* a tumor resection site) in a test subject (*e.g.,* as described herein), 30%-80% or 40%-70% of such a biomaterial *(e.g.,* polymeric biomaterial) agonist of innate immunity remains at the target site *in vivo* 5 days or more after the administration.

In some cases, a biomaterial (*e.g.*, polymeric biomaterial) agonist of innate immunity disclosed and/or utilized herein is characterized in that, when assessed *in vivo* by administering to a target site (*e.g.,* a tumor resection site) in a test subject (*e.g.,* as described herein), at least 10% or more, including, *e.g.,* at least 20%, at least 30%, at least 40%, at least 50%, at least 60%, at least 70%, at least 80%, at least 90%, or more, of such a biomaterial (*e.g.,* polymeric biomaterial) agonist of innate immunity remains at the target site *in vivo 7* days or more after the administration. In some cases, less than or equal to 90%, less than or equal to 80%, less than or equal to 70%, less than or equal to 60%, less than or equal to 50%, less than or equal to 40%, less than or equal to 30%, less than or equal to 20%, or lower, of such a biomaterial (e.g., polymeric biomaterial) agonist of innate immunity remains at a target site *in vivo* 7 days or more after the administration. Combinations of the above-mentioned are also possible. For example, in some cases, a biomaterial (e.g., polymeric biomaterial) agonist of innate immunity disclosed and/or utilized herein is characterized in that, when assessed *in vivo* by administering to a target site (*e.g.,* a tumor resection site) in a test subject (*e.g.,* as described herein), 30%-80% or 40%-70% of such a biomaterial (*e.g.,* polymeric biomaterial) agonist of innate immunity remains at the target site *in vivo* 7 days or more after the administration.

In some cases, a biomaterial (*e.g*., polymeric biomaterial) agonist of innate immunity disclosed and/or utilized herein is characterized in that, when assessed *in vivo* by administering to a target site (*e.g.,* a tumor resection site) in a test subject (*e.g.,* as described herein), at least 10% or more, including, *e.g.,* at least 20%, at least 30%, at least 40%, at least 50%, at least 60%, at least 70%, at least 80%, at least 90%, or more, of such a biomaterial (*e.g.,* polymeric biomaterial) agonist of innate immunity remains at the target site *in vivo 14* days or more after the administration. In some cases, less than or equal to 90%, less than or equal to 80%, less than or equal to 70%, less than or equal to 60%, less than or equal to 50%, less than or equal to 40%, less than or equal to 30%, less than or equal to 20%, or lower, of such a biomaterial (e.g., polymeric biomaterial) agonist of innate immunity remains at a target site *in vivo* 14 days or more after the administration. Combinations of the above-mentioned are also possible. For example, in some cases, a biomaterial (*e.g*., polymeric biomaterial) agonist of innate immunity disclosed and/or utilized herein is characterized in that, when assessed *in vivo* by administering to a target site *(e.g.,* a tumor resection site) in a test subject (*e.g.,* as described herein), 30%-80% or 40%-70% of such a biomaterial *(e.g.,* polymeric biomaterial) agonist of innate immunity remains at the target site *in vivo* 14 days or more after the administration.

In some cases, a biomaterial (*e.g*., polymeric biomaterial) agonist of innate immunity disclosed and/or utilized herein is characterized in that, when assessed *in vivo* by administering to a target site *(e.g.,* a tumor resection site) in a test subject *(e.g.,* as described herein), no more than 10% or less, including, *e.g.,* no more than 9%, no more than 8%, no more than 7%, no more than 6%, no more than 5%, no more than 4%, no more than 3%, no more than 2%, no more than 1% or less, of such a biomaterial (*e.g*., polymeric biomaterial) agonist of innate immunity remains at the target site *in vivo* 10 days or more after the administration.

In some cases, a biomaterial (*e.g.*, polymeric biomaterial) agonist of innate immunity disclosed and/or utilized herein is characterized in that, when assessed *in vivo* by administering to a target site (*e.g.,* a tumor resection site) in a test subject (*e.g.,* as described herein), such a biomaterial (*e.g*., polymeric biomaterial) agonist of innate immunity is dissolved or degraded at a rate such that innate immunity is stimulated in one or more aspects (*e.g.,* ones as described herein, including, *e.g.,* but not limited to activation of a pattern recognition receptor, an inflammasome, and/or a cGAS-STING pathway; and/or production of proinflammatory cytokines and/or upregulation of antigen presentation machinery and/or costimulatory molecules) for a period of at least 2 days or more, including, *e.g.,* at least 3 days, at least 4 days, at least 5 days, at least 6 days, at least 7 days, at least 9 days, at least 10 days or more. In some cases, a biomaterial (*e.g.*, polymeric biomaterial) agonist of innate immunity disclosed and/or utilized herein is characterized in that, when assessed *in vivo* by administering to a target site *(e.g.,* a tumor resection site) in a test subject (*e.g.,* as described herein), such a biomaterial (*e.g.,* polymeric biomaterial) agonist of innate immunity is dissolved or degraded at a rate such that innate immunity is stimulated in one or more aspects (*e.g.,* ones as described herein, including, *e.g.,* but not limited to activation of a pattern recognition receptor, an inflammasome, and/or a cGAS-STING pathway; and/or production of proinflammatory cytokines and/or upregulation of antigen presentation machinery and/or costimulatory molecules) for a period of no more than 15 days or fewer, including, *e.g.,* no more than 10 days, no more than 9 days, no more than 8 days, no more than 7 days, no more than 6 days, no more than 5 days, no more than 4 days, no more than 3 days or fewer.

A biomaterial (*e.g.*, polymeric biomaterial) agonist of innate immunity can be provided in a suitable format. In some cases, a biomaterial (*e.g.*, polymeric biomaterial) agonist of innate immunity can be provided as a liquid, *e.g.,* a viscous polymer solution. Such a liquid format (*e.g.,* a viscous polymer solution), in some cases, can be useful in circumstances where a limited impact duration of innate immunity stimulation is preferred and/or injection is more desirable. In some cases, a biomaterial (*e.g*., polymeric biomaterial) agonist of innate immunity can be provided as a pre-formed biomaterial (*e.g*., a pre-formed polymer network biomaterial), which, in some cases, can be or comprise a crosslinked or non-crosslinked polymer network biomaterial. In some cases, a biomaterial (*e.g.,* polymeric biomaterial) agonist of innate immunity can be provided as a population of particles (*e.g.*, nanoparticles or microparticles), which can be, in some cases, suspended or distributed in a carrier (*e.g.,* a solution or a biomaterial such as a polymer network biomaterial, which can be the same or a different material).

A biomaterial (*e.g.,* polymeric biomaterial) agonist of innate immunity utilized in a composition may be selected to have an appropriate elasticity and/or stiffness to suit the need of a particular application. For example, those of skill in the art, reading the present disclosure, will appreciate that appropriate elasticity and/or stiffness of a biomaterial (*e.g.,* polymeric biomaterial) agonist of innate immunity may be selected based on, for example, characteristics of tissue surrounding a tumor, administration routes, administration sites, and/or desired duration of innate immunity stimulation in which a method is being practiced. Accordingly, in some cases, a biomaterial (*e.g.,* polymeric biomaterial) agonist of innate immunity is characterized by a storage modulus of at least 10 Pa, at least 20 Pa, at least 30 Pa, at least 40 Pa, at least 50 Pa, at least 75 Pa, at least 100 Pa, at least 250 Pa, at least 500 Pa, at least 1000 Pa, at least 1500 Pa, at least 2000 Pa, at least 2500 Pa, at least 3000 Pa, at least 4000 Pa, at least 5000 Pa, at least 10 kPa, at least 15 kPa, at least 20 kPa, at least 25 kPa, at least 30 kPa, at least 35 kPa, at least 40 kPa, or higher. In some cases, a biomaterial (e.g., polymeric biomaterial) agonist of innate immunity is characterized by a storage modulus of no more than 50 kPa, no more than 40 kPa, no more than 30 kPa, no more than 20 kPa, no more than 10 kPa, no more than 5000 Pa, no more than 4000 Pa, no more than 3000 Pa, no more than 2000 Pa, no more than 1000 Pa, no more than 500 Pa, no more than 250 Pa, no more than 100 Pa, no more than 75 Pa, no more than 50 Pa, no more than 25 Pa or lower. Combinations of the above-mentioned ranges are also possible. For example, in some cases, a biomaterial (*e*.*g.,* polymeric biomaterial) agonist of innate immunity is characterized by a storage modulus of 10 Pa to 50 kPa, 500 Pa to 50 kPa, 1000 Pa to 20 kPa, or 1000 Pa to 10 kPa, or 1000 Pa to 5000 Pa, or 1000 Pa to 3000 Pa. One of those skilled in the art will appreciate that various rheological characterization methods (*e.g.,* as described in Weng *et al.,* "Rheological Characterization of *in situ* Crosslinkable Hydrogels Formulated from Oxidized Dextran and N-Carboxyethyl Chitosan" *Biomacromolecules,* 8: 1109-1115 (2007)) can be used to measure storage modulus of a material, and that, in some cases, storage modulus of a material may be measured with a rheometer and/or dynamic mechanical analysis (DMA). One of those skilled in the art will also appreciate that rheological characterization can vary with surrounding condition, *e.g.,* temperature. Accordingly, in some cases, a biomaterial (*e.g.,* polymeric biomaterial) agonist of innate immunity is characterized by a storage modulus (*e.g.,* as described herein) measured at room temperature (*e.g.,* 22°C-27°C). In some cases, a biomaterial (*e.g*., polymeric biomaterial) agonist of innate immunity is characterized by a storage modulus (*e.g.,* as described herein) measured at a body temperature of a subject *(e.g.,* 37°C of a human subject). As will be clear to one skilled in the art reading the disclosure provided herein, a storage modulus of a described biomaterial (*e.g.*, polymeric biomaterial) agonist of innate immunity, *e.g.*, in a form of particles, refers to a bulk storage modulus of particles in a population.

In some cases where a biomaterial (e.g., polymeric biomaterial) agonist of innate immunity may be provided as a liquid *(e.g.,* a viscous solution such as a viscous polymer solution), such a biomaterial (polymeric biomaterial) agonist of innate immunity is characterized by a low storage modulus (*e.g.*, relative to storage modulus of a biomaterial agonist of innate immunity in a form of a polymer network biomaterial). In particular cases, such a biomaterial (*e.g.*, polymeric biomaterial) agonist of innate immunity can be characterized by a storage modulus that is low enough for injection to a target site in a subject in need thereof. A skilled artisan will appreciate that appropriate storage modulus of a biomaterial (*e.g.,* polymeric biomaterial) for injection can be selected based on, *e.g.,* the size of a target injection site, injection volume, and/or injection needle gauge size. For example, in some cases, an injectable biomaterial (*e.g*., polymeric biomaterial) agonist of innate immunity is characterized by a storage modulus of at least 10 Pa, at least 20 Pa, at least 30 Pa, at least 40 Pa, at least 50 Pa or higher. In some cases, an injectable biomaterial (e.g., polymeric biomaterial) agonist of innate immunity is characterized by a storage modulus of no more than 500 Pa, no more than 250 Pa, no more than 100 Pa, no more than 75 Pa, no more than 50 Pa, no more than 25 Pa or lower. Combinations of the above-mentioned ranges are also possible. For example, in some cases, an injectable biomaterial (*e.g*., polymeric biomaterial) agonist of innate immunity is characterized by a storage modulus of 10 Pa to 500 Pa, 10 Pa to 250 Pa, 10 Pa to 100 Pa, 25 Pa to 500 Pa, 25 Pa to 250 Pa, or 25 Pa to 100 Pa. In some cases, such an injectable biomaterial (*e.g*., polymeric biomaterial) agonist of innate immunity, upon administration to a target site (*e.g.,* as described herein), can remain at a target site for an impact duration of innate immunity stimulation (e.g., 2-15 days or 2-10 days). In some cases, an injectable biomaterial (*e.g*., polymeric biomaterial) agonist of innate immunity may be a less viscous liquid during administration and become a more viscous liquid after administration to a target site *(e.g.,* as described herein). In some cases, such an injectable biomaterial (*e.g.*, polymeric biomaterial) agonist of innate immunity, upon administration to a target site (*e.g.,* as described herein) can form a polymer network biomaterial *in situ* at the target site, *e.g.,* in some cases, by exposing the injectable biomaterial (*e.g*., polymeric biomaterial) agonist of innate immunity, upon administration to a target site (*e.g.,* as described herein), to a crosslinking agent (*e.g.,* ones described herein). For example, in some cases, a polymer network biomaterial formed *in situ* at a target site, upon administration, may have a storage modulus that is higher than that of the initial storage modulus of an injectable form, *e.g.,* by at least 10% or higher, including, *e.g.,* at least 20%, at least 30%, at least 40%, at least 50%, at least 60%, at least 70%, at least 80%, at least 90%, at least 95%, at least 1-fold, at least 2-fold, at least 3-fold, at least 5-fold, at least 10-fold, or higher.

In some cases, a biomaterial (e.g., polymeric biomaterial) agonist of innate immunity can be characterized by a viscosity of no more than 10,000 mPa·s or lower, including, *e.g.,* no more than 9000 mPa·s, no more than 8000 mPa·s, no more than 7000 mPa·s, no more than 6000 mPa·s, no more than 5000 mPa·s, no more than 4000 mPa·s, no more than 3500 mPa·s, no more than 3000 mPa·s, no more than 2500 mPa·s, no more than 2000 mPa·s, no more than 1500 mPa·s, no more than 1000 mPa·s, no more than 500 mPa·s, no more than 250 mPa·s, no more than 200 mPa·s, no more than 150 mPa·s, no more than 100 mPa·s, no more than 75 mPa·s, no more than 50 mPa·s, no more than 25 mPa·s, no more than 20 mPa·s, no more than 15 mPa·s, no more than 10 mPa·s, or lower. In some cases, a biomaterial (*e.g.*, polymeric biomaterial) agonist of innate immunity may be characterized by a viscosity of at least 5 mPa·s or higher, including, *e.g.,* at least 10 mPa·s, at least 20 mPa·s, at least 30 mPa·s, at least 40 mPa·s, at least 50 mPa·s, at least 60 mPa·s, at least 70 mPa·s, at least 80 mPa·s, at least 90 mPa·s, at least 100 mPa·s, at least 125 mPa·s, at least 150 mPa·s, at least 175 mPa·s, at least 250 mPa·s, at least 500 mPa·s, at least 1000 mPa·s, at least 1500 mPa·s, at least 2000 mPa·s, at least 2500 mPa·s, at least 3000 mPa·s, at least 4000 mPa·s, at least 5000 mPa·s, at least 6000 mPa·s, at least 7000 mPa·s, at least 8000 mPa·s, at least 9000 mPa·s, or higher. Combinations of the above-mentioned ranges are also possible. For example, in some cases, a biomaterial (*e.g.,* polymeric biomaterial) agonist of innate immunity may be characterized by a viscosity of 5 mPa·s to 10,000 mPa·s, or 10 mPa·s to 5000 mPa·s, or 5 mPa·s to 200 mPa·s, or 20 mPa·s to 200 mPa·s, or 5 mPa·s to 20 mPa·s. One skilled in the art reading the present disclosure will appreciate that, in some cases, viscosity of a biomaterial (*e.g.,* polymeric biomaterial) agonist of innate immunity may be selected or adjusted based on, *e.g.,* administration routes (*e.g.,* injection vs. implantation), injection volume and/or time, and/or impact duration of innate immunity stimulation. As will be also understood by one skilled in the art, viscosity of a polymer depends on, *e.g.,* temperature and concentration of the polymer in a testing sample. In some cases, viscosity of a biomaterial (*e.g*., polymeric biomaterial) agonist of innate immunity as described herein may be measured at 20 °C, *e.g.,* with a shear rate of 1000 s⁻¹.

In some cases, a biomaterial (*e.g.*, polymeric biomaterial) agonist of innate immunity can be characterized by a phase angle indicative of a viscoelastic material. For example, in some cases, a biomaterial (*e.g.,* polymeric biomaterial) agonist of innate immunity can be characterized by a phase angle of 1° to 50°, or 2° to 45°, or 3° to 40°, or 3° to 35°, 3° to 30°, or 3° to 25°, or 5° to 30°, or 10° to 30°, 15° to 25°. In some cases, a biomaterial (*e.g*., polymeric biomaterial) agonist of innate immunity can be characterized by a phase angle of 10° to 30° or 15° to 25°. As will be understood by one skilled in the art, phase angle of a polymer biomaterial may be determined by dynamical mechanical analysis, *e.g.,* a frequency sweep analysis, which include, *e.g.,* determination of shear storage modulus and shear loss modulus of a sample. One skilled in the art will appreciate that a storage or elastic modulus of a material may be determined based on its stored energy and it represents the elastic property of the material, while a loss or viscous modulus may be determined based on the energy dissipated as heat and it represents the viscous property of the material. The phase angle (delta) is the arctangent of the ratio of a storage modulus to a loss modulus and its value indicates if the material is more elastic or viscous. Typically, a phase angle of > 45° indicates that the viscous property dominates and the material behaves more like a solution. As the phase angle approaches 0°, the elastic (solid or gel-like) property dominates. For example, a material with a high storage modulus and a low phase angle indicates a stronger gel (more elastic) than one with a lower storage modulus and phase angle. In some cases, the phase angle of a biomaterial (*e.g*., polymeric biomaterial) agonist of innate immunity as described herein may be determined from a frequency sweep analysis performed at a temperature corresponding to the body of the body temperature of a subject to be treated. In some cases, a frequence sweep analysis may be performed over a frequency range of 0.1 to 10 Hz with application of a constant 0.4% strain.

In some cases, a biomaterial (*e.g*., polymeric biomaterial) agonist of innate immunity administered to a target site in a subject in need thereof (*e.g.,* as described herein) is a polymer network biomaterial or forms a polymer network biomaterial *in situ* at the target site. In some cases, such a polymer network biomaterial can be or comprise a non-crosslinked polymer network biomaterial. In some cases, such a polymer network biomaterial can be or comprise a crosslinked polymer network biomaterial. An exemplary crosslinked polymer network biomaterial is or comprises a hydrogel. Various crosslinkers and methods for crosslinking polymer chains are known to those of skill in the art, *e.g.,* as described in Parhi, Adv Pharm Bull., Review 7(4): 515-530 (2017); Ahmadi et al. Res Pharm Sci., 10(1): 1-16 (2015); and Khunmanee et al. "Crosslinking method of hyaluronic-based hydrogel for biomedical applications" J Tissue Eng. 8: 1-16 (2017). Further, those of skill in the art will appreciate that appropriate crosslinking methods may be selected based on, for example, types of polymers being utilized, ease of crosslinking *in situ* at a target site, crosslinking strength and/or types, and/or an application in which a method is being practiced.

In some cases, a crosslinked polymer network biomaterial may comprise a covalent crosslink. Those of skill in the art will appreciate that, in some cases, such a covalent crosslink may be formed by using a chemical crosslinker such as, *e.g.,* a small-molecule crosslinker, which can be derived from a natural source or synthesized. Non-limiting examples of such chemical crosslinkers include genipin, dialdehyde, glutaraldehyde, glyoxal, diisocyanate, glutaric acid, succinic acid, adipic acid, acrylic acid, diacrylate, *etc.).* Additionally or alternatively, those of skill in the art will appreciate that, in some cases, such a covalent crosslink may be formed by thermal-induced crosslinking (*e.g.*, mixing with a thermal-induced crosslinker such as, *e.g.,* a thermo-responsive polymer such as, *e.g.,* poloxamer 407 or poloxamer 188), photo-induced crosslinking (*e.g*., mixing with a photo-induced crosslinker such as vinyl sulfone, methacrylate, acrylic acid, azido-benzoic acid), pH-induced crosslinking, enzyme-catalyzed crosslinking, and combinations thereof. In some cases, a polymer network biomaterial can be crosslinked by attaching thiols *(e.g.,* EXTRACEL^{®}, HYSTEM^{®}), methacrylates, hexadecylamides (e.g., HYMOVIS^{®}), and/or tyramines (*e.g*., CORGEL^{®}). In some cases, a polymer network biomaterial can be crosslinked directly with formaldehyde (*e.g.,* HYLAN-A^{®}), divinylsulfone (DVS) *(e.g.,* HYLAN-B^{®}), 1,4-butanediol diglycidyl ether (BDDE) (e.g., RESTYLANE^{®}), glutaraldehyde, and/or genipin (see, *e.g.,* Khunmanee et al. "Crosslinking method of hyaluronic-based hydrogel for biomedical applications" J Tissue Eng. 8: 1-16 (2017)). In some cases, a polymer network biomaterial is crosslinked with divinylsulfone (DVS) *(e.g.,* HYLAN-B^{®}).

In some cases, a crosslinked polymer network biomaterial may comprise an ionic crosslink. Those of skill in the art will appreciate that, in some cases, such an ionic crosslink may be formed by mixing with an oppositely charged multivalent counter ion (*e.g.,* tripolyphosphate, tricarboxylic acids, dicarboxylic acids, *etc*.) and/or mixing with an oppositely charged polymer. By way of example only, positively charged polymer chains can be crosslinked with each other through ionic interaction with added negatively charged polymer chains and *vice versa.* Therefore, as will be understood by those skill in the art, an appropriate positively charged polymer or negatively charged polymer - including, e.g., alginate, carrageenan, chitosan, chondroitin sulfate, dextran sulfate, gelatin, hyaluronic acid, polylactic acid, pectin, polyacrylic acid, polybeta amino ester, polyphosphoric acid, and/or xanthan gum - can be used in such ionic crosslinking.

In some cases, a polymer network biomaterial can be crosslinked to increase its residence time at a target site to which it is administered, *e.g.,* when it is more desirable to prolong stimulation of innate immunity, as compared to a non-crosslinked polymer network counterpart. In some cases, a crosslinked polymer network biomaterial may have a reduced degradation rate relative to its non-crosslinked polymer network counterpart. In some cases, a crosslinked polymer network biomaterial may have a reduced solubility (*e.g.,* in a physiological condition or a buffered solution) relative to that of its non-crosslinked polymer network counterpart.

In some cases, a biomaterial (*e.g*., polymeric biomaterial) agonist of innate immunity described herein may be modified to increase its solubility (*e.g*., as measured by dissolution rate such as in an aqueous environment) and/or degradability (*e.g*., as measured by degradation rate). In some cases, such modification(s) may reduce its residence time at a target site to which it is administered, as compared to a non-modified counterpart. For example, such modification(s), in some cases, may be useful for applications in which a short period of innate immunity stimulation is more desirable. Those of skill in the art will appreciate that appropriate modification approaches known in the art, *e.g.,* as described in Ahmadi et al. Res Pharm Sci., 10(1): 1-16 (2015), may be selected based on, *e.g.,* chemical structure of polymer(s) and/or an application in which a method is being practiced. For example, in some cases, a polymer for use in a biomaterial (*e.g*., polymeric biomaterial) agonist of innate immunity may be modified by addition of a hydrophilic chemical moiety *(e.g.,* a carboxylic group or a sugar such as a monosaccharide, disaccharide, or oligosaccharide) or a hydrophilic polymer (*e.g*., polyethylene glycol, polycarboxybetaine, or polysulfobetaine). In some cases, a polymer for use in a biomaterial (*e.g*., polymeric biomaterial) agonist of innate immunity may be modified by thiolation (*e.g*., by introducing at least one or more -SH groups to a polymer chain).

In some cases, a biomaterial (*e.g*., polymeric biomaterial) agonist of innate immunity described herein may be modified, for example to alter its mucoadhesiveness. For example, in some cases, mucoadhesiveness of a biomaterial (*e.g*., polymeric biomaterial) agonist of innate immunity may be increased by thiolation (*i.e*., introducing at least one or more -SH groups to a polymer chain such that it can readily form disulfide bond with cysteine-rich parts of glycoproteins present in epithelial tissues).

In some cases, a biomaterial (*e.g*., a polymeric biomaterial) agonist of innate immunity described herein may be tuned for its immunomodulatory property(ies). For example, those skilled in the art will appreciate that in some cases, one or more immunomodulatory properties of a polymeric biomaterial may be tuned by its biomaterial property(ies), including, *e.g.,* surface chemistry of a polymeric biomaterial *(e.g.,* modulated by hydrophobicity and/or hydrophilicity portions of a polymeric biomaterial, chemical moieties, and/or charge characteristics) and/or topography of a polymeric biomaterial (*e.g*., modulated by size, shape, and/or surface texture), for example as described in Mariani et al. "Biomaterials: Foreign Bodies or Tuners for the Immune Response?" International Journal of Molecular Sciences, 2019, 20, 636.

In some cases, a biomaterial (*e.g*., polymeric biomaterial) agonist of innate immunity is GMP-grade. As will be appreciated by one of those skilled in the art, a GMP-grade product is typically manufactured following current GMP guidelines, *e.g*., to maintain endotoxin level at or below an acceptable level.

In some cases, a biomaterial (*e.g*., polymeric biomaterial) agonist of innate immunity for use in accordance with the present disclosure may comprise one or more *(e.g.,* at least two or more, including such as, *e.g.,* 2, 3, 4, 5, or more) polymers *(e.g.,* as described herein). In some cases, a biomaterial agonist of innate immunity may be a single polymeric biomaterial or may comprise a plurality *(e.g.,* at least two, at least three or more) polymeric biomaterials that each confers innate immunity agonism (*e.g*., as described herein such as, for example, a carbohydrate polymer [*e.g.,* a polymer that is or comprises a carbohydrate, *e.g*., a carbohydrate backbone, including, *e.g*., but not limited to chitosan, alginate, hyaluronic acid, and/or variants thereof], polyacrylic acid, polyethylenimine (PEI), polyphosphazene, silica gel, and/or variants thereof and/or combinations thereof). In some cases, a biomaterial agonist of innate immunity may comprise at least one or more *(e.g.,* 1, 2, 3, 4, or more) polymers that each confer innate immunity agonism (*e.g*., as described herein such as, for example, a carbohydrate polymer [*e.g.,* a polymer that is or comprises a carbohydrate, *e.g*., a carbohydrate backbone, including, *e.g*., but not limited to chitosan, alginate, hyaluronic acid, and/or variants thereof], polyacrylic acid, polyethylenimine (PEI), polyphosphazene, and/or variants thereof) and at least one or more *(e.g.,* 1, 2, 3, 4, or more) biomaterials *(e.g.,* polymers) that do not necessarily confer such innate immunity agonism. In some cases, such a biomaterial that may not necessarily confer such innate immunity agonism can be a biocompatible and/or biodegradable biomaterial (*e.g*., polymer) that is known in the art. Such a biocompatible and/or biodegradable biomaterial (*e.g*., polymer) can be inert and does not necessarily induce an immune response. For example, in some cases, such a biocompatible and/or biodegradable biomaterial that may co-exist in a biomaterial agonist of innate immunity comprising polymer(s) that confer(s) innate immunity agonism (*e.g*., as described herein such as, for example, a carbohydrate polymer [*e.g.,* a polymer that is or comprises a carbohydrate, *e.g.,* a carbohydrate backbone, including, *e.g.,* but not limited to chitosan, alginate, hyaluronic acid, and/or variants thereof], polyacrylic acid, silica gels, polyethylenimine (PEI), polyphosphazene, and/or variants thereof) may be or comprise cellulose, chitin, chondroitin sulfate, collagen, dextran, gelatin, ethylene-vinyl acetate (EVA), fibrin, poly(lactic-co-glycolic) acid (PLGA), polylactic acid (PLA), polyglycolic acid (PGA), polyethylene glycol (PEG), PEG diacrylate (PEGDA), disulfide-containing PEGDA (PEGSSDA), PEG dimethacrylate (PEGDMA), polydioxanone (PDO), polyhydroxybutyrate (PHB), poly(2-hydroxyethyl methacrylate) (pHEMA), polycarboxybetaine (PCB), polysulfobetaine (PSB), polycaprolactone (PCL), poly(beta-amino ester) (PBAE), poly(ester amide), poly(propylene glycol) (PPG), poly(aspartic acid), poly(glutamic acid), poly(propylene fumarate) (PPF), poly(sebacic anhydride) (PSA), poly(trimethylene carbonate) (PTMC), poly(desaminotyrosyltyrosine alkyl ester carbonate) (PDTE), poly[bis(trifluoroethoxy)phosphazene], polyoxymethylene, single-wall carbon nanotubes, polyanhydride, poly(N-vinyl-2-pyrrolidone) (PVP), poly(vinyl alcohol) (PVA), poly(acrylic acid) (PAA), poly(methacrylic acid) (PMA), polyacetal, poly(alpha ester), poly(ortho ester), polyphosphoester, polyurethane, polycarbonate, polyamide, polyhydroxyalkanoate, polyglycerol, polyglucuronic acid, starch, variants thereof, and/or combinations thereof.

In some cases, a biomaterial (*e.g.,* polymeric biomaterial) agonist of innate immunity for use in accordance with the present disclosure is or comprises one or more carbohydrate polymers. In some cases, a biomaterial agonist of innate immunity may be a biomaterial of a single carbohydrate polymer. In some cases, a biomaterial agonist of innate immunity may be a biomaterial of at least two carbohydrate polymers (including, *e.g.,* at least three carbohydrate polymers, or more). In some cases, a biomaterial agonist of innate immunity may be or comprise a combination of at least one carbohydrate polymer and at least one additional polymer. In some cases, such an additional polymer may be or comprise a different immunomodulatory polymeric biomaterial (*e.g.,* ones described herein). In some cases, such an additional polymer may be inert and does not necessarily induce an immune response.

In some cases, carbohydrate polymer(s) may be present in a biomaterial agonist of innate immunity at a concentration of 0.5%(w/w) to 10%(w/w), or 0.5%(w/w) to 8%(w/w), or 1%(w/w) to 7%(w/w), or 2%(w/w) to 6%(w/w). In some cases, carbohydrate polymer(s) may be present in a biomaterial agonist of innate immunity at higher concentrations, *e.g*., at a concentration of 10%(w/w) or higher, including, *e.g*., but not limited to 11%(w/w), 12%(w/w), 13%(w/w), 14%(w/w), 15%(w/w), 16%(w/w), 17%(w/w), 18%(w/w), 19%(w/w), 20% (w/w), or higher. The concentration of carbohydrate polymer(s) in a biomaterial agonist of innate immunity may be adjusted based on the composition and/or nature of a biomaterial agonist of innate immunity. By way of example only, in some cases where a biomaterial agonist of innate immunity is a preparation of carbohydrate polymer(s) in the absence of any other polymers, higher concentrations of carbohydrate polymer(s) may be used. In some cases where additional polymer(s) is/are included in a biomaterial agonist of innate immunity, lower concentrations of carbohydrate polymer(s) may be used, for example, to achieve one or more desirable material properties as described herein (including, *e.g*., but not limited to storage modulus, viscosity, and/or phase angle). For clarity purposes, the symbol "w/w" means the proportion of a particular polymer within a mixture including polymer(s) and solvent.

In some cases, a biomaterial (*e.g*., polymeric biomaterial) agonist of innate immunity for use in accordance with the present disclosure is or comprises one or more carbohydrate polymers (*e.g*., ones described herein) and at least one thermo-responsive polymer *(e.g.,* a polymer that forms a crosslinked polymer network when exposed to a certain temperature, *e.g*., 35-39°C, or body temperature of a subject to be administered). Examples of thermo-responsive polymers that may be useful in accordance with the present disclosure include, but are not limited to poloxamer (*e.g*., poloxamer 407), poly(N-isopropylacrylamide, chitosan, gelatin, hyaluronic acid, poly(ethylene glycol), poly(ε-caprolactone), poly(organophosphazene), poly[2-(dimethylamino)ethyl methacrylate], hydroxypropylcellulose, poly(vinylcaprolactame), polyvinyl methyl ether, polyacrylic acid, hydroxylethyl succinamide, 2-hydroxymethyl methacrylate, and combinations thereof. Other examples of thermo-responsive polymers as described in Huang et al. "Thermo-sensitive hydorgels for delivering biotherapeutic molecules: A review" Saudi Pharmaceutical Journal (2019) 27(7):990-999; and Zarrintaj et al. "Thermo-sensitive polymers in medicine: A review" European Polymer Journal (2019) 117:402-423, can be useful in accordance with the present disclosure. In some cases, carbohydrate polymer(s) may be present in such a biomaterial agonist of innate immunity at a concentration of 0.5%(w/w) to 10%(w/w), or 0.5%(w/w) to 8%(w/w), or 1%(w/w) to 7%(w/w), or 2%(w/w) to 6%(w/w). In some cases, thermo-responsive polymer(s) may be present at a concentration such that the combination of the thermo-responsive polymer(s) and carbohydrate polymer(s) forms a crosslinked biomaterial agonist of innate immunity when the combination is exposed to 35-39°C, or body temperature (*e.g*., 37°C) of a subject to be administered. In some such cases, a crosslinked biomaterial agonist innate immunity is characterized by one or more of the biomaterial characteristics as described herein, including, *e.g*., storage modulus, viscosity, phase angle, and/or degradation rate.

### A. Chitosan and variants thereof

In some cases, a biomaterial (*e.g*., polymeric biomaterial) agonist of innate immunity described or utilized in accordance with the present disclosure is or comprises chitosan or variants thereof. Examples of chitosan and/or variants thereof that can be utilized in a method described herein include, but are not limited to chitosan, chitosan salts *(e.g.,* chitosan HCl, chitosan chloride, chitosan lactate, chitosan acetate, chitosan glutamate), alkyl chitosan, aromatic chitosan, carboxyalkyl chitosan (*e.g*., carboxymethyl chitosan), hydroxyalkyl chitosan (*e.g*., hydroxypropyl chitosan, hydroxyethyl chitosan), aminoalkyl chitosan, acylated chitosan, phosphorylated chitosan, thiolated chitosan, quaternary ammonium chitosan (*e.g*., N-(2-hydroxyl) propyl-3-trimethyl ammonium chitosan chloride), guanidinyl chitosan, chitosan oligosaccharide, glycated chitosan *(e.g.,* N-dihydrogalactochitosan), and variants or combinations thereof. In some cases, a biomaterial (*e.g*., polymeric biomaterial) agonist of innate immunity described or utilized in accordance with the present disclosure is or comprises carboalkyl chitosan (*e.g*., carboxymethyl chitosan).

Those skilled in the art will appreciate that, in some cases, chitosan and/or variants thereof can be produced by deacetylation of chitin. In some cases, chitosan or variants thereof for use in a biomaterial (*e.g*., polymeric biomaterial) agonist of innate immunity is characterized by degree of deacetylation *(i.e.,* percent of acetyl groups removed) of at least 70% or above, including, *e.g.,* at least 75%, at least 80%, at least 85%, at least 90%, at least 95%, or higher (including up to 100%). In some cases, a chitosan or variants thereof is characterized by degree of deacetylation of no more than 99%, no more than 95%, no more than 90%, no more than 85%, no more than 80%, no more than 75% or lower. Combinations of the above-mentioned ranges are also possible. For example, a chitosan or variants thereof may be characterized by degree of deacetylation of 80%-95%, 70%-95%, or 75%-90%. As will be recognized by one of those skilled in the art, degree of deacetylation (%DA) can be determined by various methods known in the art, *e.g.,* in some cases, by NMR spectroscopy.

In some cases, chitosan or variants thereof utilized in a biomaterial (*e.g.,* polymeric biomaterial) agonist of innate immunity may have an average molecular weight of at least 5 kDa or higher, including, *e.g.,* at least 10 kDa or higher, including, *e.g.,* at least 20 kDa, at least 30 kDa, at least 40 kDa, at least 50 kDa, at least 60 kDa, at least 70 kDa, at least 80 kDa, at least 90 kDa, at least 100 kDa, at least 110 kDa, at least 120 kDa, at least 130 kDa, at least 140 kDa, at least 150 kDa, at least 160 kDa, at least 170 kDa, at least 180 kDa, at least 190 kDa, at least 200 kDa, at least 210 kDa, at least 220 kDa, at least 230 kDa, at least 240 kDa, at least 250 kDa, at least 260 kDa, at least 270 kDa, at least 280 kDa, at least 290 kDa, at least 300 kDa, at least 350 kDa, at least 400 kDa, at least 500 kDa, at least 600 kDa, at least 700 kDa, or higher. In some cases, chitosan or variants thereof utilized in a biomaterial (*e.g.,* polymeric biomaterial) agonist of innate immunity may have an average molecular weight of no more than 750 kDa or lower, including, *e.g.,* no more than 700 kDa, no more than 600 kDa, no more than 500 kDa, no more than 400 kDa, no more than 300 kDa, no more than 200 kDa, no more than 100 kDa, no more than 50 kDa, or lower. Combinations of the above-mentioned ranges are also possible. For example, in some cases, chitosan or variants thereof utilized in a biomaterial (*e.g.,* polymeric biomaterial) agonist of innate immunity is characterized by an average molecular weight of 10 kDa to 700 kDa, or 20 kDa to 700 kDa, or 30 kDa to 500 kDa, or 150 kDa to 600 kDa, or 150 kDa to 400 kDa, or 50 kDa to 150 kDa, or 10 kDa to 50 kDa. In some cases, chitosan or variants thereof utilized in a biomaterial (*e.g.,* polymeric biomaterial) agonist of innate immunity is characterized by an average molecular weight of 20 kDa to 700 kDa, or 30 kDa to 500 kDa. As noted herein, an average molecular weight of chitosan or variants thereof may be a number average molecular weight, weight average molecular weight, or peak average molecular weight.

In some cases, chitosan or variants thereof utilized in a biomaterial (*e.g.,* polymeric biomaterial) agonist of innate immunity is characterized by a molecular weight distribution in a range of 10 kDa to 700 kDa, or 20 kDa or 700 kDa, or 30 kDa to 500 kDa, or 150 kDa to 600 kDa, or 150 kDa to 400 kDa, or 50 kDa to 150 kDa, or 10 kDa to 50 kDa. In some cases, chitosan or variants thereof utilized in a biomaterial (*e.g*., polymeric biomaterial) agonist of innate immunity is characterized by a molecular weight distribution in a range of 20 kDa to 700 kDa, or 30 kDa to 500 kDa.

In some cases, chitosan or variants thereof utilized in a biomaterial (*e.g*., polymeric biomaterial) agonist of innate immunity may be characterized by a viscosity of no more than 3500 mPa·s or lower, including, *e.g.,* no more than 3000 mPa·s, no more than 2500 mPa·s, no more than 2000 mPa·s, no more than 1500 mPa·s, no more than 1000 mPa·s, no more than 500 mPa·s, no more than 250 mPa·s, no more than 200 mPa·s, no more than 150 mPa·s, no more than 100 mPa·s, no more than 75 mPa·s, no more than 50 mPa·s, no more than 25 mPa·s, no more than 20 mPa·s, no more than 15 mPa·s, no more than 10 mPa·s, or lower. In some cases, chitosan or variants thereof may be characterized by a viscosity of at least 5 mPa·s or higher, including, *e.g*., at least 10 mPa·s, at least 20 mPa·s, at least 30 mPa·s, at least 40 mPa·s, at least 50 mPa·s, at least 60 mPa·s, at least 70 mPa·s, at least 80 mPa·s, at least 90 mPa·s, at least 100 mPa·s, at least 125 mPa·s, at least 150 mPa·s, at least 175 mPa·s, at least 250 mPa·s, at least 500 mPa·s, at least 1000 mPa·s, at least 1500 mPa·s, at least 2000 mPa·s, at least 2500 mPa·s, or higher. Combinations of the above-mentioned ranges are also possible. For example, in some cases, such a viscous polymer solution of or comprising chitosan or variants thereof may be characterized by a viscosity of 5 mPa·s to 3000 mPa·s, or 5 mPa·s to 300 mPa·s, 5 mPa·s to 200 mPa·s, or 20 mPa·s to 200 mPa·s, or 5 mPa·s to 20 mPa·s. In some cases, viscosity of chitosan or variants thereof described herein is measured at 1% in 1% acetic acid at 20°C.

In some cases, a biomaterial (*e.g*., polymeric biomaterial) agonist of innate immunity is or comprises at least one or more *(e.g.,* 1, 2, 3 or more) chitosan and/or variants thereof (including, *e.g*., modified chitosan and/or salts of chitosan or modified chitosan such as a chloride salt or a glutamate salt). For example, in some cases, chitosan and/or variants thereof (including, *e.g*., modified chitosan and/or salts of chitosan or modified chitosan such as a chloride salt or a glutamate salt) may be characterized by degree of deacetylation of 70%-95%, or 75%-90%, or 80%-95%, or greater than 90%. In some cases, chitosan and/or variants thereof (including, *e.g*., modified chitosan and/or salts of chitosan or modified chitosan such as a chloride salt or a glutamate salt) may be characterized by an average molecular weight of 10 kDa to 700 kDa, 20 kDa to 600 kDa, 30 kDa to 500 kDa,150 kDa to 400 kDa, or 200 kDa to 600 kDa *(e.g.,* measured as chitosan or chitosan salt, *e.g.,* chitosan acetate). In some cases, chitosan and/or variants thereof (including, *e.g*., modified chitosan and/or salts of chitosan or modified chitosan such as a chloride salt or a glutamate salt) may be characterized by a molecular weight distribution in the range of 10 kDa to 700 kDa, 20 kDa to 600 kDa, 30 kDa to 500 kDa, 150 kDa to 400 kDa, or 200 kDa to 600 kDa *(e.g.,* measured as chitosan or chitosan salt, *e.g*., chitosan acetate). In some cases, chitosan and/or variants thereof (including, *e.g*., salts thereof such as a chloride salt or a glutamate salt) may be characterized by a viscosity ranging from 5 to 3000 mPa·s, or 5 to 300 mPa·s, or 20 to 200 mPa·s. In some cases, such chitosan and/or variants thereof (including, *e.g*., salts thereof such as a chloride salt or a glutamate salt) may be or comprise PROTASAN^{™} UltraPure chitosan chloride and/or chitosan glutamate salt (*e.g*., obtained from NovoMatrix^{®}, which is a business unit of FMC Health and Nutrition (now a part of Du Pont; Product No. CL 113, CL 114, CL 213, CL 214, G 113, G 213, G 214). In some cases, such chitosan and/or variants thereof (including, *e.g*., salts thereof such as a chloride salt or a glutamate salt) may be or comprise chitosan, chitosan oligomers, and/or variants thereof (including, *e.g*., Chitosan HCl, carboxymethyl chitosan, chitosan lactate, chitosan acetate), *e.g*., obtained from Heppe Medical Chitosan GMBH (*e.g*., Chitoceuticals^{®} or Chitoscience^{®}).

In some cases, a biomaterial (*e.g*., polymeric biomaterial) agonist of innate immunity is or comprises carboxyalkyl chitosan (*e.g*., carboxymethyl chitosan) that is characterized by at least one or all of the following characteristics: (1) degree of deacetylation of 80%-95%; (ii) an average molecular weight of 30 kDa to 500 kDa; or a molecular weight distribution of 30 kDa to 500 kDa; and (iii) a viscosity ranging from 5 to 300 mPa·s.

In some cases, a biomaterial (*e.g*., polymeric biomaterial) agonist of innate immunity described or utilized herein is or comprises a variant of chitosan (*e.g*., as described herein). In some cases, such a variant of chitosan may include chemical modification(s) of one or more chemical moieties, *e.g*., hydroxyl and/or amino groups, of the chitosan chains. In some cases, such a variant of chitosan is or comprises a modified chitosan such as, *e.g.,* but not limited to a glycated chitosan (*e.g*., chitosan modified by addition of one or more monosaccharide or oligosaccharide side chains to one or more of its free amino groups). Exemplary glycated chitosan that are useful herein include, *e.g.,* but are not limited to ones described in US 5,747,475, US 6,756,363, WO 2013/109732, US 2018/0312611, and US 2019/0002594.

In some cases, a biomaterial (*e.g*., polymeric biomaterial) agonist of innate immunity described or utilized herein is or comprises chitosan conjugated with a polymer that increases its solubility in aqueous environment (*e.g*., a hydrophilic polymer such as polyethylene glycol).

In some cases, a biomaterial (*e.g*., polymeric biomaterial) agonist of innate immunity described or utilized herein is or comprises thiolated chitosan. Various modifications to chitosans, *e.g*., but not limited to carboxylation, PEGylation, galactosylation (or other glycations), and/or thiolation are known in the art, *e.g.,* as described in Ahmadi et al. Res Pharm Sci., 10(1): 1-16 (2015). Those skilled in the art reading the present disclosure will appreciate that other modified chitosans can be useful for a particular application in which a method is being practiced.

### B. Hyaluronic acid (HA)

In some cases, a biomaterial (*e.g.,* polymeric biomaterial) agonist of innate immunity described or utilized in accordance with the present disclosure is or comprises hyaluronic acid (HA), also known as hyaluronan or hyaluronate.

Without wishing to be bound by a particular theory, the present disclosure observes that biological activities of HA differ, depending on its molecular weight - for example, high molecular weight HA (high MW HA) can possess anti-inflammatory or immunosuppressive activities, while low molecular weight HA (low MW HA) may exhibit pro-inflammatory or immunostimulatory behaviors. See, *e.g.,* Gao et al. "A low molecular weight hyaluronic acid derivative accelerates excisional wound healing by modulating proinflammation, promoting epithelialization and neovascularization, and remodeling collagen" Int J. Mol Sci (2019) 20:3722; Cyphert et al. "Size Matters: Molecular Weight Specificity of Hyaluronan Effects in Cell Biology." Int. J. Cell Biol. (2015) 2015: 563818; Dicker et al. "Hyaluronan: A simple polysaccharide with diverse biological functions" Acta Biomater. (2014) 10:1558-1570; Aya and Stern "Hyaluronan in wound healing: Rediscovering a major player." Wound Repair Regen. (2014) 22:579-593; and Frenkel "The role of hyaluronan in wound healing" Int. Wound J. (2014) 11:159-163. Moreover, the present disclosure observes, without wishing to be bound by a particular theory, that low MW HA *(e.g.,* polydisperse low MW HA) can activate innate immune response, for example, via TLR2 and MyD88; induce production of pro-inflammatory cytokines, including, *e.g*., IL-12, and/or decrease anti-inflammatory signaling, including, *e.g.,* IL-10 production; influence macrophage polarity; and/or stimulate eicosanoids (including, *e.g*., induction of COX2 and/or PGE2 production, *e.g.,* via ERK 1/2 p38 and/or JNK signaling). See, *e.g.,* Monslow et al. "Hyaluronan - a functional and structural sweet spot in the tissue microenvironment" Front. Immunol. (2015) 6: 231 (19 pages); and Alaniz et al. "Low molecular weight hyaluronan preconditioning of tumor-pulsed dendritic cells increases their migratory ability and induces immunity against murine colorectal carcinoma" Cancer Immunol Immunother. (2011) 60(10): 1383-95.

The present disclosure, among other things, provides an insight that low MW HA may be a useful biomaterial agonist of innate immunity, when administered to a target site in a tumor resection subject. In some cases, the present disclosure teaches that certain low HA, for example, such HA having a molecular weight of 500 kDa or less, including, *e.g.,* 350 kDa or less, or 250 kDa or less, or 200 kDa or less, or 150 kDa or less, can be useful as a biomaterial agonist of innate immunity for administration to a target site in a tumor resection subject as described herein.

In some cases, HA that is useful for biomaterial agonists of innate immunity and uses thereof *(e.g.,* as described herein) can be extracted from a natural source *(e.g.,* an animal source) and/or produced via microbial fermentation. In some cases, HA may be a recombinant HA, for example, produced using Gram-positive and/or Gram-negative bacteria as a host, including, *e.g.,* but not limited to *Bacillus sp., Lactococcos lactis, Agrobacterium sp.,* and/or *Escherichia coli.*

### C. Polyacrylic acid polymer

In some cases, a biomaterial (*e.g*., polymeric biomaterial) agonist of innate immunity described or utilized in accordance with the present disclosure is or comprises polyacrylic acid polymer, also known as poly(1-carboxyethylene) or carbomer. In some cases, poly(acrylic acid) polymer may be or comprise homopolymer of acrylic acid. In some cases, poly(acrylic acid) polymer may be or comprise copolymer of acrylic acid, for example, in some cases, copolymer comprising acrylic acid and alkyl acrylate, such as, *e.g.,* C10-C30 alkyl acrylate. In some cases, poly(acrylic acid) polymer may be an interpolymer, for example, in some cases, an interpolymer comprising a poly(acrylic acid) homopolymer or copolymer (*e.g.,* as described or utilized herein) that contains a block polymer of a different kind *(e.g.,* a block homopolymer or copolymer). In some cases, poly(acrylic acid) polymer may be an interpolymer comprising a poly(acrylic acid) homopolymer or copolymer (*e.g.,* as described herein) that contains a block copolymer of polyethylene glycol and a long chain alkyl acid ester.

In some cases, poly(acrylic acid) polymer described or utilized in methods described herein may be crosslinked. One of those skilled in the art will appreciate that, in some cases, poly(acrylic acid) polymer may be crosslinked with allyl sucrose, allyl pentaerythritol, and/or allyl propylene.

One of those skilled in the art reading the present disclosure will also appreciate that various polyacrylic acid polymers used in cosmetics, pharmaceuticals, and personal care products can be utilized in a biomaterial (*e.g.,* polymeric biomaterial) agonist of innate immunity in accordance with the present disclosure. For example, in some cases, polyacrylic acid polymer may be or comprise one or more Carbopol^{®} polymer products from Lubrizol. In some cases, polyacrylic acid polymer may be or comprise one or more poly(acrylic acid) homopolymers (*e.g.,* polymers of acrylic acid crosslinked with allyl sucrose or allyl pentaerythritol) using a polymerization solvent comprising ethyl acetate or a polymerization co-solvent comprising ethyl acetate and cyclohexane. Examples of such carbomer homopolymers include, but are not limited to Carbopol^{®} Polymer 71G NF, 971P NF, 974P NF, 980 NF, 981 NF, 5984 EP, 934NF, 934P NF, 940 NF, and 941 NF. In some cases, polyacrylic acid polymer may be or comprise one or more poly(acrylic acid) copolymers (*e.g.,* polymers of acrylic acid and C10-C30 alkyl acrylate crosslinked with allyl pentaerythritol) using a polymerization solvent comprising benzene or a polymerization cosolvent comprising ethyl acetate and cyclohexane. Exemplary such poly(acrylic acid) copolymers include, but are not limited to Carbopol^{®} Polymer 1342 NF, Pemulen^{™} TR-1 NF, and/or Pemulen^{™} TR-2 NF. In some cases, poly(acrylic acid) polymer may be or comprise one or more poly(acrylic acid) interpolymer (*e.g*., a poly(acrylic acid) homopolymer or copolymer (*e.g*., as described or utilized herein) that contains a block copolymer of polyethylene glycol and a long chain alkyl acid ester) using a polymerization cosolvent comprising ethyl acetate and cyclohexane. Non-limiting examples of such poly(acrylic acid) interpolymers include Carbopol^{®} Polymer ETD 2020 NF and Ultrez 10 NF.

### II. Pharmaceutical Compositions

In some cases, a composition can be formulated in accordance with routine procedures as a pharmaceutical composition for administration to a subject in need thereof *(e.g.,* as described herein). In some cases, such a pharmaceutical composition can include a pharmaceutically acceptable carrier or excipient, which, as used herein, includes any and all solvents, dispersion media, diluents, or other liquid vehicles, dispersion or suspension aids, surface active agents, isotonic agents, thickening or emulsifying agents, preservatives, solid binders, lubricants and the like, as suited to the particular dosage form desired. Remington's The Science and Practice of Pharmacy, 21st Edition, A. R. Gennaro (Lippincott, Williams & Wilkins, Baltimore, MD, 2006) discloses various excipients used in formulating pharmaceutical compositions and known techniques for the preparation thereof. Suitable pharmaceutically acceptable carriers include but are not limited to water, salt solutions (*e.g*., NaCl), saline, buffered saline, glycerol, sugars such as mannitol, lactose, trehalose, sucrose, or others, dextrose, fatty acid esters, *etc.,* as well as combinations thereof.

A pharmaceutical composition can, if desired, be mixed with auxiliary agents (*e.g*., lubricants, preservatives, stabilizers, wetting agents, emulsifiers, salts for influencing osmotic pressure, buffers, coloring, flavoring and/or aromatic substances and the like), which do not deleteriously react with the active compounds or interfere with their activity. In some cases, a pharmaceutical composition can be sterile.

A suitable pharmaceutical composition, if desired, can also contain minor amounts of wetting or emulsifying agents, or pH buffering agents. A pharmaceutical composition can be a liquid solution, suspension, or emulsion.

A pharmaceutical composition can be formulated in accordance with the routine procedures as a pharmaceutical composition adapted for administration to human beings. The formulation of a pharmaceutical composition should suit the mode of administration. For example, in some cases, a pharmaceutical composition for injection may typically comprise sterile isotonic aqueous buffer. Where necessary, a pharmaceutical composition may also include a local anesthetic to ease pain at a site of injection. In some cases, components of a pharmaceutical composition (*e.g.,* as described herein) are supplied separately or mixed together in a single-use form, for example, as a dry lyophilized powder or water free concentrate in a hermetically sealed container such as an ampule or sachet or in a sterile syringe indicating the quantity of a biomaterial (*e.g.,* polymeric biomaterial) agonist of innate immunity. Where a pharmaceutical composition is to be administered by injection, in some cases, dry lyophilized powder, *e.g.,* of precursor component(s) of a biomaterial *(e.g.,* polymeric biomaterial) agonist of innate immunity, can be reconstituted with an aqueous buffered solution and then injected to a target site in a subject in need thereof.

Although the descriptions of pharmaceutical compositions described herein are principally directed to pharmaceutical compositions that are suitable for ethical administration to humans, it will be understood by the skilled artisan that such compositions are generally suitable for administration to animals of all sorts or cells *in vitro* or *ex vivo.* Modification of pharmaceutical compositions suitable for administration to humans in order to render the compositions suitable for administration to various animals or cells *in vitro* or *ex vivo* is well understood, and the ordinarily skilled practitioner, *e.g.,* a veterinary pharmacologist, can design and/or perform such modification with merely ordinary, if any, experimentation.

Formulations of the pharmaceutical compositions described herein may be prepared by any method known or hereafter developed in the art of pharmacology. For example, such preparatory methods include step of bringing the precursor component(s) of a biomaterial (*e.g.,* polymeric biomaterial) agonist of innate immunity into association with a diluent or another excipient and/or one or more other accessory ingredients and then, if necessary and/or desirable, shaping and/or packaging the product into a desired single-use unit or multi-use units. Alternatively, such preparatory methods may also include a step of forming a polymer network biomaterial from precursor component(s) thereof, prior to shaping and/or packaging the product into a desired single-use units or multi-use units.

A pharmaceutical composition in accordance with the present disclosure may be prepared, packaged, and/or sold in bulk, as a single-use unit, and/or as a plurality of single-use units. As used herein, a "single-use unit" is a discrete amount of a pharmaceutical composition described herein. For example, a single-use unit of a pharmaceutical composition comprises a predetermined amount of a biomaterial (*e.g.,* polymeric biomaterial) agonist of innate immunity (*e.g.,* in the absence of an immunomodulatory payload), which in some cases can be or comprise a pre-formed polymer network biomaterial, or in some cases can be or comprise precursor component(s) of a polymer network biomaterial and optionally a crosslinking agent, if any.

The relative amount of a biomaterial (*e.g.,* polymeric biomaterial) agonist of innate immunity (*e.g.,* as a pre-formed polymer network biomaterial or as precursor component(s) of such a polymer network biomaterial) and, optionally, any additional agents in pharmaceutical compositions described herein, *e.g.,* a pharmaceutically acceptable excipient and/or any additional ingredients, can vary, depending upon, *e.g.,* size of target site, injection volume, physical and medical condition of a subject to be treated, types of cancer, and may also further depend upon the route by which such a pharmaceutical composition is to be administered. In some cases, a biomaterial (*e.g.,* polymeric biomaterial) agonist of innate immunity is provided in an effective amount in a pharmaceutical composition to induce innate immunity in at least one or more aspects as described herein. In some cases, a biomaterial (*e.g.,* polymeric biomaterial) agonist of innate immunity is provided in an effective amount in a pharmaceutical composition to induce anti-tumor immunity. In some cases, a biomaterial (*e.g.,* polymeric biomaterial) agonist of innate immunity is provided in an effective amount in a pharmaceutical composition to inhibit or reduce risk or incidence of tumor recurrence and/or metastasis. In certain cases, the effective amount is a therapeutically effective amount of a biomaterial (*e.g.,* polymeric biomaterial) agonist of innate immunity. In certain cases, the effective amount is a prophylactically effective amount of a biomaterial (*e.g.,* polymeric biomaterial) agonist of innate immunity.

In certain cases, a pharmaceutical composition consists essentially of or consists of a biomaterial *(e.g.,* polymeric biomaterial) agonist of innate immunity *(e.g.,* ones as described herein); to the extent that such a composition may include one or more material(s)/agents other than the biomaterial, such other material(s)/agent(s) do not, individually, or together, materially alter relevant innate immunity modulatory characteristic(s) of the biomaterial. In some cases, such a pharmaceutical composition may be substantially free of an innate immunity modulatory payload.

In certain cases, pharmaceutical compositions do not include cells. In certain cases, pharmaceutical compositions do not include adoptively transferred cells. In certain cases, pharmaceutical compositions do not include T cells. In certain cases, pharmaceutical compositions do not include tumor antigens. In certain cases, pharmaceutical compositions do not include tumor antigens loaded *ex vivo.*

In certain cases, a pharmaceutical composition is in liquid form. In certain cases, a pharmaceutical composition is in a solid form *(e.g.,* a gel form, a crystal form, *etc*.)*.* In certain cases, the transition from a liquid form to a solid form may occur upon sufficient crosslinking such that the resulting material has a storage modulus consistent with a solid form that allows it to be physically manipulated and implanted in a surgical procedure. Accordingly, in some cases, a solid form may be amenable for carrying out an intended use of the present disclosure (*e.g*., surgical implantation). In certain cases, the transition from a liquid form to a solid form may occur upon thermal crosslinking *in situ* such that the resulting material has a storage modulus consistent with a solid form. In certain cases, a pharmaceutical composition is a suspension. For example, solid particles (*e.g.,* crystals such as inorganic crystals) may be delivered as suspensions.

### III. Therapeutics Uses

Technologies described herein are useful for inducing innate immune response in a subject in need thereof. For example, in some cases, technologies described herein are useful for inducing anti-tumor immunity. In some cases, technologies described herein are useful for treating cancer. In some cases, technologies described herein are useful to delay the onset of, slow the progression of, or ameliorate one or more symptoms of cancer. In some cases, technologies described herein are useful to reduce or inhibit primary tumor regrowth. In some cases, technologies described herein are useful for reducing or inhibiting incidence of tumor recurrence and/or metastasis. Accordingly, some aspects relate to methods of administering to a target site in a subject who is undergoing or has undergone tumor removal *(e.g.,* by surgical tumor resection), a composition comprising an innate immunity modulatory component and, optionally, one or more other components *(i.e.,* one or more materials/agents that is not an innate immunity modulatory agent). In some cases, an innate immunity modulatory component consists essentially of or consists of a biomaterial. In some cases, an innate immunity modulatory component consists essentially of or consists of a polymeric biomaterial. In some cases, the innate immunity modulatory component of a composition as described herein consists essentially of or consists of such a biomaterial *(e.g.,* polymeric biomaterial); to the extent that such a composition may include one or more material(s)/agent(s) other than the biomaterial (*e.g.,* polymeric biomaterial), such other material(s)/agent(s) do not, individually or together, materially alter relevant innate immunity modulatory characteristic(s) of the biomaterial (*e.g.,* polymeric biomaterial).

In some cases, such a composition may comprise an innate immunity modulatory component that is substantially free of an innate immunity modulatory payload, wherein the innate immunity modulatory component comprises a biomaterial (*e.g.,* polymeric biomaterial) agonist of innate immunity (*e.g.,* as described and/or utilized herein). In some cases, such a composition may be substantially free of any immunomodulatory payload. In some cases, such a composition utilized in methods of the present disclosure may be formulated as a pharmaceutical composition described herein.

In certain cases, a method described herein comprises administering a composition to a target site in a subject in need thereof after removal of tumor, for example, after removal of greater than or equal to 50% or higher, by weight, of the subject's tumor, including, *e.g*., greater than or equal to 55%, greater than or equal to 60%, greater than or equal to 65%, greater than or equal to 70%, greater than or equal to 75%, greater than or equal to 80%, greater than or equal to 85%, greater than or equal to 90%, greater than or equal to 95%, greater than or equal to 96%, greater than or equal to 97%, greater than or equal to 98%, or greater than or equal to 99%, by weight, of the subject's tumor. In certain cases, a method described herein comprises administering a composition to a target site in a subject in need thereof after removal of greater than or equal to 50% or higher, by volume, of the subject's tumor, including, *e.g*., greater than or equal to 55%, greater than or equal to 60%, greater than or equal to 65%, greater than or equal to 70%, greater than or equal to 75%, greater than or equal to 80%, greater than or equal to 85%, greater than or equal to 90%, greater than or equal to 95%, greater than or equal to 96%, greater than or equal to 97%, greater than or equal to 98%, or greater than or equal to 99%, by volume, of the subject's tumor. In some cases, a method described herein comprises performing a tumor resection to remove a subject's tumor, prior to administration of a composition.

In some cases, a composition described and/or utilized herein is administered to a target site in a tumor resection subject immediately after the subject's tumor has been removed by surgical tumor resection. In some cases, a composition described and/or utilized herein is intraoperatively administered to a target site in a tumor section subject. In some cases, a composition described and/or utilized herein is postoperatively administered to a target site in a tumor resection subject within 24 hours or less, including, *e.g*., within 18 hours, within 12 hours, within 6 hours, within 3 hours, within 2 hours, within 1 hour, within 30 mins, or less, after the subject's tumor has been removed by surgical tumor resection.

In some cases, a target site for administration is or comprises a tumor resection site. In some cases, such a tumor resection site may be characterized by absence of gross residual tumor antigen. In some cases, such a tumor resection site may be characterized by a negative resection margin *(i.e.,* no cancer cells seen microscopically at the resection margin, *e.g.,* based on histological assessment of tissues surrounding the tumor resection site). In some cases, such a tumor resection site may be characterized by a positive resection margin *(i.e.,* cancer cells are seen microscopically at the resection margin, *e.g.,* based on histological assessment of tissues surrounding the tumor resection site). In some cases, such a tumor resection site may be characterized by presence of gross residual tumor antigen. In some cases, a target site for administration is or comprises a site in close proximity (*e.g.,* within 4 inches, within 3.5 inches, within 3 inches, within 2.5 inches, within 2 inches, within 1.5 inches, within 1 inches, within 0.5 inches, within 0.4 inches, within 0.3 inches, within 0.2 inches, within 0.1 inches or less) to a tumor resection site. In some cases, a target site for administration is or comprises a sentinel lymph node. In some cases, a target site for administration is or comprises a draining lymph node.

As will be understood by one of ordinary skill in the art, compositions that are useful in accordance with the present disclosure can be administered to a target site in subjects in need thereof using appropriate delivery approaches known in the art. For example, in some cases, described technologies can be amenable for administration by injection. Among other things, the present disclosure appreciates that minimally invasive procedures *(e.g.,* small and/or accessible or cutaneous excisions) may produce less inflammation *(e.g.,* inflammation that is associated with immunosuppression) than open surgeries or procedures that access internal organs. Among other things, the present disclosure appreciates that minimally invasive surgery (MIS), *e.g*., robot-assisted MIS, robotic surgery, and/or laparoscopic surgery, which, for example, typically involve one or more small incisions, to perform tumor resection and/or to administer a composition described herein may produce less inflammation (*e.g*., inflammation that is associated with immunosuppression) than open surgery. For example, in some cases, the present disclosure, among other things, provides an insight that technologies described herein may be particularly useful and/or effective in the absence of an immunomodulatory payload when tumor resection and/or administration of a composition described herein is performed by minimally invasive surgery (MIS), *e.g.,* robot-assisted MIS, robotic surgery, and/or laparoscopic surgery, which, for example, typically involve one or more small incisions. In some cases, the present disclosure, among other things, provides an insight that technologies described herein may be particularly useful and/or effective in the absence of an immunomodulatory payload when tumor resection and/or administration of a composition described herein is performed in the context of accessible and/or cutaneous excisions. In some cases, described technologies can be amenable for administration (*e.g.,* by injection) intraoperatively as part of minimally invasive procedure, *e.g.,* miminally invasive surgery (MIS), *e.g.,* robot-assisted MIS, robotic surgery, and/or laparoscopic surgery, and/or procedure that involves one or more accessible and/or cutaneous excisions. In some cases, described technologies can be amenable for administration *(e.g.,* by injection) involving a robotic surgical system *(e.g.,* a da Vinci System), *e.g*., in some cases for minimally invasive administration. For example, in some cases, a composition that may be useful for injection and/or in the context of minimally invasive procedure, *e.g.,* minimally invasive surgery (MIS), *e.g.,* robot-assisted MIS, robotic surgery, and/or laparoscopic surgery and/or procedure that involves one or more accessible and/or cutaneous excisions, is liquid and a biomaterial (*e.g*., polymeric biomaterial) agonist of innate immunity in such a composition is or comprises a viscous polymer solution. In some cases, such a viscous polymer solution is pre-made prior to injecting to a target site.

In other cases, a composition that may be useful for injection and/or in the context of a minimally invasive procedure (*e.g*., small and/or accessible or cutaneous excisions), or a minimally invasive surgery (MIS), *e.g*., robot-assisted MIS, robotic surgery, and/or laparoscopic surgery, is liquid and a biomaterial (*e.g*., polymeric biomaterial) agonist of innate immunity in such a composition forms a polymer network biomaterial (*e.g*., as described and/or utilized herein) *in situ* at a target site to which it is injected. For example, in certain cases, precursor component(s) of a biomaterial (*e.g*., polymeric biomaterial) agonist of innate immunity are administered separately to a target site in a subject *(e.g.,* at the site of tumor resection). In some cases, a non-crosslinked polymer network is formed *in situ* at a target site *(e.g.,* a tumor resection site). To form a crosslinked polymer network *in vivo,* in some cases, a crosslinking agent *(e.g.,* as described herein) is also administered to a target site in a subject in need thereof. In certain cases, precursor component(s) of a biomaterial (*e.g.,* polymeric biomaterial) agonist of innate immunity and a crosslinking agent are administered sequentially. In certain cases, precursor component(s) of a biomaterial (*e.g.,* polymeric biomaterial) agonist of innate immunity and a crosslinking agent are administered concurrently. In certain cases, precursor component(s) of a biomaterial (*e.g.,* polymeric biomaterial) agonist of innate immunity and a crosslinking agent are administered as a mixture.

In some cases, administration as described herein involves administration of one or more precursor components of a biomaterial (*e.g.,* polymeric biomaterial) agonist of innate immunity that interact or react *in situ* to form a polymer network biomaterial as described herein; in some such cases, such interaction or reaction involves crosslinking that may, in some cases, occur spontaneously and may, in some cases, be triggered by application of an agent *(e.g.,* a catalyst and/or a reactant) and/or a condition *(e.g.,* one or more of heat, pH, pressure, electromagnetic radiation which may be at a particular wavelength, *etc*.). In some cases, precursor component(s) of a biomaterial (*e.g.,* polymeric biomaterial) agonist of innate immunity and a crosslinking agent are administered (*e.g.,* by injection) separately to a subject *(e.g.,* at the site of tumor resection). In some cases, precursor component(s) of a biomaterial (*e.g.,* polymeric biomaterial) agonist of innate immunity and a crosslinking agent are administered (*e.g.,* by injection) sequentially. In some cases, precursor component(s) of a biomaterial (*e.g.,* polymeric biomaterial) agonist of innate immunity and a crosslinking agent are administered (*e.g.,* by injection) concurrently. In certain cases, precursor component(s) of a biomaterial (*e.g.,* polymeric biomaterial) agonist of innate immunity and a crosslinking agent are administered as a single mixture. By way of example only, in some cases, chitosan and a crosslinking agent can be administered separately (*e.g.,* chitosan and an ionic crosslinking agent such as, *e.g.,* tripolyphosphate) or as a single mixture *(e.g.,* chitosan and a thermo-responsive crosslinking agent such as a thermo-responsive polymer, which in some cases, may be or comprise poloxamer such as, *e.g.,* poloxamer 407) to a subject *(e.g.,* at a tumor resection site) to form a polymer network biomaterial comprising chitosan *in vivo.* As another example, in some cases, alginate and a crosslinking agent *(e.g.,* salt such as calcium salt) can be administered separately or as a single mixture to a subject *(e.g.,* at a tumor resection site) to form a polymer network biomaterial comprising alginate *in vivo.* In some cases, HA *(e.g.,* low MW HA) and a crosslinking agent can be administered separately *(e.g.,* HA and a chemical crosslinking agent such as, *e.g*., divinylsulfone) or as a single mixture *(e.g.,* HA and a thermal-responsive crosslinking agent such as a thermo-responsive polymer, which in some cases, may be or comprise poloxamer such as, *e.g*., poloxamer 407) to a subject *(e.g.,* at a tumor resection site) to form a polymer network biomaterial comprising HA *in vivo.*

In some cases, technologies described herein can be amenable for administration by implantation. For example, in some cases, a biomaterial (*e.g.,* polymeric biomaterial) agonist of innate immunity described in a composition in accordance with the present disclosure is a pre-formed polymer network biomaterial, which in some cases can be or comprise a crosslinked polymer network biomaterial and/or a non-crosslinked polymer network biomaterial. An exemplary polymer network biomaterial is or comprises a hydrogel. For example, in some cases, a composition may be administered by surgical implantation to a tumor resection site *(e.g.,* void volume resulting from tumor resection). In some cases, a composition may be administered by surgical implantation to a tumor resection site and affixed with a bioadhesive. In some cases, administration may be performed intraoperatively *(i.e.,* immediately after tumor resection).

In some cases, the amount of a polymeric biomaterial agonist of innate immunity to achieve desirable therapeutic effect(s) such as, *e.g*., anti-tumor immunity, may vary from subject to subject, depending, for example, on gender, age, and general condition of a subject, type and/or severity of cancer, efficacy of a polymeric biomaterial agonist of innate immunity, and the like.

In some cases, the present disclosure provides technologies such that administration of a biomaterial *(e.g.,* polymeric biomaterial) agonist of innate immunity *(e.g.,* as described and/or utilized herein) by itself is sufficient to provide antitumor immunity and thus does not necessarily require administration of, *e.g.,* a tumor antigen, and/or adoptive transfer of immune cells *(e.g.,* T cells) to a subject in need thereof *(e.g.,* as described herein). Accordingly, in some cases, technologies described herein do not include administering a tumor antigen to a subject, *e.g.,* within 1 month or less (including, *e.g.,* within 3 weeks, within 2 weeks, within 1 week, within 5 days, within 3 days, within 1 day, within 12 hours, within 6 hours), after the subject has received a composition as described and/or utilized herein. In certain cases, technologies described herein do not include adoptive transfer of immune cells *(e.g.,* T cells) to a subject, *e.g.,* within 1 month or less (including, *e.g.,* within 3 weeks, within 2 weeks, within 1 week, within 5 days, within 3 days, within 1 day, within 12 hours, within 6 hours) after the subject has received a composition as described and/or utilized herein.

In some cases, the present disclosure provides technologies such that administration of a biomaterial *(e.g.,* polymeric biomaterial) agonist of innate immunity *(e.g.,* as described and/or utilized herein) by itself is sufficient to elicit or promote antitumor immunity and thus does not necessarily require administration of an immunomodulatory payload to a subject in need thereof *(e.g.,* as described herein). Accordingly, in some cases, technologies described herein do not include administering an immunomodulatory payload to a subject, *e.g.,* within 1 month or less (including, *e.g.,* within 3 weeks, within 2 weeks, within 1 week, within 5 days, within 3 days, within 1 day, within 12 hours, within 6 hours), after the subject has received a composition as described and/or utilized herein.

In some cases, technologies described herein are useful for treatment of cancer in a subject. In some cases, technologies described herein are for use in treatment of a resectable tumor. In some cases, technologies described herein are for use in treatment of a solid tumor *(e.g.,* but not limited to a blastoma, a carcinoma, a germ cell tumor, and/or a sarcoma). In some cases, technologies described herein are for use in treatment of lymphoma present in a spleen or a tissue outside of a lymphatic system, *e.g*., a thyroid or stomach.

In some cases, technologies described herein are useful for treating a cancer including, but not limited to, acoustic neuroma; adenocarcinoma; adrenal gland cancer; anal cancer; angiosarcoma (*e.g.,* lymphangiosarcoma, lymphangioendotheliosarcoma, hemangiosarcoma); appendix cancer; benign monoclonal gammopathy; biliary cancer *(e.g.,* cholangiocarcinoma); bile duct cancer; bladder cancer; bone cancer; breast cancer *(e.g.,* adenocarcinoma of the breast, papillary carcinoma of the breast, mammary cancer, medullary carcinoma of the breast); brain cancer (*e.g.,* meningioma, glioblastomas, glioma *(e.g.,* astrocytoma, oligodendroglioma, medulloblastoma); bronchus cancer; carcinoid tumor; cardiac tumor; cervical cancer (*e.g.,* cervical adenocarcinoma); choriocarcinoma; chordoma; craniopharyngioma; colorectal cancer *(e.g.,* colon cancer, rectal cancer, colorectal adenocarcinoma); connective tissue cancer; epithelial carcinoma; ductal carcinoma *in situ;* ependymoma; endotheliosarcoma (e.g., Kaposi's sarcoma, multiple idiopathic hemorrhagic sarcoma); endometrial cancer *(e.g.,* uterine cancer, uterine sarcoma); esophageal cancer *(e.g.,* adenocarcinoma of the esophagus, Barrett's adenocarcinoma); Ewing's sarcoma; eye cancer (*e.g.,* intraocular melanoma, retinoblastoma); familiar hypereosinophilia; gall bladder cancer; gastric cancer (*e.g.,* stomach adenocarcinoma); gastrointestinal stromal tumor (GIST); germ cell cancer; head and neck cancer (*e.g.,* head and neck squamous cell carcinoma, oral cancer *(e.g.,* oral squamous cell carcinoma), throat cancer *(e.g.,* laryngeal cancer, pharyngeal cancer, nasopharyngeal cancer, oropharyngeal cancer); hematopoietic cancer (*e.g.,* lymphomas, primary pulmonary lymphomas, bronchus-associated lymphoid tissue lymphomas, splenic lymphomas, nodal marginal zone lymphomas, pediatric B cell non-Hodgkin lymphomas); hemangioblastoma; histiocytosis; hypopharynx cancer; inflammatory myofibroblastic tumors; immunocytic amyloidosis; kidney cancer (*e.g.,* nephroblastoma a.k.a. Wilms' tumor, renal cell carcinoma); liver cancer (*e.g.,* hepatocellular cancer (HCC), malignant hepatoma); lung cancer (*e.g.,* bronchogenic carcinoma, small cell lung cancer (SCLC), non-small cell lung cancer (NSCLC), adenocarcinoma of the lung); leiomyosarcoma (LMS); melanoma; midline tract carcinoma; multiple endocrine neoplasia syndrome; muscle cancer; mesothelioma; nasopharynx cancer; neuroblastoma; neurofibroma (*e.g.,* neurofibromatosis (NF) type 1 or type 2, schwannomatosis); neuroendocrine cancer (*e.g.,* gastroenteropancreatic neuroendocrine tumor (GEP-NET), carcinoid tumor); osteosarcoma (*e.g.,* bone cancer); ovarian cancer (*e.g.,* cystadenocarcinoma, ovarian embryonal carcinoma, ovarian adenocarcinoma); papillary adenocarcinoma; pancreatic cancer (*e.g.,* pancreatic andenocarcinoma, intraductal papillary mucinous neoplasm (IPMN), Islet cell tumors); parathryroid cancer; papillary adenocarcinoma; penile cancer (*e.g.,* Paget's disease of the penis and scrotum); pharyngeal cancer; pinealoma; pituitary cancer; pleuropulmonary blastoma; primitive neuroectodermal tumor (PNT); plasma cell neoplasia; paraneoplastic syndromes; intraepithelial neoplasms; prostate cancer (*e.g.,* prostate adenocarcinoma); rectal cancer; rhabdomyosarcoma; retinoblastoma; salivary gland cancer; skin cancer *(e.g.,* squamous cell carcinoma (SCC), keratoacanthoma (KA), melanoma, basal cell carcinoma (BCC)); small bowel cancer *(e.g.,* appendix cancer); soft tissue sarcoma *(e.g.,* malignant fibrous histiocytoma (MFH), liposarcoma, malignant peripheral nerve sheath tumor (MPNST), chondrosarcoma, fibrosarcoma, myxosarcoma); sebaceous gland carcinoma; stomach cancer; small intestine cancer; sweat gland carcinoma; synovioma; testicular cancer *(e.g.,* seminoma, testicular embryonal carcinoma); thymic cancer; thyroid cancer *(e.g.,* papillary carcinoma of the thyroid, papillary thyroid carcinoma (PTC), medullary thyroid cancer); urethral cancer; uterine cancer; vaginal cancer; vulvar cancer (*e.g.,* Paget's disease of the vulva), or any combination thereof.

In certain cases, the cancer is breast cancer. In certain cases, the cancer is skin cancer. In certain cases, the cancer is melanoma. In certain cases, the cancer is lung cancer. In certain cases, the cancer is kidney cancer. In certain cases, the cancer is liver cancer. In certain cases, the cancer is pancreatic cancer. In certain cases, the cancer is colorectal cancer. In certain cases, the cancer is bladder cancer. In certain cases, the cancer is lymphoma. In certain cases, the cancer is prostate cancer. In certain cases, the cancer is thyroid cancer. In certain cases, the cancer is brain cancer. In certain cases, the cancer is stomach cancer. In certain cases, the cancer is esophageal cancer.

In some cases, technologies described herein are useful in treating adenocarcinoma, adrenal gland cancer, anal cancer, angiosarcoma, appendix cancer, bile duct cancer, bladder cancer, bone cancer, brain cancer, breast cancer, bronchus cancer, carcinoid tumor, cardiac tumor, cervical cancer, choriocarcinoma, chordoma, colorectal cancer, connective tissue cancer, craniopharyngioma, ductal carcinoma *in situ,* endotheliosarcoma, endometrial cancer, ependymoma, epithelial carcinoma, esophageal cancer, Ewing's sarcoma, eye cancer, familiar hypereosinophilia, gall bladder cancer, gastric cancer, gastrointestinal carcinoid tumor, gastrointestinal stromal tumor (GIST), germ cell cancer, head and neck cancer, hemangioblastoma, histiocytosis, Hodgkin lymphoma, hypopharynx cancer, inflammatory myofibroblastic tumors, intraepithelial neoplasms, immunocytic amyloidosis, Kaposi sarcoma, kidney cancer, liver cancer, lung cancer, leiomyosarcoma (LMS), melanoma, midline tract carcinoma, multiple endocrine neoplasia syndrome, muscle cancer, mesothelioma, myeloproliferative disorder (MPD), nasopharynx cancer, neuroblastoma, neurofibroma, neuroendocrine cancer, non-Hodgkin lymphoma, osteosarcoma, ovarian cancer, pancreatic cancer, paraneoplastic syndromes, parathryroid cancer, papillary adenocarcinoma, penile cancer, pharyngeal cancer, pheochromocytoma, pinealoma, pituitary cancer, pleuropulmonary blastoma, primitive neuroectodermal tumor (PNT), plasma cell neoplasia, prostate cancer, rectal cancer, retinoblastoma, rhabdomyosarcoma, salivary gland cancer, sebaceous gland carcinoma, skin cancer, small bowel cancer, small intestine cancer, soft tissue sarcoma, stomach cancer, sweat gland carcinoma, synovioma, testicular cancer, thymic cancer, thyroid cancer, urethral cancer, uterine cancer, vaginal cancer, vascular cancer, vulvar cancer, or a combination thereof.

In some cases, a method described herein may comprise administering to a target site *(e.g.,* as described herein) in a tumor resection subject a described composition and, optionally, monitoring the tumor resection site or distal sites for risk or incidence of tumor regrowth or tumor outgrowth in the subject after the administration, *e.g*., every 3 months or longer after the administration, including, *e.g*., every 6 months, every 9 months, every year, or longer. When the subject is determined to have risk or incidence of tumor recurrence based on the monitoring report, in some cases, a subject can be administered with a second composition *(e.g.,* as described herein) and/or a different treatment regimen *(e.g.,* chemotherapy).

In some cases, technologies described herein may be useful for treating subjects who are suffering from metastatic cancer. For example, in some cases, a method described herein may comprise administering to a target site *(e.g.,* as described herein) in a subject suffering from one or more metastases who has undergone a tumor resection (*e.g.,* surgical resection of a primary tumor) and, optionally, monitoring at least one metastatic site in the subject after the administration, *e.g*., every 3 months or longer after the administration, including, *e.g*., every 6 months, every 9 months, every year, or longer. Based on results of the monitoring report, in some cases, a subject can be administered with a second composition *(e.g.,* as described herein) and/or a different treatment regimen *(e.g.,* chemotherapy).

In certain cases, the methods described herein do not comprise administering a described composition prior to tumor resection.

It will be also appreciated that compositions described herein can be administered in combination with one or more additional pharmaceutical agents. For example, compositions can be administered in combination with additional pharmaceutical agents that reduce and/or modify their metabolism, inhibit their excretion, and/or modify their distribution within the body. It will also be appreciated that the additional therapy employed may achieve a desired effect for the same disorder, and/or it may achieve different effects. In certain cases, an additional pharmaceutical agent is not adoptively transferred cells. In certain cases, an additional pharmaceutical agent is not T cells. In certain cases, an additional pharmaceutical agent is administered multiple days or weeks after administration of a composition described herein.

In certain cases, a subject being treated is a mammal. In certain cases, a subject is a human. In certain cases, a subject is a human patient who has received neoadjuvant (pre-operative) therapy. In certain cases, a subject is a human patient who has not received neoadjuvant therapy. In certain cases, a subject is a human patient who has received neoadjuvant (pre-operative) chemotherapy. In certain cases, a subject is a human patient who has received neoadjuvant radiation therapy. In certain cases, a subject is a human patient who has received neoadjuvant chemotherapy and radiation therapy. In certain cases, a subject is a human patient who has received neoadjuvant molecular targeted therapy. In certain cases, a subject is a human patient who has received neoadjuvant immunotherapy, including immune checkpoint blockade (*e.g.,* anti-CTLA-4, anti-PD-1, and/or anti-PD-L1). In certain cases, a subject is a human patient who has not received neoadjuvant immunotherapy, including immune checkpoint blockade (*e.g.,* anti-CTLA-4, anti-PD-1, and/or anti-PD-L1). In certain cases, a subject is a human patient whose tumor has not objectively responded to neoadjuvant therapy (as defined by Response Evaluation Criteria in Solid Tumors (RECIST) or immunerelated Response Criteria (irRC)) *(e.g.,* stable disease, progressive disease). In certain cases, a subject is a human patient whose target lesion has objectively responded to neoadjuvant therapy (*e.g.,* partial response, complete response). Non-target lesions may exhibit an incomplete response, stable disease, or progressive disease. In certain cases, a subject is a human patient who would be eligible to receive immunotherapy in an adjuvant (postoperative) setting. In certain cases, a subject is a domesticated animal, such as a dog, cat, cow, pig, horse, sheep, or goat. In certain cases, a subject is a companion animal such as a dog or cat. In certain cases, a subject is a livestock animal such as a cow, pig, horse, sheep, or goat. In certain cases, a subject is a zoo animal. In another case, a subject is a research animal, such as a rodent, pig, dog, or non-human primate. In certain cases, a subject is a non-human transgenic animal such as a transgenic mouse or transgenic pig.

### IV. Kits

The present disclosure also provides kits that find use in practicing technologies as described herein. In some cases, a kit comprises a composition or a pharmaceutical composition described herein and a container (*e.g.,* a vial, ampule, bottle, syringe, and/or dispenser package, or other suitable container). In some cases, one or more component(s) of a composition or a pharmaceutical composition described herein are separately provided in one or more containers. For example, individual precursor component(s) of a biomaterial (*e.g.,* polymeric biomaterial) agonist of innate immunity (*e.g.,* ones described herein, for example, but not limited to chitosan or variants thereof) may be, in some cases, provided in separate containers. In some cases, individual precursor component(s) of a biomaterial (*e.g.,* polymeric biomaterial) agonist of innate immunity (*e.g.,* ones described herein, for example, but not limited to chitosan or variants thereof) may be provided as dry lyophilized powder. In some cases, individual precursor component(s) of a biomaterial (*e.g.,* polymeric biomaterial) agonist of innate immunity (*e.g.,* ones described herein, for example, but not limited to chitosan or variants thereof) may be provided as dry particles. In some cases, individual precursor component(s) of a polymeric biomaterial agonist of innate immunity (*e.g.,* ones described herein, for example, but not limited to chitosan or variants thereof) may be provided as liquid. In some cases, a pre-formed biomaterial (*e.g.,* polymeric biomaterial) agonist of innate immunity (in a form of polymer network biomaterial) may be provided in a container. In some cases, such a pre-formed biomaterial (*e.g.,* polymeric biomaterial) agonist of innate immunity may be provided in a dried state. In some cases, a pre-formed biomaterial (*e.g.,* polymeric biomaterial) agonist of innate immunity (in a form of a viscous polymer solution) may be provided in a container.

In some cases, provided kits may optionally include a container comprising a pharmaceutical excipient for dilution or suspension of a composition or pharmaceutical composition described herein. In some cases, provided kits may include a container comprising an aqueous solution. In some cases, provided kits may include a container comprising a buffered solution.

In some cases, provided kits may optionally include a container comprising a crosslinking agent that is useful for forming a crosslinked polymer network.

In certain cases, provided kits may not comprise an immunomodulatory payload. For example, in some cases, provided kits may not comprise an activator of innate immune response. In some cases, provided kits may not comprise an activator of adaptive immune response. In some cases, provided kits may not comprise an inhibitor of a proinflammatory response. In some cases, provided kit may not comprise an immunomodulatory cytokine.

In certain cases, a kit described herein further includes instructions for practicing methods described herein. A kit described herein may also include information as required by a regulatory agency such as the U.S. Food and Drug Administration (FDA). In certain cases, information included in kits provided herein is prescribing information, *e.g*., for treatment for cancer. Instructions may be present in kits in a variety of forms, one or more of which may be present in the kits. One form in which these instructions may be present is as printed information on a suitable medium or substrate, *e.g*., a piece or pieces of paper on which the information is printed, in the packaging of kits, in a package insert, *etc.* Yet another means may be a computer readable medium, *e.g.,* diskette, CD, USB drive, *etc.,* on which instructional information has been recorded. Yet another means that may be present is a website address which may be used via the internet to access instructional information. Any convenient means may be present in the kits.

In some cases, a kit described herein may include one or more additional therapeutic agents described herein as a separate composition.

Other features of the invention will become apparent in the course of the following examples, which are given for illustration of the invention and are not intended to be limiting thereof.

### EXEMPLIFICATION

### Example 1. Identification and/or characterization of exemplary compositions - Survival assessment

The present Example describes identification and/or characterization of a biomaterial (*e.g.,* polymeric biomaterial) agonist of innate immunity for antitumor efficacy, in particular by assessing its ability to extend survival of one or more subjects who have undergone a tumor resection. Accordingly, the present Example also describes identification and/or characterization of a biomaterial (*e.g.,* polymeric biomaterial) agonist of innate immunity that may be useful for cancer treatment (*e.g.,* as described herein).

In some cases, administration of a biomaterial (*e.g.,* polymeric biomaterial) agonist of innate immunity to a target site following a tumor resection increases survival of a subject who has undergone a tumor resection, as compared to that observed when such a biomaterial (*e.g.,* polymeric biomaterial) agonist of innate immunity is not administered.

In some cases, an animal model of cancer can be used to identify and/or characterize a biomaterial (*e.g.,* polymeric biomaterial) agonist of innate immunity. For example, a tumor resection is performed on a tumor-bearing mouse, and a composition described herein, *e.g.,* comprising a biomaterial *(e.g.,* polymeric biomaterial) agonist of innate immunity in the absence of an immunomodulatory payload, is administered to the tumor resection site. Survival of treated subjects are then monitored. In some cases, a biomaterial (*e.g.,* polymeric biomaterial) agonist of innate immunity is considered and/or determined to be useful in accordance with the present disclosure when it is characterized, in that when tested *in vivo* as described in the present Example, it extends survival of a treated subject, *e.g*., by at least 1 week, at least 2 weeks, at least 3 weeks, at least 4 weeks, at least 2 months, at least 3 months, at least 4 months, at least 5 months, at least 6 months, or longer, as compared to that observed in a control reference *(e.g.,* a control in which a biomaterial *(e.g.,* polymeric biomaterial) agonist of innate immunity is not administered). For example, in some cases, a control reference may be administration of a non-immunomodulatory polymeric biomaterial in the absence of an innate immunity immunomodulatory component. In some cases, a control reference may be administration of no polymeric biomaterial.

In some cases, female BALB/cJ mice are inoculated orthotopically with 100,000 breast cancer cells *(e.g.,* 4T1-Luc2 cells). Ten days later, tumors are surgically resected, and either (i) a composition described herein, *e.g.,* comprising a biomaterial *(e.g.,* polymeric biomaterial) agonist of innate immunity in the absence of an immunomodulatory payload *(e.g.,* so that the immunomodulatory component of the composition consists essentially of or consists of the biomaterial), or (ii) a negative control composition (*e.g.,* a buffered solution without such a biomaterial agonist of innate immunity or a non-immunomodulatory polymeric biomaterial) is administered into the resection cavity. Animal survival can be monitored to inspect for induction of antitumor immunity. To confirm that an administered biomaterial (*e.g.,* polymeric biomaterial) agonist of innate immunity functions mechanistically by inducing innate immune signaling, animal survival may be monitored following neutralization of innate immune signaling (*e.g.,* by administration of anti-IFNAR1). To assess whether an administered biomaterial (*e.g.,* polymeric biomaterial) agonist of innate immunity induces an adaptive antitumor immune response, animal survival may be monitored following depletion of particular leukocyte subsets (*e.g.,* NK cells, CD4⁺ T cells, or CD8⁺ T cells).

In particular, the present Example demonstrates administration of a biomaterial agonist of innate immunity comprising (i) a chitosan or a variant thereof (*e.g.,* carboxymethyl chitosan) as an innate immunity modulatory component and (ii) a thermo-responsive polymer that facilitates formation of a crosslinked hydrogel when exposed to body temperature of a tumor resection subject (*e.g.,* upon administration to a subject in need thereof) to a target site in a tumor resection subject improved survival of the tumor resection subject, as compared to that observed when such a biomaterial agonist of innate immunity was not administered.

***Exemplary liquid preparations:*** In some cases, a liquid preparation of a biomaterial agonist of innate immunity was prepared as follows. For example, in one instance, a 2.5 weight percent (wt%) carboxymethyl chitosan (CMCH) (*e.g.,* obtained from Heppe Medical Chitosan, Part Number 43002, Lot Number 312-210519-02) and a thermoresponsive non-immunomodulatory polymer at an appropriate concentration was prepared in a buffered system that is appropriate for injection administration. In another instance, a 5 wt% CMCH (*e.g.,* obtained from Heppe Medical Chitosan, Part Number 43002, Lot Number 312-210519-02) and a thermo-responsive non-immunomodulatory polymer at an appropriate concentration was prepared in a buffered system that is appropriate for injection administration. For example, in some cases, such a buffered system has a physiological pH. The liquid preparation was loaded into a 1 mL syringe for administration.

***Exemplary mouse tumor models:*** In some cases, animal experiments were performed using 6-8 weeks old female BALB/c mice (Jackson Laboratories, #000651). For animal survival studies, 10⁵ 4T1-Luc2 cells were inoculated orthotopically into the fourth mammary fat pad of a mouse. Tumor sizes were measured with calipers. Following sizematching, mice were randomly assigned to treatment groups, and surgery was performed on day 10 after tumor inoculation. For primary tumor resection, mice were anesthetized with 2% isoflurane, the tumor was resected, and a liquid preparation of a biomaterial agonist of innate immunity (*e.g.,* as described herein) that gels at body temperature was administered to a tumor resection site at the time of surgery.

***Figure* 1** shows survival data of animals receiving a liquid preparation of biomaterial agonist of innate immunity (*e.g.,* a liquid preparation of a combination of carboxymethyl chitosan at different concentrations and a thermo-responsive polymer), as compared to animals receiving a liquid preparation of a thermo-responsive polymer alone. As demonstrated in ***Figure 1******,*** the group of animals having *in situ* formation of a crosslinked hydrogel combination of carboxymethyl chitosan (CMCH) with a thermo-responsive polymer at a tumor resection site survived over a longer period of time, *e.g.,* by at least 50% or more, as compared to the control group that did not receive such a biomaterial agonist of innate immunity. In addition, the group of animals receiving such a combination of carboxymethyl chitosan with a thermo-responsive polymer exhibited a much higher survival rate than the control group without such a biomaterial agonist of innate immunity.

### Example 2. Identification and/or characterization of exemplary compositions - Residence time/degradation assessment

The present Example describes identification and/or characterization of a biomaterial (*e.g.,* polymeric biomaterial) agonist of innate immunity for antitumor efficacy, in particular by assessing degradation kinetics and/or residence time at a target site to which it is administered. Accordingly, the present Example also describes identification and/or characterization of a biomaterial (*e.g.,* polymeric biomaterial) agonist of innate immunity that may be useful for cancer treatment (*e.g.,* as described herein).

In some cases, a biomaterial (*e.g.,* polymeric biomaterial) agonist of innate immunity is characterized in that, when tested *in vivo* by administering a biomaterial *(e.g.,* polymeric biomaterial) to a target site in a test subject, the biomaterial (*e.g.,* polymeric biomaterial) remains at the target site *in vivo* over a period of time after the administration, *e.g.,* for at least 1 hour, at least 2 hours, at least 3 hours, at least 12 hours, at least 24 hours, at least 48 hours, at least 72 hours, at least 96 hours, at least 7 days, or longer.

In some cases, a tumor resection is performed on a tumor-bearing mouse, and a composition described herein, *e.g.,* comprising a fluorophore-labeled biomaterial *(e.g.,* polymeric biomaterial) agonist of innate immunity (or candidate) in the absence of an immunomodulatory payload *(e.g.,* so that the immunomodulatory component of the composition consists essentially of or consists of the biomaterial), is administered to the tumor resection site. Residence time and/or degradation kinetics are monitored, for example by detecting fluorescence signal of fluorophore-labeled biomaterial (*e.g.,* polymeric biomaterial) (and/or degradation products thereof) at the target site (*e.g.,* tumor resection site). In some cases, a biomaterial (*e.g.,* polymeric biomaterial) agonist of innate immunity is considered and/or determined to be useful in accordance with the present disclosure when it is characterized in that, when tested *in vivo* as described in the present Example, at least 10% or more (including, *e.g.,* at least 20%, at least 30%, at least 40%, at least 50%, at least 60%, at least 70%, at least 80%, at least 90%, or more) of the biomaterial (e.g., polymeric biomaterial) agonist of innate immunity remains at the target site *in vivo* after the administration for at least 1 hour, at least 2 hours, at least 3 hours, at least 12 hours, at least 24 hours, at least 48 hours, at least 72 hours, at least 96 hours, at least 7 days, or longer.

In some cases, female BALB/cJ mice are inoculated orthotopically with 100,000 breast cancer cells *(e.g.,* 4T1-Luc2 cells). Ten days later, tumors are surgically resected, and various compositions described herein, *e.g*., each comprising a fluorophore-labeled biomaterial (*e.g.,* polymeric biomaterial) agonist of innate immunity (or candidate) in the absence of an immunomodulatory payload, are administered into the resection cavity. Residence time of fluorophore may be detected and/or monitored, for example, by IVIS imaging, to determine a preferred residence time profile for a polymeric biomaterial agonist of innate immunity that may be useful to confer antitumor efficacy.

### Example 3. Identification and/or characterization of exemplary compositions - In vitro detection of activated leukocytes

The present Example describes identification and/or characterization of a biomaterial (*e.g.,* polymeric biomaterial) agonist of innate immunity for antitumor efficacy, in particular by assessing its ability to activate leukocytes *in vitro.* Accordingly, the present Example also describes identification and/or characterization of a biomaterial (*e.g.,* polymeric biomaterial) agonist of innate immunity that may be useful for cancer treatment (*e.g.,* as described herein).

In some cases, a biomaterial (*e.g.,* polymeric biomaterial) agonist of innate immunity is characterized in that, when tested *in vitro* by contacting dendritic cells and/or NK cells with such a biomaterial (*e.g.,* polymeric biomaterial), (i) dendritic cells are activated; (ii) NK cells are activated; and/or (iii) level of one or more proinflammatory cytokines is increased.

To determine whether a biomaterial (*e.g.,* polymeric biomaterial) or a composition described herein, *e.g.,* comprising a biomaterial *(e.g.,* polymeric biomaterial) in the absence of an immunomodulatory payload *(e.g.,* so that the immunomodulatory component of the composition consists essentially of or consists of the biomaterial), stimulates innate immunity, in some cases, markers of activation on cells of interest (*e.g.,* cells associated with innate immune system) are assessed following incubation of such a biomaterial (*e.g.,* polymeric biomaterial) with the cells of interest. In some cases, a biomaterial (*e.g.,* polymeric biomaterial) agonist of innate immunity is considered and/or determined to be useful in accordance with the present disclosure when it is characterized in that, when tested *in vitro* by contacting a biomaterial *(e.g.,* polymeric biomaterial) with dendritic cells, such a biomaterial (*e.g.,* polymeric biomaterial) increases level of antigen presentation machinery *(e.g.,* MHC I and/or MHC II) and/or co-stimulatory molecules *(e.g.,* CD40, CD80, and/or CD86) on the surface of dendritic cells after 16-24 hours, *e.g.,* by at least 10% or more (including, *e.g.,* at least 20%, at least 30%, at least 40%, at least 50%, at least 60%, at least 70%, at least 80%, at least 90%, or more), as compared to that which is observed when the dendritic cells are not contacted with such a biomaterial (*e.g.,* polymeric biomaterial). Additionally or alternatively, a biomaterial (*e.g.,* polymeric biomaterial) agonist of innate immunity is considered and/or determined to be useful in accordance with the present disclosure when it is characterized in that, when tested *in vitro* as described in the present Example, such a biomaterial (*e.g.,* polymeric biomaterial) increases the number of dendritic cells expressing MHC I and/or MHC II and/or co-stimulatory molecules, *e.g*., by at least 10% or more (including, *e.g.,* at least 20%, at least 30%, at least 40%, at least 50%, at least 60%, at least 70%, at least 80%, at least 90%, or more), as compared to that which is observed when the dendritic cells are not contacted with such a biomaterial (*e.g.,* polymeric biomaterial).

In some cases, a biomaterial *(e.g.,* polymeric biomaterial) agonist of innate immunity is considered and/or determined to be useful in accordance with the present disclosure when it is characterized in that, when tested *in vitro* by contacting a biomaterial *(e.g.,* polymeric biomaterial) with NK cells, such a biomaterial *(e.g.,* polymeric biomaterial) increases level of CD69 and/or KLRG1 after 16-24 hours, *e.g.,* by at least 10% or more (including, *e.g.,* at least 20%, at least 30%, at least 40%, at least 50%, at least 60%, at least 70%, at least 80%, at least 90%, or more), as compared to that which is observed when the NK cells are not contacted with such a biomaterial (*e.g.,* polymeric biomaterial). Additionally or alternatively, a biomaterial (*e.g.,* polymeric biomaterial) agonist of innate immunity is considered and/or determined to be useful in accordance with the present disclosure when it is characterized in that, when tested *in vitro* as described in the present Example, such a biomaterial (*e.g.,* polymeric biomaterial) increases the number of NK cells expressing CD69 and/or KLRG1 after 16-24 hours, *e.g.,* by at least 10% or more (including, *e.g.,* at least 20%, at least 30%, at least 40%, at least 50%, at least 60%, at least 70%, at least 80%, at least 90%, or more), as compared to that which is observed when the NK cells are not contacted with such a biomaterial (*e.g.,* polymeric biomaterial).

In some cases, a biomaterial (*e.g.,* polymeric biomaterial) agonist of innate immunity is considered and/or determined to be useful in accordance with the present disclosure when it is characterized in that, when tested *in vitro* by contacting a biomaterial *(e.g.,* polymeric biomaterial) with dendritic cells and/or NK cells, such a biomaterial *(e.g.,* polymeric biomaterial) increases level and/or activity and/or production of one or more proinflammatory cytokines after 16-24 hours *(e.g.,* CXCL10, IFN-α, IFN-β, IL-1β, IL-6, IL-18, and/or TNF-α), *e.g.,* by at least 10% or more (including, *e.g.,* at least 20%, at least 30%, at least 40%, at least 50%, at least 60%, at least 70%, at least 80%, at least 90%, or more), as compared to that which is observed when the dendritic cells and/or NK cells are not contacted with such a biomaterial (*e.g.,* polymeric biomaterial).

For example, in some cases, primary dendritic cells of murine or human origin (*e.g.,* murine bone marrow-derived dendritic cells or human monocyte-derived dendritic cells) can be incubated in the presence of a biomaterial (*e.g.,* polymeric biomaterial) of interest, and the cells can then be analyzed 16-24 hours later, for example by flow cytometry, to assess activation of dendritic cells, for example, by detecting level and/or activity of antigen presentation machinery (*e.g.,* MHC I and/or MHC II) and co-stimulatory molecules *(e.g.,* CD40, CD80, and/or CD86) on the surface of dendritic cells. Additionally or alternatively, NK cells can be incubated in the presence of a biomaterial (*e.g*., polymeric biomaterial) of interest, and the cells can then be analyzed 16-24 hours later, for example by flow cytometry, for relevant markers of activation (including, *e.g*., CD69 and/or KLRG1). Additionally and/or alternatively, level and/or activity of at least one or more *(e.g.,* 1, 2, 3, 4, or more) proinflammatory cytokines (including, *e.g*., CXCL10, IFN-α, IFN-β, IL-1β, IL-6, IL-18, and/or TNF-α) can also be assessed.

Various methods for assessing activation of antigen presentation machinery, costimulatory molecules, and/or proinflammatory cytokines are known in the art. Those skilled in the art will appreciate that, in some cases, level of antigen presentation machinery, costimulatory molecules, and/or proinflammatory cytokines can be assessed based on gene expression *(e.g.,* mRNA levels) in target immune cells, *e.g.,* using quantitative polymerase chain reaction. Those skilled in the art will also appreciate that immunoassays (*e.g.,* ELISA, immunostaining, and/or flow cytometry) can be used to assess such activation as well. For example, protein expression of antigen presentation machinery and/or co-stimulatory molecules on the surface of dendritic cells can be assessed, *e.g*., by immunostaining and/or flow cytometry. Concentrations of proinflammatory soluble factors such as proinflammatory cytokines in conditioned culture media can be measured, for example, using ELISA and/or multiplexing laser bead technology.

### Example 4. Identification and/or characterization of exemplary compositions - Detection of leukocytes that have been activated in vivo

The present Example describes identification and/or characterization of a biomaterial (*e.g*., polymeric biomaterial) agonist of innate immunity for antitumor efficacy, in particular by assessing its ability to activate leukocytes *in vivo.* Accordingly, the present Example also describes identification and/or characterization of a biomaterial (*e.g*., polymeric biomaterial) agonist of innate immunity that may be useful for cancer treatment (*e.g*., as described herein).

In some cases, a biomaterial (*e.g.,* polymeric biomaterial) agonist of innate immunity is characterized in that, when tested *in vivo* by administering a biomaterial *(e.g.,* polymeric biomaterial) to a tumor resection site in a test subject, (i) dendritic cells are activated; (ii) NK cells are activated; (iii) level of one or more proinflammatory cytokines, *e.g.,* in peripheral blood, is increased; and/or (iv) adaptive antitumor response is induced.

To determine whether a biomaterial (*e.g.,* polymeric biomaterial) or a composition described herein, *e.g.,* comprising a biomaterial *(e.g.,* polymeric biomaterial) in the absence of an immunomodulatory payload *(e.g.,* so that the immunomodulatory component of the composition consists essentially of or consists of the biomaterial), stimulates innate immunity, in some cases, markers of activation on cells of interest (*e.g.,* cells associated with innate and/or adaptive immune system) from a spleen are assessed 3 or 14 days following administration of such a biomaterial (*e.g.,* polymeric biomaterial) to a target tumor resection site. In some cases, a biomaterial (*e.g.,* polymeric biomaterial) agonist of innate immunity is considered and/or determined to be useful in accordance with the present disclosure when it is characterized in that, when tested *in vivo* by administering a biomaterial (*e.g.,* polymeric biomaterial) to a target site in a test subject following a tumor resection, such a biomaterial (*e.g.,* polymeric biomaterial) increases level of antigen presentation machinery *(e.g.,* MHC I and/or MHC II) and/or co-stimulatory molecules *(e.g.,* CD40, CD80, and/or CD86) on the surface of dendritic cells recovered from the test subject's spleen post-surgery *(e.g.,* 3 days post-surgery), *e.g.,* by at least 10% or more (including, *e.g.,* at least 20%, at least 30%, at least 40%, at least 50%, at least 60%, at least 70%, at least 80%, at least 90%, or more), as compared to that which is observed when such a biomaterial (*e.g.,* polymeric biomaterial) is not administered. Additionally or alternatively, a biomaterial (*e.g.,* polymeric biomaterial) agonist of innate immunity is considered and/or determined to be useful in accordance with the present disclosure when it is characterized in that, when tested *in vivo* as described in the present Example, such a biomaterial *(e.g.,* polymeric biomaterial) increases the number of dendritic cells recovered from the test subject's spleen post-surgery *(e.g.,* 3 days post-surgery) expressing antigen presentation machinery *(e.g.,* MHC I and/or MHC II) and/or co-stimulatory molecules, *e.g.,* by at least 10% or more (including, *e.g.,* at least 20%, at least 30%, at least 40%, at least 50%, at least 60%, at least 70%, at least 80%, at least 90%, or more), as compared to that which is observed when such a biomaterial (*e.g.,* polymeric biomaterial) is not administered. Additionally or alternatively, a biomaterial (*e.g.,* polymeric biomaterial) agonist of innate immunity is considered and/or determined to be useful in accordance with the present disclosure when it is characterized in that, when tested *in vivo* as described in the present Example, such a biomaterial *(e.g.,* polymeric biomaterial) increases the number of dendritic cells recovered from the test subject's spleen post-surgery (*e.g.,* 3 days post-surgery) expressing CD8 and/or CD103, and/or increases the number of plasmacytoid dendritic cells expressing B220 and/or PDCA1, *e.g.,* by at least 10% or more (including, *e.g.,* at least 20%, at least 30%, at least 40%, at least 50%, at least 60%, at least 70%, at least 80%, at least 90%, or more), as compared to that which is observed when such a biomaterial (*e.g.,* polymeric biomaterial) is not administered.

In some cases, a biomaterial (*e.g.,* polymeric biomaterial) agonist of innate immunity is considered and/or determined to be useful in accordance with the present disclosure when it is characterized in that, when tested *in vivo* by administering a biomaterial (*e.g.,* polymeric biomaterial) to a target site in a test subject following a tumor resection, such a biomaterial (*e.g.,* polymeric biomaterial) increases level of one or more activation markers *(e.g.,* CD69 and/or KLRG1) and/or one or more effector markers *(e.g.,* CD11b and/or CD27) in NK cells recovered from the test subject's spleen post-surgery (*e.g.,* 3 days post-surgery), *e.g.,* by at least 10% or more (including, *e.g.,* at least 20%, at least 30%, at least 40%, at least 50%, at least 60%, at least 70%, at least 80%, at least 90%, or more), as compared to that which is observed when such a biomaterial (*e.g.,* polymeric biomaterial) is not administered. Additionally or alternatively, a biomaterial (*e.g.,* polymeric biomaterial) agonist of innate immunity is considered and/or determined to be useful in accordance with the present disclosure when it is characterized in that, when tested *in vivo* as described in the present Example, such a biomaterial (*e.g.,* polymeric biomaterial) increases the number of NK cells recovered from the test subject's spleen post-surgery (*e.g.,* 3 days post-surgery) expressing one or more activation markers (*e.g.,* CD69 and/or KLRG1) and/or one or more effector markers *(e.g.,* CD11b and/or CD27), *e.g.,* by at least 10% or more (including, *e.g.,* at least 20%, at least 30%, at least 40%, at least 50%, at least 60%, at least 70%, at least 80%, at least 90%, or more), as compared to that which is observed when such a biomaterial (*e.g.,* polymeric biomaterial) is not administered.

In some cases, a biomaterial (*e.g.,* polymeric biomaterial) agonist of innate immunity is considered and/or determined to be useful in accordance with the present disclosure when it is characterized in that, when tested *in vivo* by administering a biomaterial (*e.g.,* polymeric biomaterial) to a target site in a test subject following a tumor resection, such a biomaterial (*e.g.,* polymeric biomaterial) increases level of one or more proinflammatory cytokines *(e.g.,* CXCL10, IFN-α, IFN-β, IL-1β, IL-6, IL-18, and/or TNF-α), *e.g.,* in peripheral blood, for example, by at least 10% or more (including, *e.g*., at least 20%, at least 30%, at least 40%, at least 50%, at least 60%, at least 70%, at least 80%, at least 90%, or more), as compared to that which is observed when such a biomaterial (*e.g.,* polymeric biomaterial) is not administered.

For example, in some cases, to dissect cellular and/or molecular changes among immune cell subsets after local administration of a biomaterial (*e.g.,* polymeric biomaterial) agonist of innate immunity in the context of surgical tumor resection, composition, activation status, and/or function of leukocytes in the spleen can be assessed. In some cases, spleens can be recovered from mice after surgical tumor resection, for example for flow cytometric analysis. At early time points *(e.g.,* day 3 post-surgery), the innate arm of the immune system of the mice, including, *e.g*., NK cells and dendritic cells, can be characterized. In some cases, the number of activated *(e.g.,* CD69+ and/or KLRG1+) and/or high effector *(e.g.,* CD11b+ and/or CD27+) NK cells from spleen can be quantified. Additionally or alternatively, in some cases, the number of dendritic cells from spleen *(e.g.,* CD8+ and/or CD103+), as well as plasmacytoid dendritic cells from spleen *(e.g.,* B220+ and/or PDCA1+) can be determined. Additionally or alternatively, in some cases, level and/or activity of antigen presentation machinery *(e.g.,* MHC I and/or MHC II) and/or costimulatory molecules *(e.g.,* CD40, CD80, and/or CD86) on the surface of dendritic cells from spleen can be assessed for activation.

In some cases, one or more soluble factors that are associated with activation of innate and/or adaptive immune cells can be assessed, for example by measuring the concentrations of one or more relevant cytokines, *e.g*., in peripheral blood. For example, in some cases, at multiple time points after administration of a biomaterial (*e.g.,* polymeric biomaterial) agonist of innate immunity to a tumor resection site, *e.g.,* after 1.5 hours, 6 hours, 3 days, or 14 days, plasma can be collected and analyzed for one or more proinflammatory cytokines of interest (e.g., CXCL10, IFN-α, IFN-β, IL-1β, IL-6, IL-18, and/or TNF-α). One of skill in the art will appreciate that various methods are available to detect and/or analyze proinflammatory cytokines, one of which may be, for example, multiplexing laser bead technology.

Various methods for assessing activation of antigen presentation machinery, costimulatory molecules, and/or proinflammatory cytokines are known in the art. Those skilled in the art will appreciate that, in some cases, level of antigen presentation machinery, costimulatory molecules, and/or proinflammatory cytokines can be assessed based on gene expression *(e.g.,* mRNA levels) in target immune cells, *e.g.,* using quantitative polymerase chain reaction. Those skilled in the art will also appreciate that immunoassays (*e.g.,* ELISA, immunostaining, and/or flow cytometry) can be used to assess such activation as well. For example, protein expression of antigen presentation machinery and/or co-stimulatory molecules on the surface of dendritic cells can be assessed, *e.g*., by immunostaining and/or flow cytometry. Concentrations of proinflammatory soluble factors such as proinflammatory cytokines in a sample *(e.g.,* a blood sample) from a subject can be measured, for example, using ELISA and/or multiplexing laser bead technology.

### Example 5. Identification and/or characterization of exemplary compositions - In vivo memory response assessment

The present Example describes identification and/or characterization of a biomaterial (*e.g.,* polymeric biomaterial) agonist of innate immunity for antitumor efficacy, in particular by assessing its ability to induce a memory response to cancer cells *in vivo.* Accordingly, the present Example also describes identification and/or characterization of a biomaterial (*e.g.,* polymeric biomaterial) agonist of innate immunity that may be useful for cancer treatment (*e.g.,* as described herein).

In some cases, a biomaterial (*e.g.,* polymeric biomaterial) agonist of innate immunity is characterized in that, when tested *in vivo* by administering a biomaterial *(e.g.,* polymeric biomaterial) to a tumor resection site in a test subject, induction of a memory response to cancer cells is observed. For example, in some cases, a biomaterial (*e.g.,* polymeric biomaterial) agonist of innate immunity is considered and/or determined to be useful in accordance with the present disclosure when it is characterized in that, when tested *in vivo* by administering a biomaterial *(e.g.,* polymeric biomaterial) to a target site in a test subject following a tumor resection, such a biomaterial (*e.g.,* polymeric biomaterial) induces a memory response to freshly inoculated cancer cells that are introduced to a test subject that has survived at least 90 days post-surgery (and post-administration of the biomaterial). Induction of a memory response to cancer cells can be demonstrated, for example, by extending survival of a treated subject by at least 1 week or more (including, *e.g.,* at least 2 weeks, at least 3 weeks, at least 4 weeks, at least 2 months, at least 3 months, at least 4 months, at least 5 months, at least 6 months, or longer), as compared to that which is observed when such a biomaterial (*e.g.,* polymeric biomaterial) is not administered prior to introduction of cancer cells. Alternatively or additionally, induction of a memory response to cancer cells can be demonstrated, for example, by a reduction in tumor volume size or tumor growth observed in a treated subject by at least 10% or more (including, *e.g.,* at least 20%, at least 30%, at least 40%, at least 50%, at least 60%, at least 70%, at least 80%, at least 90%, or more), as compared to that which is observed when such a biomaterial (*e.g.,* polymeric biomaterial) is not administered prior to introduction of cancer cells.

For example, in some cases, induction of a memory response can be assessed by re-challenging surviving mice that have been administered a biomaterial (*e.g.,* polymeric biomaterial) agonist of innate immunity after tumor resection with breast cancer cells *(e.g.,* 4T1-Luc2 cells). Such breast cancer cells can be inoculated into a mammary fat pad of the surviving mice that was not previously inoculated with cancer cells, and rejection of such inoculated breast cancer cells can be detected to assess the extent of a memory response, for example, by measuring tumor volume and/or animal survival. As a control, breast cancer cells *(e.g.,* 4T1-Luc2 cells) can be inoculated into a mammary fat pad of naive mice.

In some instances, it may be useful to assess whether induction of memory response upon administration of a biomaterial (*e.g.,* polymeric biomaterial) agonist of innate immunity is associated with systemic expansion of tumor antigen-specific CD8⁺ T cells. For example, splenocytes can be isolated from the treated mice and control groups (as described in the present Example) and re-stimulated *in vitro,* for example, for 6 hours, with an immunodominant peptide that is expressed by the inoculated cancer cells. For example, an immunodominant peptide of murine leukemia virus envelope glycoprotein gp70 (amino acids 423 to 431), SPSYVYHQF, is expressed by 4T1 cells. The proportion of T cells within the splenocytes producing one or more proinflammatory cytokines (*e.g.,* IFN-γ, IL-2, and/or TNF-α) and/or cytolytic molecules (*e.g.,* granzyme B) can be quantified after the re-stimulation. In some cases, a biomaterial (*e.g.,* polymeric biomaterial) agonist of innate immunity is considered and/or determined to be useful in accordance with the present disclosure when it is characterized in that, when tested *in vivo* (as described in the present Example), such a biomaterial (*e.g.,* polymeric biomaterial) increases the proportion of T cells (producing one or more proinflammatory cytokines such as, *e.g.,* IFN-γ, IL-2, and/or TNF-α) and/or cytolytic molecules (*e.g.,* granzyme B) within splenocytes recovered from a treated subject after re-stimulation with an immunodominant peptide that is expressed by cancer cells, *e.g.,* by at least 10% or more (including, *e.g.,* at least 20%, at least 30%, at least 40%, at least 50%, at least 60%, at least 70%, at least 80%, at least 90%, or more), as compared to that which is observed when such a biomaterial (*e.g.,* polymeric biomaterial) is not administered prior to introduction of cancer cells.

Various methods for assessing the proportion of T cells that produce one or more proinflammatory cytokines *(e.g.,* as described herein) and/or cytolytic molecules *(e.g.,* as described herein) are known in the art. One of skill in the art will appreciate that, in some instances, such a portion of T cells can be detected and/or quantified, for example, by flow cytometry. For example, intracellular cytokine staining, followed by flow cytometry, can be performed to identify immune cells *(e.g.,* T cells) that produce one or more proinflammatory cytokines and/or cytolytic molecules. Examples of such markers include, but are not limited to, CD3, CD4, and/or CD8 to define specific T cell subsets; CD44, CD69, and/or GITR for activated T cells; IFN-γ, IL-2, and/or TNF-α as proinflammatory cytokines; and granzyme B as a cytolytic molecule.

### Example 6. Identification and/or characterization of exemplary compositions - In vivo adaptive antitumor response

The present Example describes identification and/or characterization of a biomaterial (*e.g.,* polymeric biomaterial) agonist of innate immunity for antitumor efficacy, in particular by assessing its ability to induce adaptive antitumor response *in vivo* through innate immunity stimulation. Accordingly, the present Example also describes identification and/or characterization of a biomaterial (*e.g.,* polymeric biomaterial) agonist of innate immunity that may be useful for cancer treatment (*e.g.,* as described herein). In some cases, a biomaterial (*e.g.,* polymeric biomaterial) agonist of innate immunity is characterized in that, when tested *in vivo* by administering a biomaterial *(e.g.,* polymeric biomaterial) to a tumor resection site in a test subject, induction of adaptive antitumor response (in combination with stimulation of innate immunity in at least one or aspects as described herein) is observed. In some cases, a biomaterial (*e.g.,* polymeric biomaterial) agonist of innate immunity is considered and/or determined to be useful in accordance with the present disclosure when it is characterized in that, when tested *in vivo* by administering a biomaterial *(e.g.,* polymeric biomaterial) to a target site in a test subject following a tumor resection, such a biomaterial (*e.g.,* polymeric biomaterial) increases level of one or more activation markers (*e.g.,* CD69 and/or GITR) and/or one or more effector markers (*e.g.,* IFN-y and/or IL-2) in CD4⁺ and/or CD8⁺ T cells recovered from the test subject's spleen post-surgery *(e.g.,* 14 days post-surgery), *e.g.,* by at least 10% or more (including, *e.g.,* at least 20%, at least 30%, at least 40%, at least 50%, at least 60%, at least 70%, at least 80%, at least 90%, or more), as compared to that which is observed when such a biomaterial (*e.g.,* polymeric biomaterial) is not administered. Additionally or alternatively, a biomaterial (*e.g.,* polymeric biomaterial) agonist of innate immunity is considered and/or determined to be useful in accordance with the present disclosure when it is characterized in that, when tested *in vivo* as described in the present Example, such a biomaterial (*e.g.,* polymeric biomaterial) increases the number of CD4⁺ and/or CD8⁺ T cells recovered from the test subject's spleen post-surgery (*e.g.,* 14 days post-surgery) expressing one or more activation markers (*e.g.,* CD69 and/or GITR) and/or one or more effector markers *(e.g.,* IFN-y and/or IL-2), *e.g.,* by at least 10% or more (including, *e.g.,* at least 20%, at least 30%, at least 40%, at least 50%, at least 60%, at least 70%, at least 80%, at least 90%, or more), as compared to that which is observed when such a biomaterial (*e.g.,* polymeric biomaterial) is not administered.

In some cases, an adaptive antitumor response in mice subjects following administration of a biomaterial (*e.g.,* polymeric biomaterial) agonist of innate immunity can be evaluated, for example, by evaluating the T cell compartment of the spleen, for example 14 days after the administration of the biomaterial (*e.g.,* polymeric biomaterial) agonist of innate immunity. In some cases, the number of T cells *(e.g.,* ones expressing CD4 and/or CD8) and/or the proportion that expresses markers of activation (*e.g.,* CD69 and/or GITR) can be assessed.

Various methods for assessing level of activation and/or effector markers (*e.g.,* as described herein) in CD4⁺ and/or CD8⁺ T cells are known in the art. Those skilled in the art will appreciate that, in some cases, level of activation and/or effector markers (*e.g.,* as described herein) in CD4⁺ and/or CD8⁺ T cells can be assessed based on gene expression *(e.g.,* mRNA levels) in CD4⁺ and/or CD8⁺ T cells, *e.g.,* using quantitative polymerase chain reaction. Those skilled in the art will also appreciate that immunoassays (*e.g.,* ELISA, immunostaining, and/or flow cytometry) can be used to perform such assessment. For example, the presence of one or more activation markers (*e.g.,* CD69 and/or GITR) and/or one or more effector markers (*e.g.,* IFN-y and/or IL-2) in CD4⁺ and/or CD8⁺ T cells can be assessed, *e.g*., by immunostaining and/or flow cytometry.

### Example 7. Identification and/or characterization of exemplary compositions - In vivo inhibition of tumor recurrence and/or metastasis

The present Example describes identification and/or characterization of a biomaterial (*e.g.,* polymeric biomaterial) agonist of innate immunity for antitumor efficacy, in particular by assessing its ability to inhibit or reduce tumor recurrence and/or metastasis. Accordingly, the present Example also describes identification and/or characterization of a biomaterial (*e.g.,* polymeric biomaterial) agonist of innate immunity that may be useful for cancer treatment (*e.g.,* as described herein).

In some cases, a biomaterial (*e.g.,* polymeric biomaterial) agonist of innate immunity is characterized in that, when tested *in vivo* by administering a biomaterial *(e.g.,* polymeric biomaterial) to a tumor resection site in a test subject, incidence of tumor recurrence and/or metastasis is reduced, as compared to that which is observed when such a biomaterial (*e.g.,* polymeric biomaterial) is not administered. In some cases, a biomaterial (*e.g.,* polymeric biomaterial) agonist of innate immunity is considered and/or determined to be useful in accordance with the present disclosure when it is characterized in that, when tested *in vivo* by administering a biomaterial *(e.g.,* polymeric biomaterial) to a target site in a test subject following a tumor resection, such a biomaterial (*e.g.,* polymeric biomaterial) reduces incidence of tumor recurrence and/or metastasis after the tumor resection (*e.g.,* at least 1 month after tumor resection when the test subject is a mouse subject, or at least 3 months after tumor resection when the test subject is a human subject), for example, by at least 10% or more (including, *e.g.,* at least 20%, at least 30%, at least 40%, at least 50%, at least 60%, at least 70%, at least 80%, at least 90%, or more), as compared to that which is observed when such a biomaterial (*e.g.,* polymeric biomaterial) is not administered.

To assess incidence of tumor recurrence and/or metastasis *in vivo,* luciferaseexpressing breast cancer cells *(e.g.,* 4T1 cells) can be inoculated to a mammary fat pad of mice subjects. Ten days later, tumors are surgically resected and either (i) a composition described herein, *e.g.,* comprising a biomaterial *(e.g.,* polymeric biomaterial) agonist of innate immunity in the absence of an immunomodulatory payload *(e.g.,* so that the immunomodulatory component of the composition consists essentially of or consists of the biomaterial), or (ii) a negative control composition (*e.g.,* a buffered solution without such a biomaterial agonist of innate immunity) is administered to the tumor resection site of the mouse subjects. Bioluminescence signal can be subsequently detected and/or monitored over a period of time, *e.g.,* over a period of at least 1 month or longer, such as at least two months, at least three months, or longer, wherein presence of a detectable bioluminescence signal *(e.g.,* at least 10% or more above the background signal level) in the mouse subjects is indicative of tumor recurrence and/or metastasis. Incidence of tumor recurrence and/or metastasis *in vivo* can also be monitored by measuring tumor volume and/or assessing animal survival.

### EQUIVALENTS AND SCOPE

In the claims, articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. Contemplated are cases in which exactly one member of the group is present in, employed in, or otherwise relevant to a given product or process. Also contemplated are cases in which more than one, or all of the group members are present in, employed in, or otherwise relevant to a given product or process. It should be understood that, in general, where the technical disclosure, or aspects of the technical disclosure, is/are referred to as comprising particular elements and/or features, certain cases or aspects consist, or consist essentially of, such elements and/or features. For purposes of simplicity, those cases have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different cases, to the tenth of the unit of the lower limit of the range, unless the context clearly dictates otherwise.

Those skilled in the art will recognize, or be able to ascertain using no more than routine experimentation, many equivalents to the specific technical disclosure herein, but only in as far as covered by the claims. It is to be understood that the invention is not limited to what is presented in the examples by way of illustration.

## Claims

1. A composition comprising an innate immunity modulatory component, wherein the innate immunity modulatory component is a polymeric biomaterial agonist of innate immunity, wherein the polymeric biomaterial agonist of innate immunity is or comprises carboxymethyl chitosan, for use in a method of treating cancer, wherein the method comprises a step of administering said composition to a target site in a subject who is undergoing or has recently undergone a tumor resection procedure;
wherein the target site is (a) a tumor resection site; (b) a site within 4 inches of the tumor resection site; and/or (c) a sentinel lymph node.

2. The composition for use according to claim1, wherein the step of administering is by injection, optionally wherein the injection is performed with a robotic surgical system.

3. The composition for use according to claim 1 or claim 2, further comprising, prior to the step of administering, a step of performing tumor resection on the subject, optionally wherein: (a) the tumor resection is performed by non-invasive surgery and/or minimally invasive surgery (MIS), for example wherein the non-invasive surgery and/or MIS is or comprises robot-assisted MIS, robotic surgery, and/or laparoscopic surgery; and/or (b) the step of administering is performed intraoperatively.

4. The composition for use according to claim 2 or claim 3, wherein the composition is liquid and the polymeric biomaterial agonist of innate immunity is a polymer solution **characterized by** a viscosity of at least 100 mPa·s or higher.

5. The composition for use according to claim 4, wherein the polymer solution is **characterized by** a viscosity of at least 100 mPa·s and no more than 10,000 mPa·s.

6. The composition for use according to claim 2 or claim 3, wherein the composition is liquid and the polymeric biomaterial agonist of innate immunity, upon the administration, forms a polymer network biomaterial *in situ* at the target site, optionally wherein:
(a) the polymeric biomaterial agonist of innate immunity is thermo-responsive, for example wherein the polymeric biomaterial agonist of innate immunity forms a polymer network biomaterial *in situ* at the target site when it is exposed to the body temperature of the subject upon the administration; and/or
(b) the polymeric biomaterial agonist of innate immunity comprises (i) at least one innate immunity immunomodulatory polymer and (ii) at least one non-immunomodulatory polymer or a crosslinking agent, for example wherein the polymeric biomaterial agonist of innate immunity comprises a carbohydrate polymer and a thermo-responsive crosslinking agent, e.g. wherein the thermo-responsive crosslinking agent is or comprises a thermo-responsive polymer; and/or
(c) the polymer network biomaterial comprises or is a crosslinked polymer network biomaterial; and/or
(d) the polymer network biomaterial comprises or is a non-crosslinked polymer network biomaterial.

7. The composition for use according to any one of the preceding claims, wherein:
(a) the polymeric biomaterial agonist of innate immunity is **characterized in that** when tested *in vivo* by administering a polymeric biomaterial to a test subject, at least 10% of the polymeric biomaterial agonist of innate immunity remains at the target site *in vivo* 3 days after the administration; and/or
(b) the polymeric biomaterial agonist of innate immunity is **characterized by** a storage modulus of about 10 Pa to about 50,000 Pa; and/or
(c) the polymeric biomaterial comprises or is a hydrogel; and/or
(d) the step of administering does not involve administration of a tumor antigen to the tumor resection subject; and/or
(e) the step of administering does not involve administration of a microparticle to the tumor resection subject; and/or
(f) the step of administering does not involve adoptive transfer of immune cells to the tumor resection subject; and/or
(g) the step of administering does not involve administration of an immunomodulatory payload; and/or
(h) the polymeric biomaterial is biodegradable *in vivo*; and/or
(i) the tumor resection site is **characterized by** absence of gross residual tumor antigen.

8. The composition for use according to any one of the preceding claims, wherein the tumor resection subject is suffering from metastatic cancer.

9. The composition for use according to claim 8, further comprising a step of monitoring at least one metastatic site in the tumor resection subject after the administration.

10. The composition for use according to claim 1, wherein the composition consists essentially of the innate immunity modulatory component.

## Patentansprüche

1. Zusammensetzung, umfassend eine die angeborene Immunität modulierende Komponente, wobei die die angeborene Immunität modulierende Komponente ein polymerer Biomaterial-Agonist der angeborenen Immunität ist, wobei der polymere Biomaterial-Agonist der angeborenen Immunität Carboxymethylchitosan ist oder umfasst, zur Verwendung in einem Verfahren zur Behandlung von Krebs, wobei das Verfahren einen Schritt des Verabreichens der Zusammensetzung an eine Zielstelle in einem Subjekt umfasst, das sich einem Tumorresektionseingriff unterzieht oder vor kurzem unterzogen hat;
wobei die Zielstelle (a) eine Tumorresektionsstelle ist; (b) eine Stelle innerhalb von 4 Zoll von der Tumorresektionsstelle ist; und/oder (c) ein Wächterlymphknoten ist.

2. Zusammensetzung zur Verwendung nach Anspruch 1, wobei der Schritt des Verabreichens durch Injektion erfolgt, optional wobei die Injektion mit einem chirurgischen Robotersystem durchgeführt wird.

3. Zusammensetzung zur Verwendung nach Anspruch 1 oder Anspruch 2, ferner umfassend, vor dem Schritt des Verabreichens, einen Schritt des Durchführens einer Tumorresektion an dem Subjekt, optional wobei: (a) die Tumorresektion durch nicht-invasive Chirurgie und/oder minimal-invasive Chirurgie (MIS) durchgeführt wird, zum Beispiel wobei die nicht-invasive Chirurgie und/oder MIS robotergestützte MIS, Roboterchirurgie und/oder laparoskopische Chirurgie ist oder umfasst; und/oder (b) der Schritt des Verabreichens intraoperativ durchgeführt wird.

4. Zusammensetzung zur Verwendung nach Anspruch 2 oder Anspruch 3, wobei die Zusammensetzung flüssig ist und der polymere Biomaterial-Agonist der angeborenen Immunität eine Polymerlösung ist, **gekennzeichnet durch** eine Viskosität von mindestens 100 mPa·s oder höher.

5. Zusammensetzung zur Verwendung nach Anspruch 4, wobei die Polymerlösung **gekennzeichnet ist durch** eine Viskosität von mindestens 100 mPa·s und nicht mehr als 10.000 mPa·s.

6. Zusammensetzung zur Verwendung nach Anspruch 2 oder Anspruch 3, wobei die Zusammensetzung flüssig ist und der polymere Biomaterial-Agonist der angeborenen Immunität bei der Verabreichung ein Polymer-Netzwerk-Biomaterial *in situ* an der Zielstelle bildet, optional wobei:
(a) der polymere Biomaterial-Agonist der angeborenen Immunität thermoresponsiv ist, zum Beispiel wobei der polymere Biomaterial-Agonist der angeborenen Immunität ein Polymer-Netzwerk-Biomaterial *in situ* an der Zielstelle bildet, wenn er bei der Verabreichung der Körpertemperatur des Subjekts ausgesetzt wird; und/oder
(b) der polymere Biomaterial-Agonist der angeborenen Immunität (i) mindestens ein immunmodulatorisches Polymer der angeborenen Immunität und (ii) mindestens ein nicht-immunmodulatorisches Polymer oder ein Vernetzungsmittel umfasst, zum Beispiel wobei der polymere Biomaterial-Agonist der angeborenen Immunität ein Kohlenhydratpolymer und ein thermoresponsives Vernetzungsmittel umfasst, z. B. wobei das thermoresponsive Vernetzungsmittel ein thermoresponsives Polymer ist oder umfasst; und/oder
(c) das Polymernetzwerk-Biomaterial ein vernetztes Polymernetzwerk-Biomaterial umfasst oder ist; und/oder
(d) das Polymernetzwerk-Biomaterial ein nicht vernetztes Polymernetzwerk-Biomaterial umfasst oder ist.

7. Zusammensetzung zur Verwendung nach einem der vorstehenden Ansprüche, wobei:
(a) der polymere Biomaterial-Agonist der angeborenen Immunität **dadurch gekennzeichnet ist, dass**, wenn er *in vivo* durch Verabreichen eines polymeren Biomaterials an ein Testsubjekt getestet wird, mindestens 10 % des polymeren Biomaterial-Agonisten der angeborenen Immunität 3 Tage nach der Verabreichung *in vivo* an der Zielstelle verbleiben; und/oder
(b) der polymere Biomaterial-Agonist der angeborenen Immunität **gekennzeichnet ist durch** einen Speichermodul von etwa 10 Pa bis etwa 50.000 Pa; und/oder
(c) das polymere Biomaterial ein Hydrogel umfasst oder ist; und/oder
(d) der Schritt des Verabreichens nicht die Verabreichung eines Tumorantigens an das Tumorresektionssubjekt umfasst; und/oder
(e) der Schritt des Verabreichens keine Verabreichung eines Mikropartikels an das Tumorresektionssubjekt umfasst; und/oder
(f) der Schritt des Verabreichens keinen adoptiven Transfer von Immunzellen auf das Tumorresektionssubjekt umfasst; und/oder
(g) der Schritt des Verabreichens nicht die Verabreichung eines immunmodulatorischen Wirkstoffs umfasst; und/oder
(h) das polymere Biomaterial *in vivo* biologisch abbaubar ist, und/oder
(i) die Tumorresektionsstelle **gekennzeichnet ist durch** das Fehlen von makroskopischem Resttumorantigen.

8. Zusammensetzung zur Verwendung nach einem der vorstehenden Ansprüche, wobei das Tumorresektionssubjekt an metastasierendem Krebs leidet.

9. Zusammensetzung zur Verwendung nach Anspruch 8, ferner umfassend einen Schritt des Überwachens mindestens einer metastatischen Stelle in dem Tumorresektionssubjekt nach der Verabreichung.

10. Zusammensetzung zur Verwendung nach Anspruch 1, wobei die Zusammensetzung im Wesentlichen aus der die angeborene Immunität modulierenden Komponente besteht.

## Revendications

1. Composition comprenant un composant modulateur d'immunité innée, dans laquelle le composant modulateur d'immunité innée est un biomatériau polymère agoniste d'immunité innée, dans laquelle le biomatériau polymère agoniste d'immunité innée est ou comprend du carboxyméthylchitosane, pour utilisation dans un procédé de traitement du cancer, dans laquelle le procédé comprend une étape d'administration de ladite composition à un site cible chez un sujet qui est en train de subir ou a subi récemment une procédure de résection de tumeur ;
dans laquelle le site cible est (a) un site de résection de tumeur ; (b) un site à moins de 4 pouces du site de résection de tumeur ; et/ou (c) un ganglion lymphatique sentinelle.

2. Composition pour utilisation selon la revendication 1, dans laquelle l'étape d'administration est par injection, facultativement dans laquelle l'injection est mise en œuvre avec un système chirurgical robotique.

3. Composition pour utilisation selon la revendication 1 ou la revendication 2, comprenant en outre, avant l'étape d'administration, une étape de mise en œuvre de résection de tumeur sur le sujet, facultativement dans laquelle : (a) la résection de tumeur est mise en œuvre par chirurgie non invasive et/ou chirurgie miniinvasive (MIS), par exemple dans laquelle la chirurgie non invasive et/ou la MIS sont ou comprennent une MIS à assistance robotique, une chirurgie robotique et/ou une chirurgie laparoscopique ; et/ou (b) l'étape d'administration est mise en œuvre de manière peropératoire.

4. Composition pour utilisation selon la revendication 2 ou la revendication 3, dans laquelle la composition est liquide et le biomatériau polymère agoniste d'immunité innée est une solution de polymère **caractérisée par** une viscosité d'au moins 100 mPa·s ou plus élevée.

5. Composition pour utilisation selon la revendication 4, dans laquelle la solution de polymère est **caractérisée par** une viscosité d'au moins 100 mPa·s et d'au plus 10 000 mPa·s.

6. Composition pour utilisation selon la revendication 2 ou la revendication 3, dans laquelle la composition est liquide et le biomatériau polymère agoniste d'immunité innée, lors de l'administration, forme un biomatériau à réseau polymère *in situ* au niveau du site cible, facultativement dans laquelle :
(a) le biomatériau polymère agoniste d'immunité innée est thermoréactif, par exemple dans laquelle le biomatériau polymère agoniste d'immunité innée forme un biomatériau à réseau polymère *in situ* au niveau du site cible lorsqu'il est exposé à la température corporelle du sujet lors de l'administration ; et/ou
(b) le biomatériau polymère agoniste d'immunité innée comprend (i) au moins un polymère immunomodulateur d'immunité innée et (ii) au moins un polymère non immunomodulateur ou un agent de réticulation, par exemple dans laquelle le biomatériau polymère agoniste d'immunité innée comprend un polymère glucidique et un agent de réticulation thermoréactif, par exemple dans laquelle l'agent de réticulation thermoréactif est ou comprend un polymère thermoréactif ; et/ou
(c) le biomatériau à réseau polymère comprend ou est un biomatériau à réseau polymère réticulé ; et/ou
(d) le biomatériau à réseau polymère comprend ou est un biomatériau à réseau polymère non réticulé.

7. Composition pour utilisation selon l'une quelconque des revendications précédentes, dans laquelle :
(a) le biomatériau polymère agoniste d'immunité innée est **caractérisé en ce que** lorsqu'il est testé *in vivo* en administrant un biomatériau polymère à un sujet de test, au moins 10 % du biomatériau polymère agoniste d'immunité innée demeure au niveau du site cible *in vivo* 3 jours après l'administration ; et/ou
(b) le biomatériau polymère agoniste d'immunité innée est **caractérisé par** un module de conservation d'environ 10 Pa à environ 50 000 Pa ; et/ou
(c) le biomatériau polymère comprend ou est un hydrogel ; et/ou
(d) l'étape d'administration n'implique pas d'administration d'un antigène tumoral au sujet de résection de tumeur ; et/ou
(e) l'étape d'administration n'implique pas d'administration d'une microparticule au sujet de résection de tumeur ; et/ou
(f) l'étape d'administration n'implique pas de transfert adoptif de cellules immunitaires au sujet de résection de tumeur ; et/ou
(g) l'étape d'administration n'implique pas d'administration d'une charge utile immunomodulatrice ; et/ou
(h) le biomatériau polymère est biodégradable *in vivo,* et/ou
(i) le site de résection de tumeur est **caractérisé par** l'absence d'antigène tumoral résiduel macroscopique.

8. Composition pour utilisation selon l'une quelconque des revendications précédentes, dans laquelle le sujet de résection de tumeur souffre d'un cancer métastatique.

9. Composition pour utilisation selon la revendication 8, comprenant en outre une étape de surveillance d'au moins un site métastatique chez le sujet de résection de tumeur après l'administration.

10. Composition pour utilisation selon la revendication 1, dans laquelle la composition est constituée sensiblement du composant modulateur d'immunité innée.
